# EUROPEAN PATENT SPECIFICATION

(11) **EP 3 458 604 B1**
(45) Date of publication and mention of the grant of the patent: **14.04.2021**
(21) Application number: 17726856.2
(22) Date of filing: 17.05.2017
(51) Int. Cl.: C12Q 1/6886

(54) **METHOD FOR DETERMINING SENSITIVITY TO A CDK4/6 INHIBITOR**
VERFAHREN ZUR BESTIMMUNG DER EMPFINDLICHKEIT GEGENÜBER EINES CDK4/6-INHIBITORS
PROCÉDÉ DE DÉTERMINATION DE LA SENSIBILITÉ À UN INHIBITEUR CDK4/6

(30) Priority: 18.05.2016 EP 16170146
(43) Date of publication of application: 27.03.2019
(73) Proprietor: Université Libre de Bruxelles, 1050 Brussels (BE)
(72) Inventor: RASPÉ, Eric, 7700 Mouscron (BE); ROGER, Pierre, Philippe, 1040 Bruxelles (BE); COULONVAL, Katia, 7850 Enghien (BE); PATERNOT, Sabine, 7830 Hoves (BE); GOMES PITA, Jaime, Miguel, 1040 Brussels (BE); SOTIRIOU, Christos, 1180 Bruxelles (BE); IGNATIADIS, Michail, 1000 Bruxelles (BE); ROTHÉ, Françoise, 1160 Bruxelles (BE)
(74) Representative: De Clercq & Partners
(86) International application number: PCT/EP2017/061780
(87) International publication number: WO 2017/198685

(56) References cited:
- WO-A1-2015/134674
- "Affymetrix GeneChip Human Genome U133 Plus 2.0 Array", GEO EXPRESSION, 7 November 2003 (2003-11-07), XP002361326,
- I. MIGLIACCIO ET AL: "40O * Identification of gene expression signatures of palbociclib (PD) response in breast cancer (BC)", ANNALS OF ONCOLOGY., vol. 26, no. suppl 3, 1 May 2015 (2015-05-01), pages iii15-iii15, XP055312820, NL ISSN: 0923-7534, DOI: 10.1093/annonc/mdv117.02
- G. E. KONECNY ET AL: "Expression of p16 and Retinoblastoma Determines Response to CDK4/6 Inhibition in Ovarian Cancer", CLINICAL CANCER RESEARCH, vol. 17, no. 6, 28 January 2011 (2011-01-28), pages 1591-1602, XP055223034, US ISSN: 1078-0432, DOI: 10.1158/1078-0432.CCR-10-2307
- RICHARD S. FINN ET AL: "Targeting the cyclin-dependent kinases (CDK) 4/6 in estrogen receptor-positive breast cancers", BREAST CANCER RESEARCH, vol. 12, no. 11 Suppl, 9 February 2016 (2016-02-09), XP055312817, GB ISSN: 1465-5411, DOI: 10.1186/s13058-015-0661-5

## Description

### FIELD OF THE INVENTION

The present invention relates to methods for determining sensitivity to a cyclin dependent kinase 4 or 6 (CDK4/6) inhibitor in a human subject having breast cancer. The present invention provides a gene expression signature as a companion diagnostic to a CDK4/6 inhibitor that allows determining whether a human subject having breast cancer will benefit from treatment with a CDK4/6 inhibitor or whether the subject has no direct clinical benefit from such treatment or whether the subject will be insensitive to the drug.

### BACKGROUND OF THE INVENTION

Breast cancer is a heterogeneous disease subdivided in at least five different molecular subtypes such as Estrogen Receptor (ER) positive LumA or LumB tumors, EGF receptor 2 (HER2/ERBB2) positive tumors, basal-like tumor, and normal-like tumors. These subtypes are clinically relevant as they are associated with different risks and sites of recurrence and different sensitivity to chemotherapy. In the beginning of 2015, the clinical use of palbociclib (PD0332991, Ibrance) was approved in combination with letrozole, an aromatase inhibitor. Palbociclib is the first member of a new class of drugs for the treatment of advanced ER positive breast tumors (mainly LumB tumors). Palbociclib and other similar drugs, e.g., LEE-011 (Ribociclib) from Novartis and LY2835219 (Abemaciclib) from Eli Lilly, are tested in a growing number of phase II/III clinical trials against various cancers. This new class of drugs targets CDK4 and CDK6, the first cyclin dependent kinases active in the cell cycle. The cell cycle is the biological process wherein a cell duplicates its genetic material and shares it equally between two daughter cells. Its deregulation is a necessary hallmark of all cancers. Eukaryotic cell cycle depends on the sequential formation and activation of different cyclin-dependent (CDK) complexes. Cell cycle commitment at the G1 phase restriction (R) point is initiated by inactivating phosphorylation(s) of the central cell cycle/tumor suppressor pRb by CDK4 and CDK6. CDK4/6 activity requires their binding to a cyclin D (CCND1-3 genes) which is competed by INK4 CDK4 inhibitors such as p16 (CDKN2A-D).

The sensitivity of breast cancer cell lines or tumors to CDK4/6 inhibitors varies and depends to some extent on their molecular subtype. A companion diagnostic for CDK4/6 inhibitors is therefore required to determine whether a patient will benefit from a treatment with the CDK4/6 inhibitor drugs. Until now, the prediction of CDK4/6 inhibitor response relies mostly on the immunohistochemical detection of the pRb protein, without any possibility of verifying its integrity and thus capacity to be re-activated.

Migliaccio et al., (2015, Annals of Oncology, vol. 26, suppl. 3, pages iii15-iii15) concerns the identification of gene expression signatures of palbociclib response in breast cancer. A first signature, the RBsig (87 genes), was predictive of Retinoblastoma (RB) status in the TCGA dataset across all molecular subtypes. Untreated or endocrine treated patients with ER+ tumors expressing high RBsig had significantly worse recurrence free survival (RFS) compared to those with low RBsig. A second signature, PDSENSsig, was composed of 20 genes upregulated in the palbociclib sensitive cell lines. Untreated or endocrine treated patients with ER+ tumors expressing high PDSENSsig had significantly better RFS compared to those with low PDSENSsig. However, further validation in cohorts of breast cancer patients treated with palbociclib is warranted.

In view thereof, there remains a need in the art for further and/or improved companion diagnostics to CDK4/6 inhibitors.

### SUMMARY OF THE INVENTION

As illustrated in the example section, the present inventors have found that by 2D gel electrophoresis assay three cyclin-dependent kinase 4 (CDK4) modification profiles were detected in breast cancer cell lines and in tumors of breast cancer patients. A first CDK4 modification profile (referred to herein as CDK4 modification profile 1) was characterized by undetectable phosphorylation on threonine 172 (T172) of CDK4 despite a high proliferation rate. In a second CDK4 modification profile (referred to herein as CDK4 modification profile 2), T172 phosphorylated CDK4 was the predominant form of CDK4 modification. In a third CDK4 modification profile (referred to herein as CDK4 modification profile 3), phosphorylation on T172 of CDK4 was detectable but was the minor modified CDK4 form.

Importantly, the present inventors found that the detection of the CDK4 modification profiles in breast cancer cell lines samples correlated with a particular sensitivity to a CDK4/6 inhibitor.

Unfortunately, protein phosphorylation is labile during formalin fixation of tissues, the standard technique in anatomo-pathology, thereby impeding the detection of CDK4 modification profiles in formalin fixed paraffin-embedded (FFPE) samples. On the other hand, while the detection of CDK4 modification profiles was demonstrated to be possible on fresh frozen samples (as shown in the example section), the analysis of fresh frozen samples, such as fresh frozen tissues, is difficult and not common in routine clinical practice due to logistical reasons. In view thereof, the present inventors set out to find a companion diagnostic for CDK4/6 inhibitors compatible with FFPE samples.

The present inventors found that a gene expression profile comprising cyclin-dependent kinase inhibitor 2A (CDKN2A), cyclin E1 (CCNE1), transgelin 2 (TAGLN2), translocase of inner mitochondrial membrane 17 homolog A (yeast) (TIMM17A), RAB31, member of RAS oncogene family (RAB31), spindle apparatus coiled-coil protein 1 (SPDL1), nucleoporin 155kDa (NUP155), F-box and leucine-rich repeat protein 5 (FBXL5), gelsolin (GSN), TP53 target 1 (non-protein coding) (TP53TG1), and protein phosphatase 1 regulatory subunit 3C (PPP1R3C) allows to predict the CDK4 modification profile of a human subject, and hence the sensitivity of the subject to a CDK4/6 inhibitor, and thereby the requirement of treatment with the CDK4/6 inhibitor.

Accordingly, the invention provides subject-matter as set forth in any one and all of the appended claims 1 to 14.

A first aspect relates to a method for determining a cyclin-dependent kinase 4 (CDK4) modification profile in a human subject having breast cancer, the method comprising the steps of:
- determining in a sample from the subject the level of expression of 11 genes or fragments thereof selected from the group consisting of cyclin-dependent kinase inhibitor 2A (CDKN2A), cyclin E1 (CCNE1), transgelin 2 (TAGLN2), translocase of inner mitochondrial membrane 17 homolog A (yeast) (TIMM17A), RAB31, member RAS oncogene family (RAB31), spindle apparatus coiled-coil protein 1 (SPDL1), nucleoporin 155kDa (NUP155), F-box and leucine-rich repeat protein 5 (FBXL5), gelsolin (GSN), TP53 target 1 (non-protein coding) (TP53TG1), and protein phosphatase 1 regulatory subunit 3C (PPP1R3C);
- providing three reference profiles of the level of expression of the 11 genes or fragments thereof:
   (i) a first reference profile representing CDK4 modification profile 1 comprising no phosphorylation on threonine at amino acid position 172 of CDK4;
   (ii) a second reference profile representing CDK4 modification profile 2 comprising a first modified form of CDK4 which is phosphorylated on threonine at amino acid position 172 and a second modified form of CDK4, wherein the abundance of the first modified form of CDK4 is equal to or more than 90% of the abundance of the second modified form of CDK4; and
   (iii) a third reference profile representing CDK4 modification profile 3 comprising a first modified form of CDK4 which is phosphorylated on threonine at amino acid position 172 and a second modified form of CDK4, wherein the abundance of the first modified form of CDK4 is less than 90% of the abundance of the second modified form of CDK4;
- correlating the level of expression of the 11 genes or fragments thereof in the sample with the level of expression of the 11 genes or fragments thereof in the three reference profiles;
- identifying the reference profile which provides the highest correlation with the level of expression of the 11 genes or fragments thereof in the sample; and
- inferring from the identification of the reference profile which provides the highest correlation with the level of expression of the 11 genes or fragments thereof in the sample that the subject has the CDK4 modification profile corresponding to the CDK4 modification profile of the identified reference profile.

The present method allows determining the CDK4 modification profile in a sample from a human subject having breast cancer, also in FFPE samples which are routinely prepared in the clinic. Since each CDK4 modification profile correlates with a particular sensitivity of the subject to a CDK4/6 inhibitor, the present method allows to determine that the subject is insensitive to treatment with a CDK4/6 inhibitor in case the subject has CDK4 modification profile 1 (and hence the subject is preferably not to be treated with a CDK4/6 inhibitor but with classical surgery, optionally in combination with a chemotherapeutic agent and/or radiotherapy); that the subject is therapeutically sensitive to treatment with a CDK4/6 inhibitor in case the subject has CDK4 modification profile 2 (and hence the subject is preferably to be treated with a CDK4/6 inhibitor, such as with palbociclib, e.g., in combination with letrozole); or the subject is sensitive to treatment with a CDK4/6 inhibitor but has no clinical benefit from treatment with a CDK4/6 inhibitor in case the subject has CDK4 modification profile 3 (and hence the subject is preferably to be treated by classical surgery and hormone (e.g., anti-estrogen) therapy).

A second aspect relates to a method for determining sensitivity to a CDK4/6 inhibitor in a human subject having breast cancer, the method comprising the steps of:
- determining in a sample from the subject the level of expression of 11 genes or fragments thereof selected from the group consisting of cyclin-dependent kinase inhibitor 2A (CDKN2A), cyclin E1 (CCNE1), transgelin 2 (TAGLN2), translocase of inner mitochondrial membrane 17 homolog A (yeast) (TIMM17A), RAB31, member of RAS oncogene family (RAB31), spindle apparatus coiled-coil protein 1 (SPDL1), nucleoporin 155kDa (NUP155), F-box and leucine-rich repeat protein 5 (FBXL5), gelsolin (GSN), TP53 target 1 (non-protein coding) (TP53TG1), and protein phosphatase 1 regulatory subunit 3C (PPP1R3C);
- providing three reference profiles of the level of expression of the 11 genes or fragments thereof: a first reference profile representing insensitivity to the CDK4/6 inhibitor; a second reference profile representing therapeutic sensitivity to the CDK4/6 inhibitor; and a third reference profile representing sensitivity to the CDK4/6 inhibitor but without therapeutic benefit over classical treatment by surgery and hormone (e.g., anti-estrogen) therapy;
- correlating the level of expression of the 11 genes or fragments thereof in the sample with the level of expression of the 11 genes or fragments thereof in the three reference profiles;
- identifying the reference profile which provides the highest correlation with the level of expression of the 11 genes or fragments thereof in the sample; and
- inferring from the finding that:
   (i) the first reference profile provides the highest correlation with the level of expression of the 11 genes or fragments thereof in the sample, that the subject is insensitive to the CDK4/6 inhibitor;
   (ii) the second reference profile provides the highest correlation with the level of expression of the 11 genes or fragments thereof in the sample, that the subject is therapeutically sensitive to the CDK4/6 inhibitor; or
   (iii) the third reference profile provides the highest correlation with the level of expression of the 11 genes or fragments thereof in the sample, that the subject is sensitive to the CDK4/6 inhibitor but has no clinical benefit from treatment with the CDK4/6 inhibitor.

The methods illustrating the principles of the present invention advantageously allow determining the sensitivity of a human subject to a CDK4/6 inhibitor, such as palbociclib, and to identify those subjects to be treated with the CDK4/6 inhibitor. The present methods advantageously allow determining sensitivity of a human subject to a CDK4/6 inhibitor in FFPE samples which are routinely prepared in the clinic. Furthermore, the present methods allow determining sensitivity of a human subject to a CDK4/6 inhibitor independently from the molecular subtype of the breast cancer (which has been shown to incorrectly determine the sensitivity to a CDK4/6 inhibitor for a certain percentage of patients).

A further aspect relates to the use of a kit of parts for determining sensitivity to a CDK4/6 inhibitor in a human subject having breast cancer, the kit comprising means for determining the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, in a sample from a human subject, preferably wherein the kit comprises primers capable of specifically binding the 11 genes or fragments thereof. Such a kit advantageously allows determining sensitivity to a CDK4/6 inhibitor in a human subject having breast cancer.

A further aspect provides the use of a nucleic acid array or nucleic acid microarray for determining sensitivity to a CDK4/6 inhibitor in a human subject having breast cancer, the nucleic acid array or nucleic acid microarray comprising probes capable of binding to 11 genes or fragments thereof selected from the group consisting of CDKN2A, CCNE1, TAGLN2, TIMM17A, RAB31, SPDL1, NUP155, FBXL5, GSN, TP53TG1, and PPP1R3C. Such products (e.g., kits of parts, nucleic acid arrays, nucleic acid microarrays) allow determining the sensitivity to a CDK4/6 inhibitor, such as palbociclib, in a human subject having breast cancer. The advantages of the present products include that they are compatible with the analysis of FFPE samples which are routinely used in the clinic and that they allow to readily determine whether the subject will benefit from treatment with the CDK4/6 inhibitor or whether the subject has no clinical benefit from such treatment. This avoids that subjects being resistant to treatment with a CDK4/6 inhibitor or subjects having no clinical benefit of being treated, are treated despite ineffectiveness of the CDK4/6 inhibitor. Thereby, the present products (e.g., kits of parts, nucleic acid arrays, nucleic acid microarrays) avoid unnecessary costs and unnecessary exposure of the patients to the adverse effects of CDK4/6 inhibitors such as fatigue and neutropenia. The present products are thus essential to the safe and effective use of a CDK4/6 inhibitor, such as palbociclib.

A further aspect relates to a CDK4/6 inhibitor for use in treating breast cancer in a human subject, wherein the subject has been selected to have or has a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, whereby the gene expression profile of the subject correlates with a reference profile representing therapeutic sensitivity to the CDK4/6 inhibitor.

A related aspect provides a CDK4/6 inhibitor for use in a method of treating breast cancer in a human subject, wherein the method comprises the steps of:
- determining the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, in a sample from the subject, thereby obtaining a gene expression profile of the subject; and
- identifying whether the gene expression profile of the subject correlates with a reference profile representing therapeutic sensitivity to a CDK4/6 inhibitor.

Also provided in the specification is a method of treating breast cancer in a human subject in need of such a treatment, comprising administering a therapeutically effective amount of the CDK4/6 inhibitor to the subject, wherein the subject has been selected to have or has a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, that correlates with a reference profile representing therapeutic sensitivity to the CDK4/6 inhibitor.

Further provided in the specification is a method of treating breast cancer in a human subject in need of such a treatment, the method comprising:
- determining in a sample from the subject the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, thereby obtaining a gene expression profile of the subject;
- identifying whether the gene expression profile of the subject correlates with a reference profile representing therapeutic sensitivity to a CDK4/6 inhibitor; and
- if the gene expression profile of the subject correlates with the reference profile, administering a therapeutically effective amount of the CDK4/6 inhibitor to the subject, thereby treating the subject.

The present methods of treatment advantageously allow, for instance a medical practitioner such as an oncologist, to treat only those subjects with a CDK4/6 inhibitor that will immediately need and benefit from such treatment.

Those skilled in the art will immediate recognize the many other effects and advantages of the present methods, uses, or products and the numerous possibilities for end uses of the present invention from the detailed description and examples provided below.

### BRIEF DESCRIPTION OF THE FIGURES

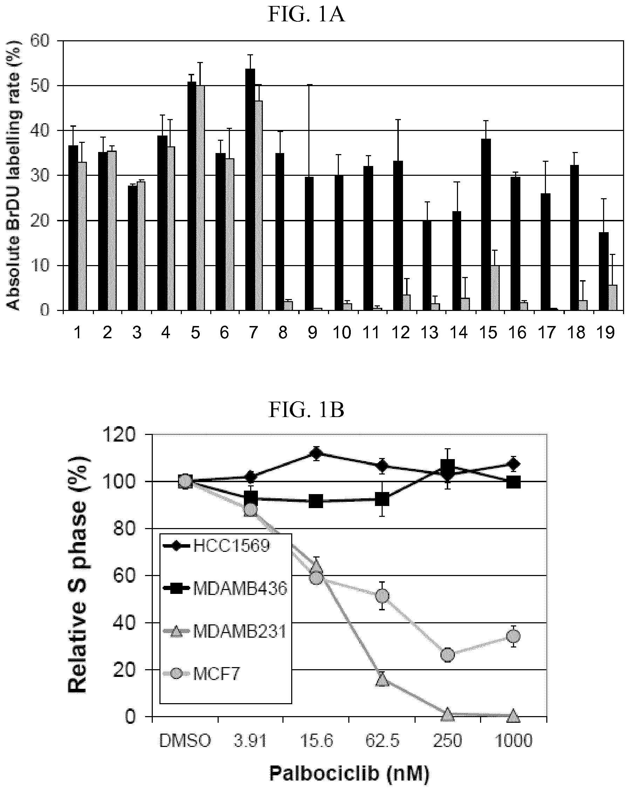

**FIG. 1A** represents a graph illustrating the sensitivity of selected breast tumor cell lines to PD0332991. 1: MDAMB436; 2: MDAMB468; 3: HCC1937; 4: BT549; 5: HCC70; 6: HCC1569; 7: HCC1806; 8: HCC38; 9: HC1500; 10: BT20; 11: MDAMB231; 12: MCF7; 13: T47D; 14: MDAMB134VI; 15: HCC1954; 16: SKBR3; 17: ZR75-1; 18: MDAMB361; and 19: BT474. Black: control asynchronous growth in the presence of 10% serum, grey: growth in the presence of 1 µM palbociclib. Average values from at least three independent experiments.
**FIG. 1B** represents a graph illustrating the relative number of cells in S phase in function of the concentration of palbociclib obtained in two sensitive (MCF7 and MDAMB231) and two insensitive (MDAMB436 and HCC1569) breast cancer cell lines. Relative proportion of BrdU-stained cells is expressed as % of the mean value of untreated control cells.

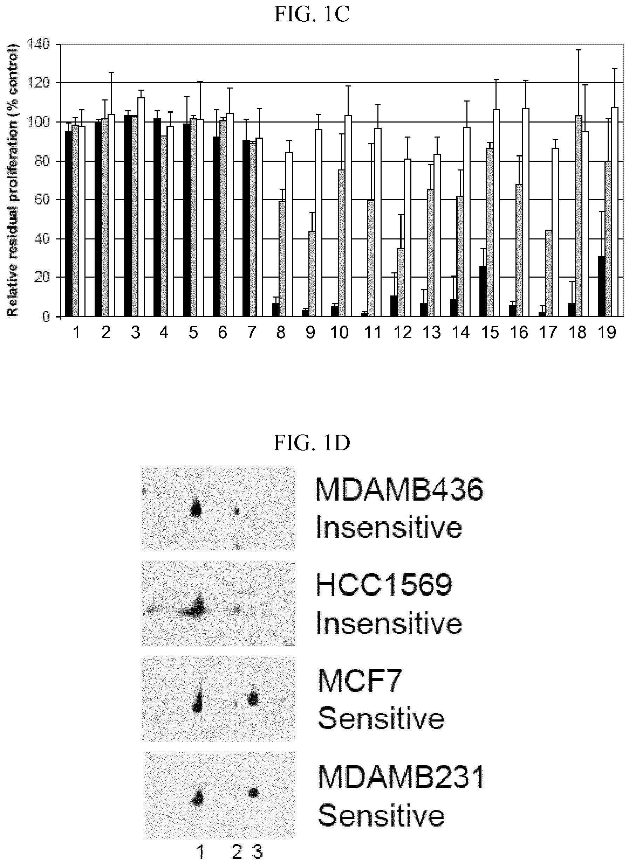

**FIG. 1C** represents a graph illustrating the average values from at least three independent experiments of the residual proliferation rate after 1 µM PD0332991 challenge expressed as % of the control value determined using BrdU labeling (black), sulforhodamine (grey) and MTT (white) assays. 1: MDAMB436; 2: MDAMB468; 3: HCC1937; 4: BT549; 5: HCC70; 6: HCC1569; 7: HCC1806; 8: HCC38; 9: HC1500; 10: BT20; 11: MDAMB231; 12: MCF7; 13: T47D; 14: MDAMB134VI; 15: HCC1954; 16: SKBR3; 17: ZR75-1; 18: MDAMB361; and 19: BT474.
**FIG. 1D** represents Western blots of 2D gel electrophoresis profiles of proteins extracted from two sensitive (MCF7 and MDAMB231) and two insensitive (MDAMB436 and HCC1569) breast cancer cell lines revealed with an anti-CDK4 antibody. 1: unmodified CDK4; 2: (unidentified) modified form of CDK4; 3: T172 phosphorylated CDK4.

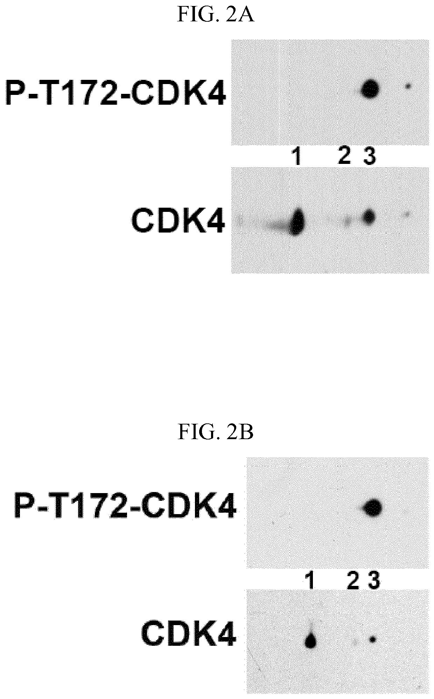

**FIG. 2A** represents Western blots illustrating the identification of T172 phosphorylated spots using a CDK4 T172-phosphospecific antibody (P-T172-CDK4, upper panel) and the CDK4 H22 antibody (CDK4, lower panel) for the MCF7 cell line. **FIG. 2B** represents Western blots illustrating the identification of T172 phosphorylated spots using a CDK4 T172-phosphospecific antibody (P-T172-CDK4, upper panel) and the CDK4 H22 antibody (CDK4, lower panel) for a frozen breast tumor sample GSM519776. 1: unmodified CDK4; 2: (unidentified) modified form of CDK4; 3: T172 phosphorylated CDK4.

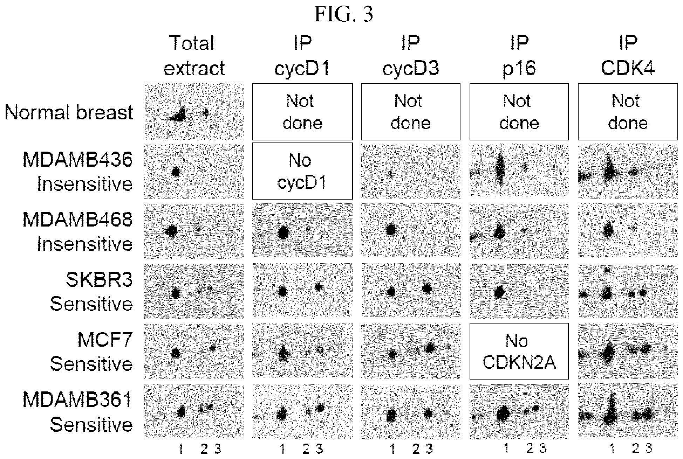

FIG. 3 represents Western blots illustrating CDK4 modification profiles of total extract or cyclin D1, cyclin D3, p16 or CDK4 immunoprecipitates. Proteins were detected with an anti-CDK4 antibody. 1: unmodified CDK4; 2: (unidentified) modified form of CDK4; 3: T172 phosphorylated CDK4.

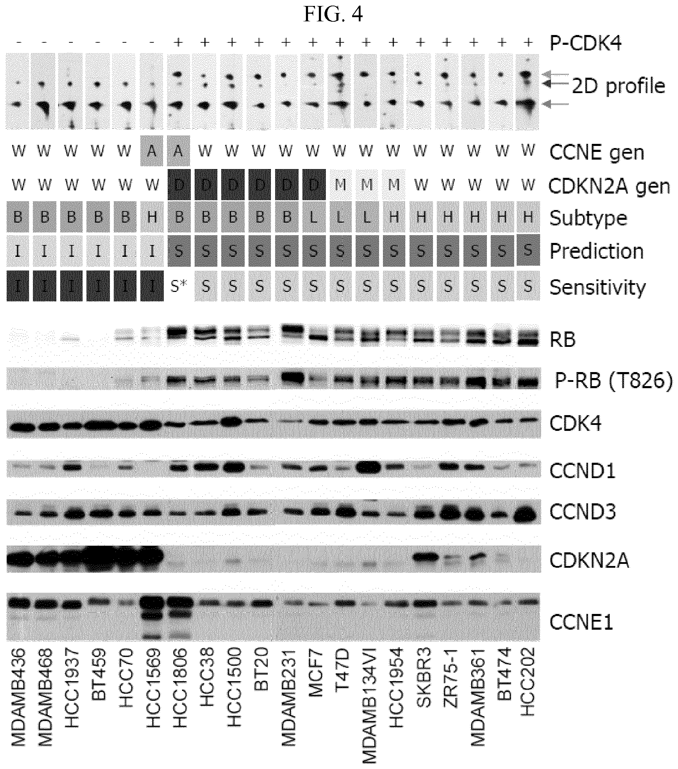

**FIG. 4** represents Western blots illustrating proteomic profiling of breast cancer cell lines. Proteins from total extracts were resolved by 1D SDS PAGE gel electrophoresis and detected with the indicated antibodies. Proteins from total extracts were also resolved by 2D gel electrophoresis and detected with an anti-CDK4 antibody. The corresponding intrinsic molecular subtypes defined based on the cell gene expression profiles, *CDKN2A* and *CCNE1* genomic locus status according to the Cosmic database, the observed and predicted palbociclib sensitivity and the detection of phosphorylated CDK4 in the corresponding 2D gel electrophoresis profiles are indicated above each 1D profiles.

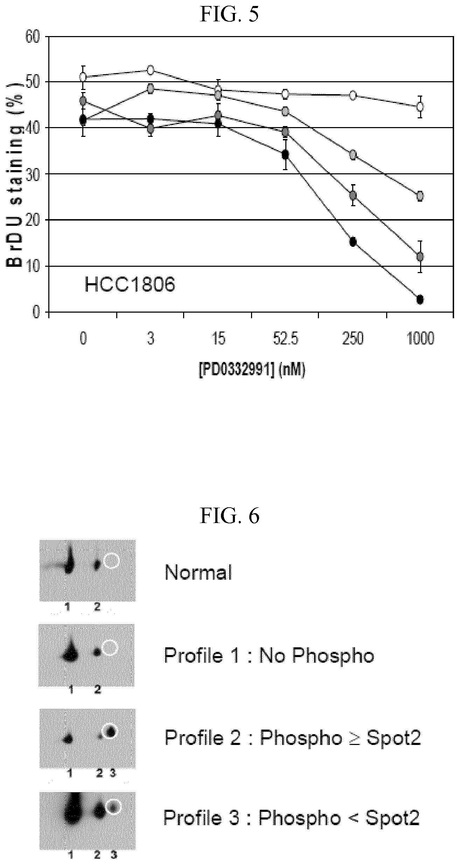

**FIG. 5** represents a graph illustrating the effects of combined administration of increasing concentrations of PD0332991 and Roscovitine on the proportion of HCC1806 cells incorporating BrdU. White: control; light grey: 2.5 µM Roscovitine; dark grey: 5 µM Roscovitine; black: 10 µM Roscovitine.
**FIG. 6** represents Western blots illustrating CDK4 modification profiles and gene expression profiles of breast tumors. Proteins extracted from frozen sections of breast tumor samples were resolved by 2D gel electrophoresis and detected with an anti-CDK4 antibody. Representative proteomic profiles are displayed. 1: unmodified CDK4; 2: (unidentified) modified form of CDK4; 3: T172 phosphorylated CDK4 (circled).

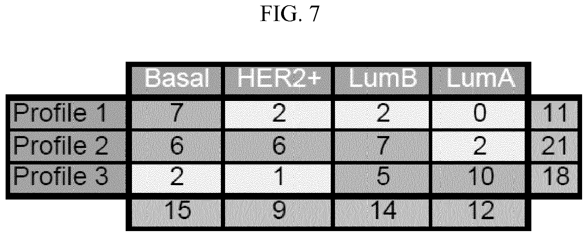

**FIG. 7** represents a table illustrating the relative proportion of the CDK4 modification profiles 1, 2, and 3 in the different intrinsic molecular subtypes of 50 analyzed breast cancer samples.

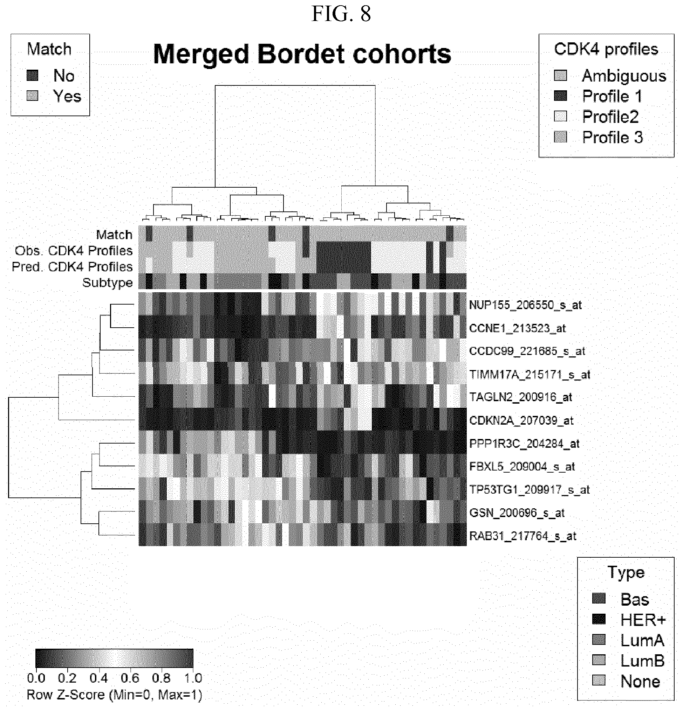

**FIG. 8** represents a heatmap illustrating the gene expression profile of the breast tumors analyzed at the Bordet Institute. RNA was extracted from the tumor samples and quantified with the Affymetrix HG-U133plus 2 platform. Heatmaps were drawn with the heatmap.plus R package using the normalized expression values of the 11 probe sets. The molecular subtype defined with the GENEFU package based on each sample expression value, the observed and predicted CDK4 modification profiles and the matches between these values are displayed above the heatmap. Breast tumors from left to right: GSM519761; Her2_7; LumA7; LumB5; LumB8; GSM519750; Her2_6; LumB1; GSM519776; GSM519749; LumB10; LumA8; LumA9; LumA4; GSM519735; GSM519804; GSM519736; LumA3; GSM519794; GSM519786; Her2_1; GSM519746; Her2_8; LumB4; Her2_3; TN4; GSM519802; TN8; GSM519745; GSM519784; LumB3; TN10; TN2; TN6; GSM519756; TN5; TN7; LumB7; LumB6; LumB9; GSM519806; Her2_2; GSM519774; TN1; Her2_4; TN3; GSM519729; TN9.

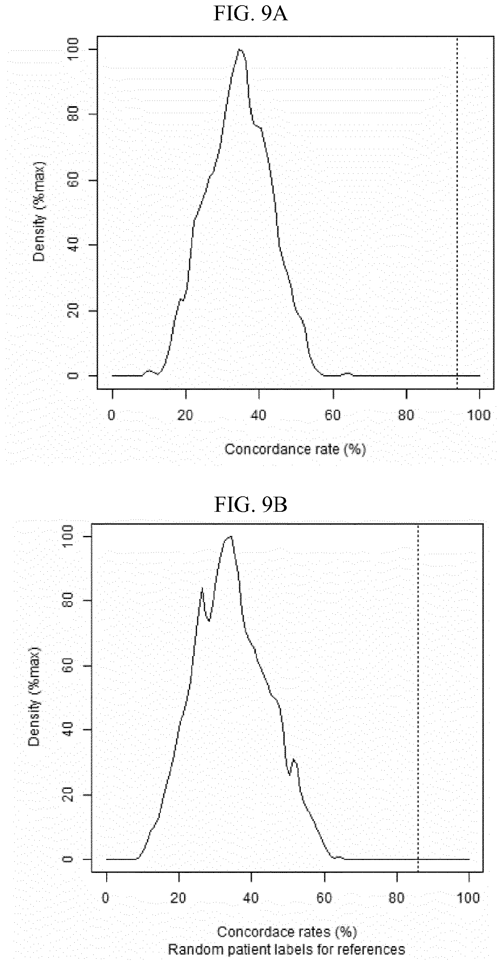

**FIG. 9** represents graphs illustrating the statistical evaluation of the performance of the CDK4 modification profile prediction. In **FIG. 9A****,** the performance of the CDK4 modification profile prediction using the optimal set of 11 probes (dotted line) was compared to the distribution of the performances of 1000 random lists of 11 probes. In **FIG. 9B****,** the performance of CDK4 modification profile prediction using the optimal set of 11 probes (dotted line) in the whole Bordet breast cancer sample cohort was compared to the distribution of the corresponding performances achieved when the patient labels were randomly switched 1000 times.

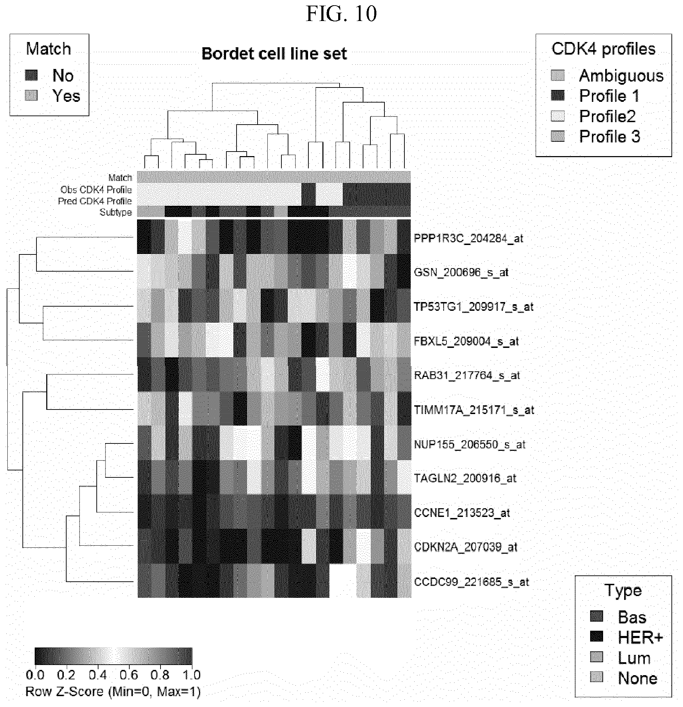

**FIG. 10** represents a heatmap illustrating the gene expression profile of the 20 selected breast cancer cell lines analyzed at the Bordet Institute. RNA was extracted from the samples and quantified with the Affymetrix HG-U133plus 2 platform. Heatmaps were drawn with the heatmap.plus R package using the normalized expression values of the 11 probe sets. The molecular subtype defined with the R GENEFU package based on each sample expression value, the observed and predicted CDK4 modification profiles and the matches between these values are displayed above each heatmap. Breast cancer cell lines from left to right: MDAMB134VI; T47D; BT474; HCC202; HCC1500; MDAMB361; BT20; HCC1806; HCC1954; MDAMB231; MCF7; ZR751; HCC1569; SKBR3; HCC38; MDAMB468; HCC1937; HCC70; BT549; MDAMB436.

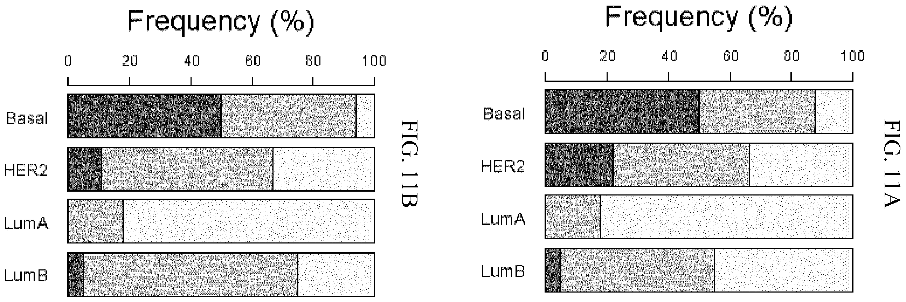

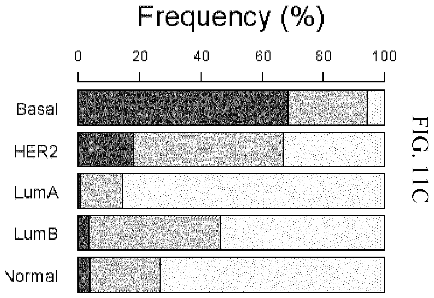

**FIG. 11** represents graphs illustrating the proportion of CDK4 modification profiles 1, 2, and 3 among breast cancer subtypes. The proportions of the observed CDK4 modification profiles **(****FIG. 11A**) or CDK4 modification profiles predicted by a method according to an embodiment of the invention **(****FIG. 11B**) among the breast cancer intrinsic subtypes defined with the PAM50 references of the R GENEFU package in the 56 breast tumors of the Bordet Institute and Antwerp University are displayed. The corresponding proportions in a set of 4034 breast cancer patients with previously published gene expression profiles obtained with the Affymetrix HG-U133A or HG-U133plus 2 platforms and downloaded from the GEO and Array Express databases are shown in **FIG. 11C**. Black: CDK4 modification profile 1; Grey: CDK4 modification profile 2; white: CDK4 modification profile 3.

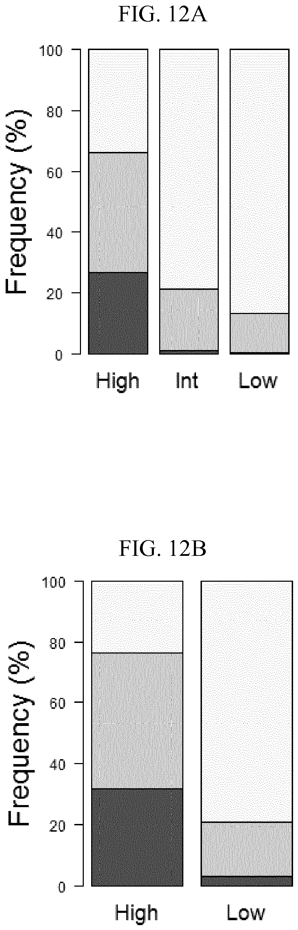

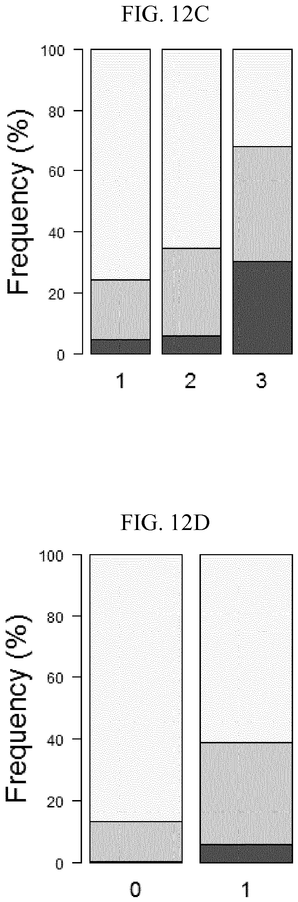

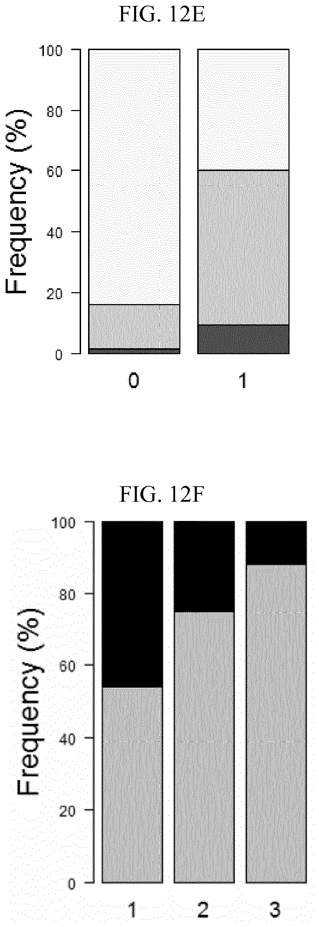

**FIG. 12** represents graphs illustrating the proportion of predicted CDK4 modification profiles 1, 2, and 3 among risk profiles or grade in 4034 breast cancer patients with previously published gene expression profiles. The gene expression profiles from breast cancer patients obtained with the Affymetrix HG-U133A or HG-U133plus 2 platforms were downloaded from the GEO database and gathered in 3 cohorts. CDK4 modification profiles were predicted by a method according to an embodiment of the invention.
**FIG. 12A** illustrates the proportion of predicted CDK4 modification profiles among Oncotype DX risk categories. Oncotype DX risk categories (high, intermediate, low) were defined using the GENEFU package in R. **FIG. 12D** illustrates the corresponding proportions in ER positive tumors.0: low Oncotype DX risk; 1: high or intermediate Oncotype DX risk. Black: CDK4 modification profile 1; Grey: CDK4 modification profile 2; white: CDK4 modification profile 3.
**FIG. 12B** illustrates the proportion of predicted CDK4 modification profiles among GGI risk categories. GGI risk categories (high, low) were defined using the GENEFU package in R. **FIG. 12E** illustrates the corresponding proportions in ER positive tumors 0: low GGI risk; 1: high GGI risk. Black: CDK4 modification profile 1; Grey: CDK4 modification profile 2; white: CDK4 modification profile 3.
**FIG. 12C** illustrates the proportions of predicted CDK4 modification profiles among published grade. 1: grade 1; 2: grade 2; 3: grade 3. Black: CDK4 modification profile 1; Grey: CDK4 modification profile 2; white: CDK4 modification profile 3.
**FIG. 12** **F** illustrates the proportions of predicted CDK4 modification profiles among pathological complete response (pCR) upon anthracycline-taxane treatment. Black: pCR = 1; grey: pCR = 0. 1: CDK4 modification profile 1; 2: CDK4 modification profile 2; 3: CDK4 modification profile 3.

### DETAILED DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of', which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of ± 10% or less, preferably ± 5% or less, more preferably ± 1% or less, and still more preferably ± 0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

As noted above, the inventors have realised that a gene expression signature comprising CDKN2A, CCNE1, TAGLN2, TIMM17A, RAB31, SPDL1, NUP155, FBXL5, GSN, TP53TG1, and PPP1R3C allows determining the CDK4 modification profile of a human subject, and hence the sensitivity of the subject to a CDK4/6 inhibitor.

Accordingly, a first aspect of the invention provides a method or assay for determining a cyclin-dependent kinase 4 (CDK4) modification profile in a human subject having breast cancer, the method comprising the steps of:
- determining in a sample from the subject the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C;
- providing three reference profiles of the level of expression of the 11 genes or fragments thereof:
   (i) a first reference profile representing CDK4 modification profile 1 comprising no phosphorylation on threonine at amino acid position 172 of CDK4;
   (ii) a second reference profile representing CDK4 modification profile 2 comprising a first modified form of CDK4 which is phosphorylated on threonine at amino acid position 172 and a second modified form of CDK4, wherein the abundance of the first modified form of CDK4 is equal to or more than 90% of the abundance of the second modified form of CDK4; and
   (iii) a third reference profile representing CDK4 modification profile 3 comprising a first modified form of CDK4 which is phosphorylated on threonine at amino acid position 172 and a second modified form of CDK4, wherein the abundance of the first modified form of CDK4 is less than 90% of the abundance of the second modified form of CDK4;
- correlating the level of expression of the 11 genes or fragments thereof in the sample with the level of expression of the 11 genes or fragments thereof in the three reference profiles;
- identifying the reference profile which provides the highest correlation with the level of expression of the 11 genes or fragments thereof in the sample; and
- inferring from the identification of the reference profile which provides the highest correlation with the level of expression of the 11 genes or fragments thereof in the sample that the subject has the CDK4 modification profile corresponding to the CDK4 modification profile of the identified reference profile.

In certain embodiments, the invention provides a method or assay for determining a CDK4 modification profile in a human subject having breast cancer.

The methods or assays as taught herein are generally performed *in vitro,* on a sample (such as a biopsy, e.g., tissue) obtained from a subject.

The term "*in vitro*" generally denotes outside, or external to, animal or human body. The term "*ex vivo*" typically refers to tissues or cells removed from an animal or human body and maintained or propagated outside the body, e.g., in a culture vessel. The term "*in vitro*" as used herein should be understood to include "*ex vivo*"*.* The term "*in vivo*" generally denotes inside, on, or internal to, animal or human body.

Except when noted, "subject" or "patient" are used interchangeably and refer to animals, preferably warm-blooded animals, more preferably vertebrates, even more preferably mammals, still more preferably primates, and specifically includes human patients and non-human mammals and primates. Preferred subjects are human subjects.

The term "mammal" includes any animal classified as such, including, but not limited to, humans, domestic and farm animals, zoo animals, sport animals, pet animals, companion animals and experimental animals, such as, for example, mice, rats, hamsters, rabbits, dogs, cats, guinea pigs, cattle, cows, sheep, horses, pigs and primates, e.g., monkeys and apes. Particularly preferred are human subjects, including both genders and all age categories thereof.

The CDK4 as disclosed herein denotes wild-type CDK4.

As used herein, the term "wild-type" as applied to a nucleic acid or polypeptide refers to a nucleic acid or a polypeptide that occurs in, or is produced by, a biological organism as that biological organism exists in nature. The term "wild-type" may to some extent be synonymous with "native", the latter encompassing nucleic acids or polypeptides having a native sequence, i.e., ones of which the primary sequence is the same as that of the nucleic acids or polypeptides found in or derived from nature. A skilled person understands that native sequences may differ between or within different individuals of the same species due to normal genetic diversity (variation) within a given species. Also, native sequences may differ between or within different individuals of the same species due to post-transcriptional or post-translational modifications. Any such variants or isoforms of nucleic acids or polypeptides are encompassed herein as being "native". Accordingly, all sequences of nucleic acids or polypeptides found in or derived from nature are considered "native". The term "native" encompasses the nucleic acids or polypeptides when forming a part of a living organism, organ, tissue or cell, when forming a part of a biological sample, as well as when at least partly isolated from such sources.

Since the methods or assays as taught herein determine the CDK4 modification profile in a human subject, the term "CDK4" as used herein refers to human CDK4.

Human cyclin-dependent kinase 4 (CDK4) (EC 2.7.11.22) is a member of the Ser/Thr protein kinase family. CDK4 is a catalytic subunit of a protein kinase complex that is important for cell cycle G1 phase progression. The activity of CDK4 is restricted to the G1-S phase, which is controlled by the regulatory subunits D-type cyclins and CDK inhibitor p16^{INK4a}. CDK4 is responsible for the phosphorylation of retinoblastoma gene product (Rb). CDK4 has been described in the literature, such as in Bockstaele et al. (2006, Cell Div., 1, 25).

Exemplary human CDK4 protein sequence may be as annotated under U.S. government's National Center for Biotechnology Information (NCBI) Genbank (http://www.ncbi.nlm.nih.gov/) accession number NP_000066.1 (sequence version 1), or Swissprot/Uniprot (http://www.uniprot.org/) accession number P11802-1 (isoform 1). Exemplary human CDK4 mRNA (cDNA) sequence may be as annotated under NCBI Genbank accession number NM_000075.3 (sequence version 3).

In certain embodiments, the subject has or has been diagnosed with breast cancer. Methods for diagnosing breast cancer are known in the art and include microscopic analysis of a sample (biopsy) of the affected area of the breast.

The term "breast cancer" refers to cancer that develops from breast tissue.

The term "cancer" as used herein refers to a malignant neoplasm characterized by deregulated or unregulated cell growth.

As used herein, the terms "tumour" or "tumour tissue" refer to an abnormal mass of tissue resulting from excessive cell division. A tumour or tumour tissue comprises "tumour cells" which are neoplastic cells with abnormal growth properties and no useful bodily function. Tumours, tumour tissue and tumour cells may be benign, pre-malignant or malignant, or may represent a lesion without any cancerous potential. A tumour or tumour tissue may also comprise "tumour-associated non-tumour cells", e.g., vascular cells which form blood vessels to supply the tumour or tumour tissue. Non-tumour cells may be induced to replicate and develop by tumour cells, for example, the induction of angiogenesis in a tumour or tumour tissue.

In certain embodiments, the breast cancer may be a malignant breast tumor.

In certain embodiments, the molecular subtype of the breast cancer may be basal-like (triple negative), luminal A, luminal B, or HER2 positive (HER2+).

In certain embodiments of the methods, uses, or products, as taught herein, the CDK4 modification profile may be CDK4 modification profile 1 comprising no phosphorylation on threonine at amino acid position 172 of CDK4; CDK4 modification profile 2 comprising a first modified form of CDK4 which is phosphorylated on threonine at amino acid position 172 and a second modified form of CDK4, wherein the abundance of the first modified form of CDK4 is equal to or more than 90% of the abundance of the second modified form of CDK4; and CDK4 modification profile 3 comprising a first modified form of CDK4 which is phosphorylated on threonine at amino acid position 172 and a second modified form of CDK4, wherein the abundance of the first modified form of CDK4 is less than 90% of the abundance of the second modified form of CDK4.

The term "CDK4 modification profile" as used herein refers to a pattern of the different protein forms of endogenous CDK4.

The term "endogenous CDK4" as used herein refers to a CDK4 protein resulting from expression of the *CDK4* gene naturally found in the genome of the cells in which the CDK4 protein is expressed.

The terms "protein", "polypeptide", or "peptide" can be used interchangeably and relate to any natural or recombinant molecule comprising amino acids joined together by peptide bonds between adjacent amino acid residues. A "peptide bond", "peptide link" or "amide bond" is a covalent bond formed between two amino acids when the carboxyl group of one amino acid reacts with the amino group of the other amino acid, thereby releasing a molecule of water. The terms "amino acid" and "amino acid residue" may be used interchangeably herein.

By "nucleic acid" is meant oligomers and polymers of any length composed essentially of nucleotides, e.g., deoxyribonucleotides and/or ribonucleotides. Nucleic acids can comprise purine and/or pyrimidine bases and/or other natural (e.g., xanthine, inosine, hypoxanthine), chemically or biochemically modified (e.g., methylated), non-natural, or derivative nucleotide bases. The backbone of nucleic acids can comprise sugars and phosphate groups, as can typically be found in RNA or DNA, and/or one or more modified or substituted sugars and/or one or more modified or substituted phosphate groups. Modifications of phosphate groups or sugars may be introduced to improve stability, resistance to enzymatic degradation, or some other useful property. A "nucleic acid" can be for example double-stranded, partly double stranded, or single-stranded. Where single-stranded, the nucleic acid can be the sense strand or the antisense strand. In addition, nucleic acid can be circular or linear. The term "nucleic acid" as used herein preferably encompasses DNA and RNA, specifically including RNA, genomic RNA, cDNA, DNA, provirus, pre-mRNA and mRNA.

The CDK4 modification profile may include unmodified CDK4 (i.e., CDK4 not modified by posttranslational modification such as phosphorylation and/or acetylation) and one or more, such as two, posttranslational modified forms of CDK4. The posttranslational modified forms of CDK4 include a T172 phosphorylated form of CDK4 (referred to herein as "first modified form of CDK4") and at least one other, yet unidentified but not phosphorylated, form of CDK4 (referred to herein as "second modified form of CDK4"). The unmodified form of CDK4 is the most basic form, the first modified form of CDK4 is the most acidic form, and the second modified form of CDK4 is an intermediate form, as detected by 2D gel electrophoresis and Western blotting. The second modified form of CDK4 does not incorporate [32P] phosphate. Therefore, it is considered that the second modified form is not phosphorylated.

In certain embodiments of the methods as taught herein, the CDK4 modification profile 1 comprises no phosphorylation on threonine at amino acid position 172 (T172) of CDK4. In certain embodiments, the CDK4 modification profile 1 may comprise unmodified CDK4 and the second modified form of CDK4.

In certain embodiments of the methods as taught herein, the CDK4 modification profile comprises a first modified form of CDK4 which is phosphorylated on threonine at amino acid position 172 and a second modified form of CDK4, wherein the abundance of the first modified form of CDK4 is equal to or more than 90% of the abundance of the second modified form of CDK4. In certain embodiments of the methods as taught herein, the CDK4 modification profile 2 may comprise unmodified CDK4.

In certain embodiments of the methods as taught herein, the CDK4 modification profile 3 comprising a first modified form of CDK4 which is phosphorylated on threonine at amino acid position 172 and a second modified form of CDK4, wherein the abundance of the first modified form of CDK4 is less than 90% of the abundance of the second modified form of CDK4. In certain embodiments, the CDK4 modification profile 2 may comprise unmodified CDK4.

In certain embodiments of the methods as taught herein, the CDK4 modification profile may be determined by measuring the abundance of the first modified form of CDK4 and the abundance of the second modified form of CDK4, calculating the ratio of the abundance of the first modified form to the abundance of the second modified form (ratio = abundance first modified form of CDK4/abundance second modified form CDK4), and assigning to the subject CDK4 modification profile 1 when the ratio is below 0.1; assigning to the subject CDK4 modification profile 2 when the ratio is above 0.9; or assigning to the subject CDK4 modification profile 3 when the ratio is between 0.1 and 0.9.

In certain embodiments of the methods as taught herein, the presence of the different protein forms of CDK4 may be determined by methods known in the art for detecting unmodified and posttranslational modified proteins such as mass spectrometry or 2D gel electrophoresis analysis and Western blotting. In certain embodiments of the methods as taught herein, the abundance of the different protein forms of CDK4 may be measured by methods known in the art such by as mass spectrometry or 2D gel electrophoresis analysis and immuno-detection methods including Western blotting. After 2D gel electrophoresis analysis and Western blotting, allowing detection of the different forms of CDK4 by any appropriate methods known in the art using antibodies coupled to any labels, probes, dyes or enzymes, the abundance of the different protein forms of CDK4 may be determined by any technique including autoradiography, phosphor-imaging, fluorescence imaging, colorimetric reaction, or chemiluminescence reaction detected by film exposure or any camera design and imager, and quantifying the volumes of the spots corresponding to the different forms of CDK4.

In certain embodiments, the method comprises determining or measuring in a sample from the subject the level of expression of 11 (eleven) genes or fragments thereof selected from the group consisting of CDKN2A, CCNE1, TAGLN2, TIMM17A, RAB31, SPDL1, NUP155, FBXL5, GSN, TP53TG1, and PPP1R3C.

The wording "gene expression signature" refers to the combination of the at least 11 genes or fragments thereof as defined herein. For instance, the gene expression signature may comprise or consist of CDKN2A or a fragment thereof, CCNE1 or a fragment thereof, TAGLN2 or a fragment thereof, TIMM17A or a fragment thereof, RAB31 or a fragment thereof, SPDL1 or a fragment thereof, NUP155 or a fragment thereof, FBXL5 or a fragment thereof, GSN or a fragment thereof, TP53TG1 or a fragment thereof, and PPP1R3C or a fragment thereof. The gene expression signature advantageously allows determining sensitivity to a CDK4/6 inhibitor in a human subject having breast cancer.

The genes or fragments thereof as taught herein are also referred to herein as markers or biomarkers.

The term "gene" generally refers to a locus (or region) of nucleic acid that encodes a functional RNA or protein. As used herein the term "fragment" shall be understood to mean a nucleic acid that is the same as part of, but not all of, a nucleic acid that forms a gene. Without limitation, a fragment of a gene may represent at least about 5% (by nucleic acid number), or at least about 10%, e.g., 20% or more, 30% or more, or 40% or more, such as preferably 50% or more, e.g., 60% or more, 70% or more, 80% or more, 90% or more, or 95% or more of the nucleic acid sequence of said gene.

The terms "sample" or "biological sample" as used herein include any biological specimen obtained from a subject.

Samples may include biopsies and tissue samples, tissue homogenates and the like. Samples may also include, without limitation, fine needle aspirate and cell lysates. Preferred samples may include ones comprising any one or more markers as taught herein in detectable quantities. Preferably, the sample is a biopsy, tissue sample, or fine needle aspirate, more preferably, the sample is a biopsy. Preferably the sample is readily obtainable by surgical methods or by minimally invasive methods, allowing the removal or isolation of said sample from the subject.

In certain embodiments, the sample is isolated from a malignant breast tumour.

Preferably, the sample used to determine the levels of expression of the 11 genes or fragments thereof as taught herein is a biopsy. The term "biopsy" generally denotes a sample of cells or tissues removed from a living subject for examination. The biopsy may be an excisional biopsy (i.e., when an entire lump or suspicious area is removed), an incisional biopsy or core biopsy (i.e., when only a sample of tissue is removed with preservation of the histological architecture of the tissue's cells), or a needle aspiration biopsy (i.e., when a sample of tissue or fluid is removed with a needle in such a way that cells are removed without preserving the histological architecture of the tissue cells).

The sample can be subjected to a variety of well-known post-collection preparative and storage techniques (e. g. fixation, storage, freezing, lysis, homogenization, DNA or RNA extraction, ultrafiltration, concentration, evaporation, centrifugation, etc.) prior to determining the level of expression in the sample.

In certain embodiments of the methods, uses, or products as taught herein, the sample may be a formalin-fixed paraffin-embedded (FFPE) sample or fresh frozen sample. Preferably, the sample is a FFPE sample. The present methods advantageously allow determining the sensitivity of a human subject having breast cancer to a CDK4/6 inhibitor in FFPE samples which are routinely used in the clinic.

The recitation "determining in a sample the level of expression" refers to determining the gene expression profile.

As used herein, the terms "profile", "gene profile" or "gene expression profile" refers to a repository of the expression level data that can be used to compare the expression levels of different genes among various subjects.

The phrase "determining the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C" refers to determining the level of expression of CDKN2A or a fragment thereof, CCNE1 or a fragment thereof, TAGLN2 or a fragment thereof, TIMM17A or a fragment thereof, RAB31 or a fragment thereof, SPDL1 or a fragment thereof, NUP155 or a fragment thereof, FBXL5 or a fragment thereof, GSN or a fragment thereof, TP53TG1 or a fragment thereof, and PPP1R3C or a fragment thereof.

The level of expression of a gene or a group of two or more genes is "determined" in a sample when the quantity of the gene or of the group of two or more genes is detected or measured in the sample.

The terms "quantity", "amount" and "level" are synonymous and generally well-understood in the art. The terms as used herein may particularly refer to an absolute quantification of a molecule or an analyte, such as a gene, in a sample, or to a relative quantification of a molecule or analyte in a sample, i.e., relative to another value such as relative to a reference value as taught herein, or to a range of values indicating a base-line of the biomarker. These values or ranges may be obtained from a single patient or from a group of patients.

An absolute quantity of a molecule or analyte in a sample may depend on the method used to determine the level of expression of a gene or fragment thereof. For instance, an absolute quantity of a molecule or analyte in a sample may be advantageously expressed as read counts (eventually normalized) (e.g., when using RNA sequencing), photon counts (pixels or fluorescence intensity) (e.g., when using microarray), or photon counts at m/z (e.g., when using mass spectrometry).

A relative quantity of a molecule or analyte in a sample may be advantageously expressed as an increase or decrease or as a fold-increase or fold-decrease relative to said another value, such as relative to a reference value as taught herein (e.g., when using quantitative real-time PCR (qPCR)). Performing a relative comparison between first and second parameters (e.g., first and second quantities) may but need not require determining first the absolute values of said first and second parameters. For example, a measurement method may produce quantifiable readouts (such as, e.g., signal intensities) for said first and second parameters, wherein said readouts are a function of the value of said parameters, and wherein said readouts may be directly compared to produce a relative value for the first parameter vs. the second parameter, without the actual need to first convert the readouts to absolute values of the respective parameters.

As used herein, the reference to any one marker (biomarker) or gene corresponds to the marker or gene commonly known under the respective designations in the art. The terms encompass such markers or genes of any organism where found, and particularly of animals, preferably warm-blooded animals, more preferably vertebrates, yet more preferably mammals, including humans and non-human mammals, still more preferably of humans. The terms particularly encompass such markers or genes with a native sequence, i.e., ones of which the primary sequence is the same as that of the markers or genes in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to post-transcriptional or post-translational modifications. Any such variants or isoforms of markers or genes are intended herein. Accordingly, all sequences of markers or genes found in or derived from nature are considered "native". The terms encompass the markers or genes when forming a part of a living organism, organ, tissue or cell, when forming a part of a biological sample, as well as when at least partly isolated from such sources.

Exemplary human genes as taught herein may be as annotated under Swissprot/Uniprot (http://www.uniprot.org/) or NCBI Genbank (http://www.ncbi.nlm.nih.gov/) accession numbers given below. A skilled person will appreciate that although only one or more isoforms may be listed below, all isoforms are intended. The entries in Table 1 are presented in the form: gene, NCBI Genbank EntrezID, name, Swissprot entry, Genbank RefSeq for one or more representative amino acid sequences (e.g., isoforms) followed by the Genbank sequence version, Genbank RefSeq for one or more representative mRNA sequences followed by the Genbank sequence version.

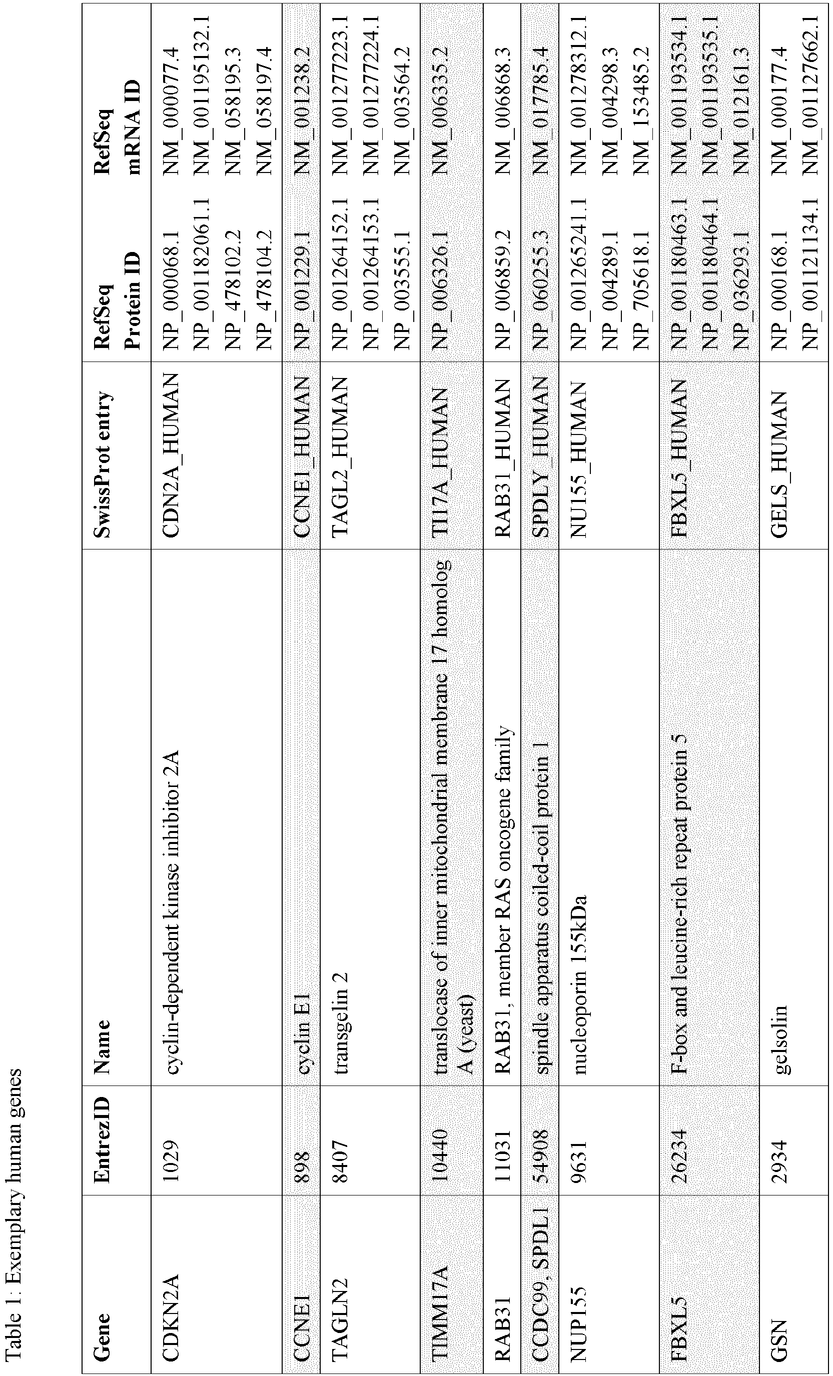

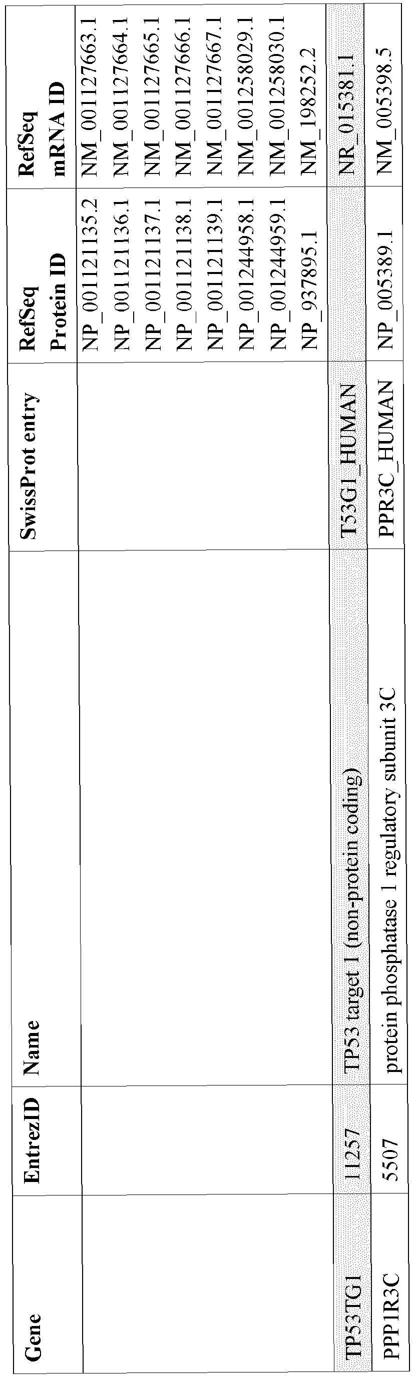

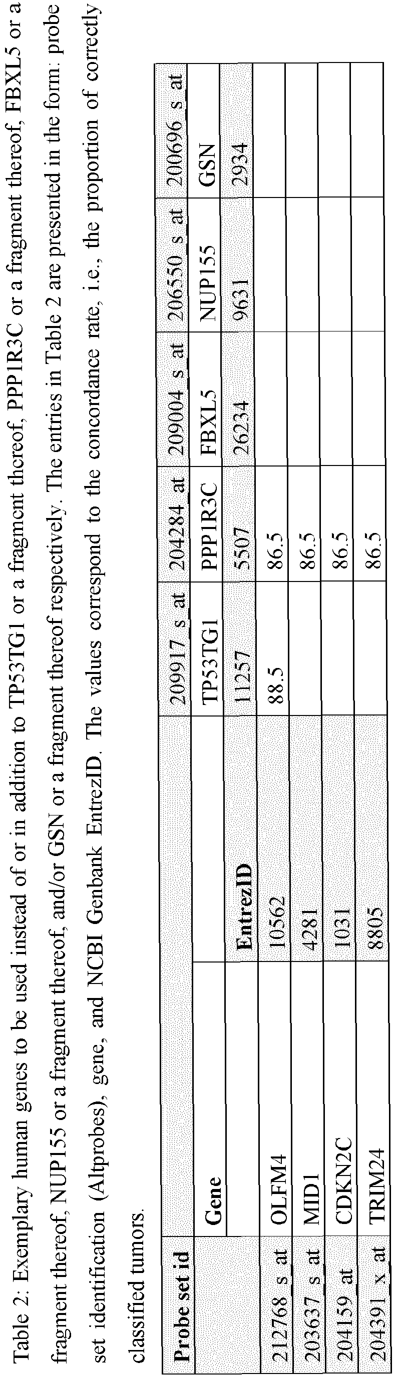

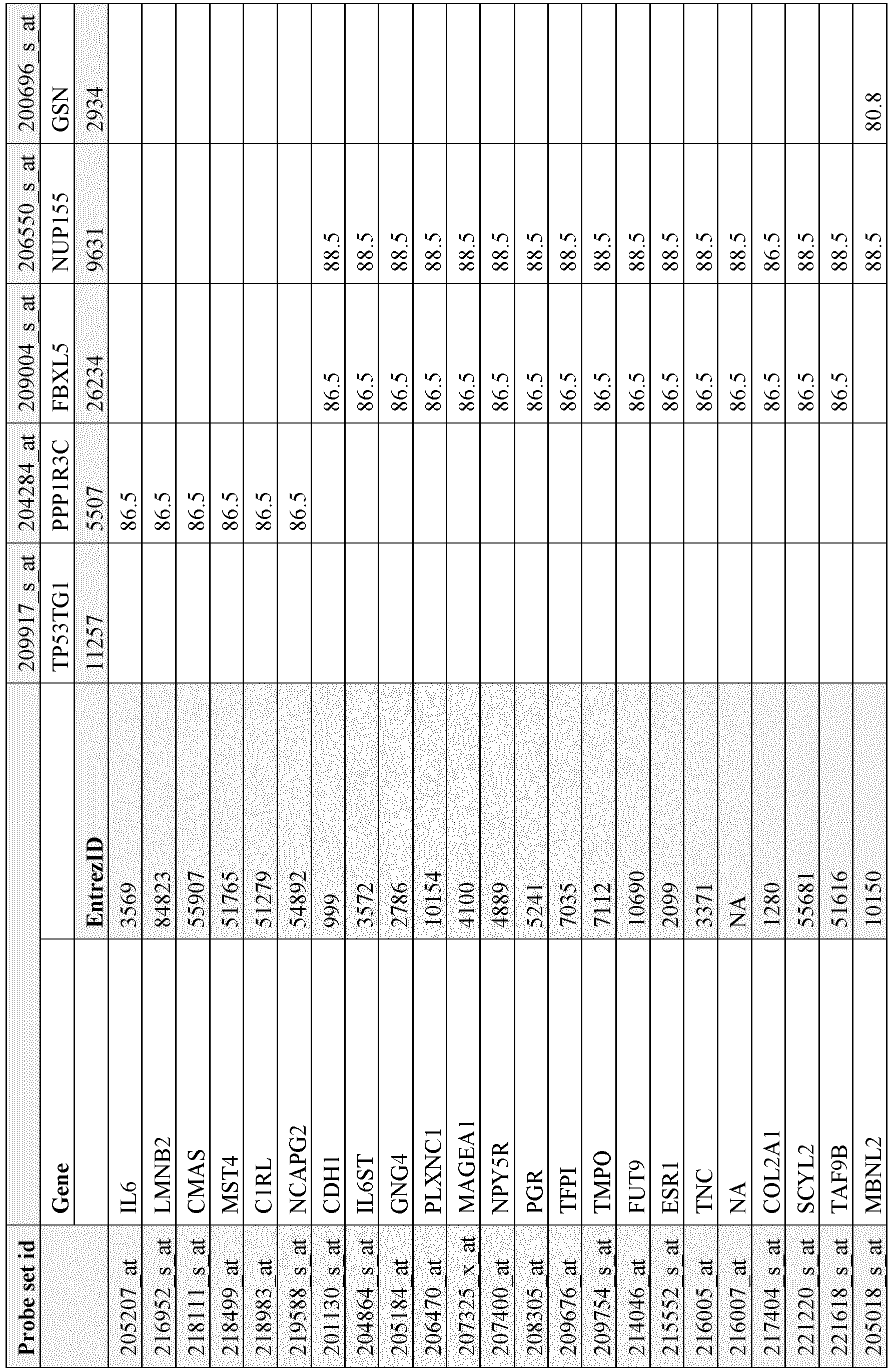

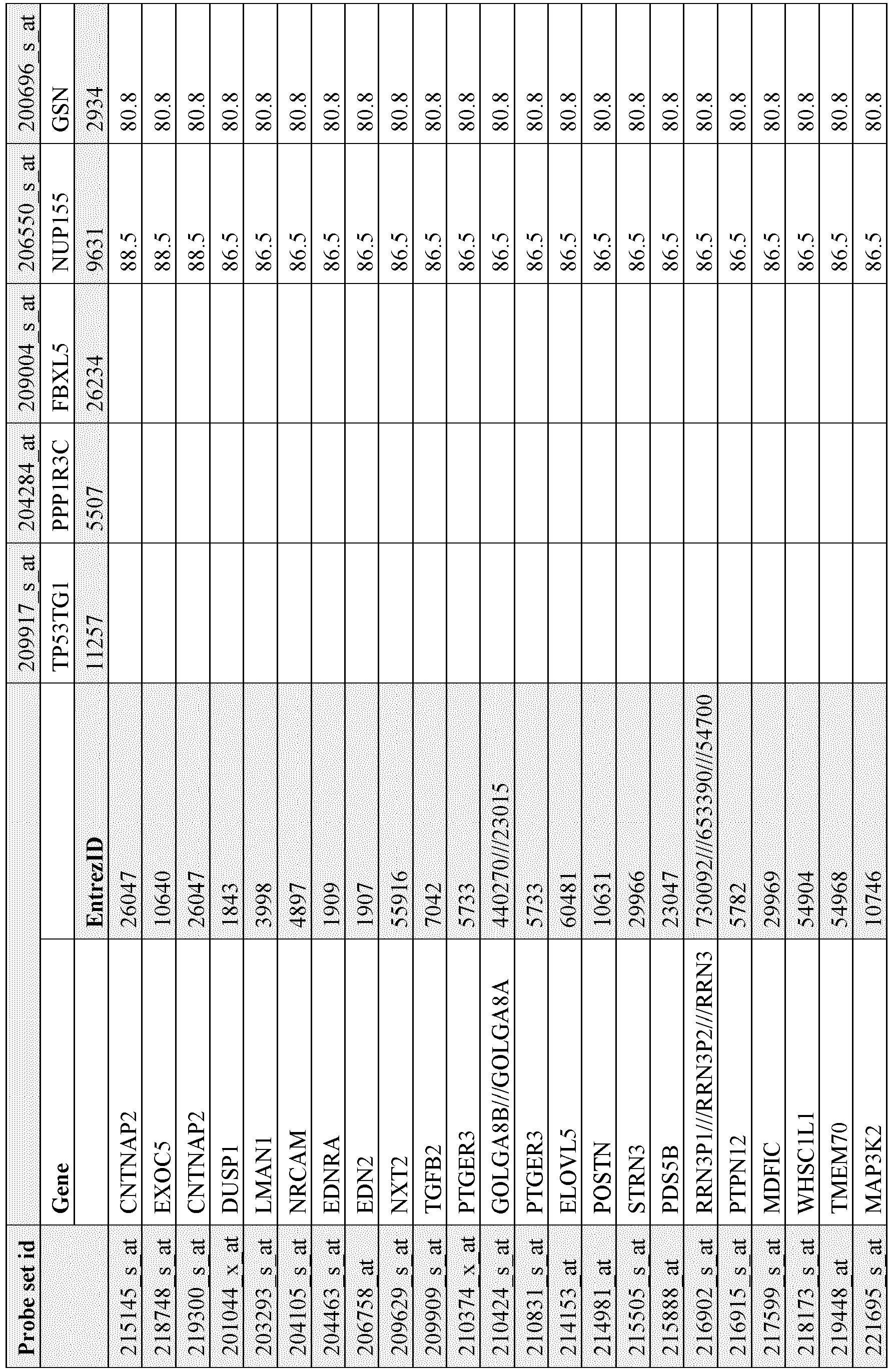

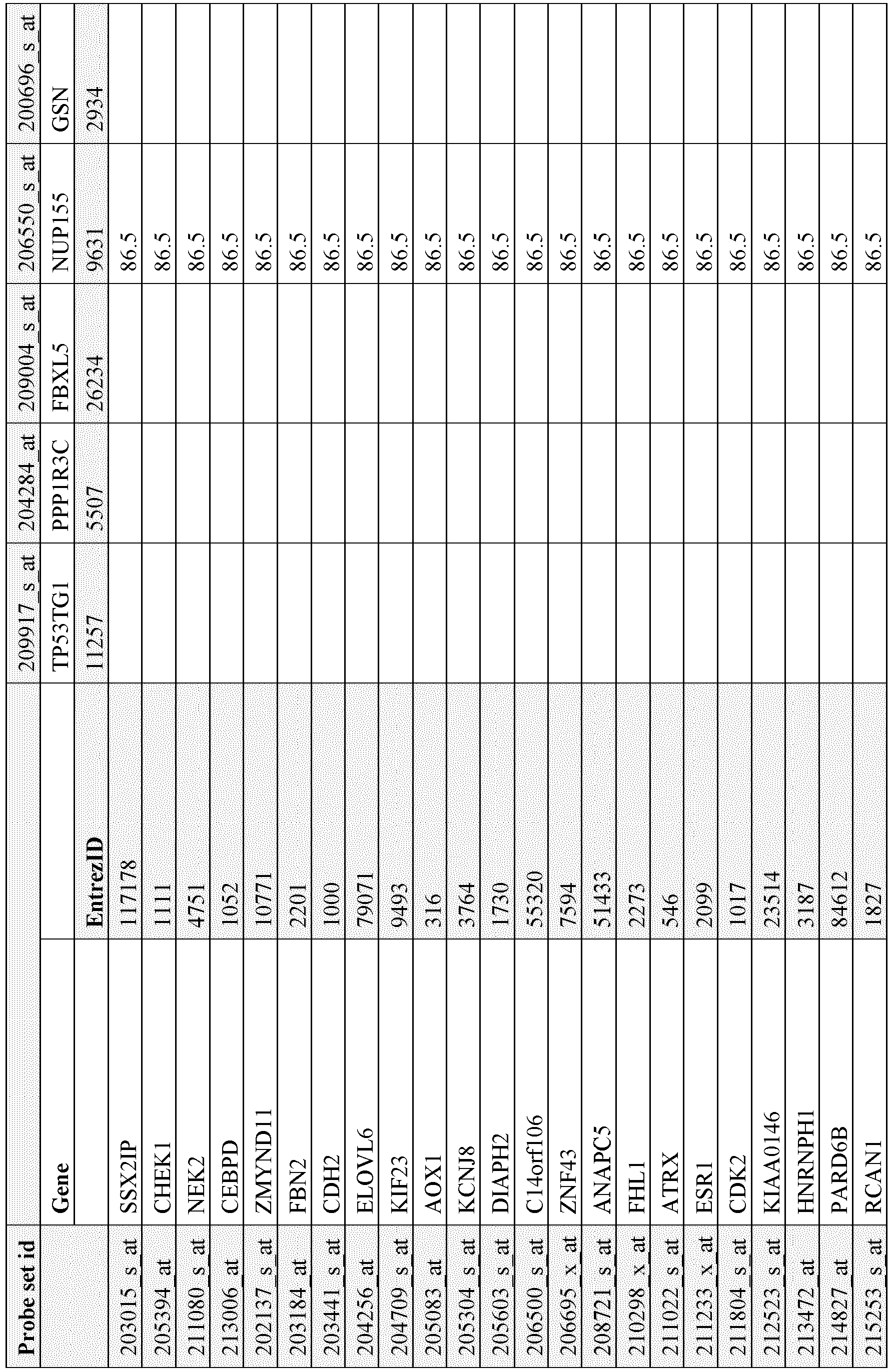

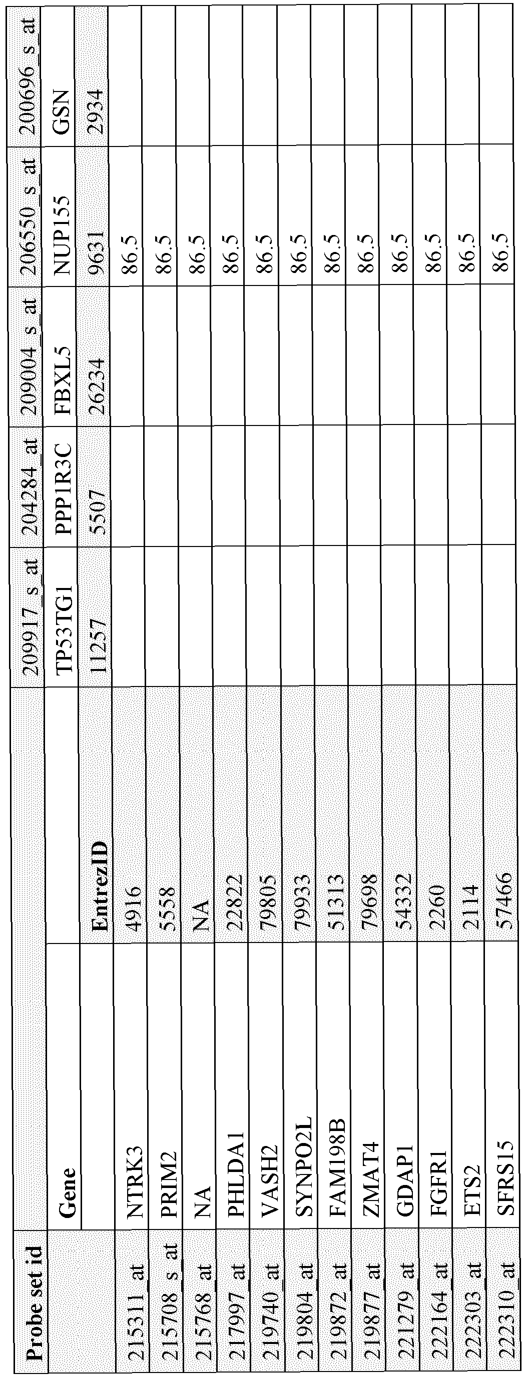

In certain embodiments, the method comprises providing three reference profiles of the level of expression of the 11 genes or fragments thereof as taught herein:
(i) a first reference profile representing CDK4 modification profile 1 comprising no phosphorylation on threonine at amino acid position 172 of CDK4;
(ii) a second reference profile representing CDK4 modification profile 2 comprising a first modified form of CDK4 which is phosphorylated on threonine at amino acid position 172 and a second modified form of CDK4, wherein the abundance of the first modified form of CDK4 is equal to or more than 90% of the abundance of the second modified form of CDK4; and
(iii) a third reference profile representing CDK4 modification profile 3 comprising a first modified form of CDK4 which is phosphorylated on threonine at amino acid position 172 and a second modified form of CDK4, wherein the abundance of the first modified form of CDK4 is less than 90% of the abundance of the second modified form of CDK4.

In certain embodiments of the methods, uses, or products, as taught herein, a reference profile may comprise or consist of the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C. In an embodiment, a reference profile may comprise or consist of the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, as provided in Table 5.

In certain embodiments of the methods, uses, or products, as taught herein, the reference profiles may be provided as standards.

In certain embodiments of the methods, uses, or products, as taught herein, the reference profiles may be obtained from reference samples. In certain embodiments, the reference profiles may be obtained from reference samples which are obtained from reference subjects. A reference subject or group of reference subjects may have or may be known to have CDK4 modification profile 1, 2, or 3. A reference subject or group of reference subjects may be or may be known to be insensitive to the CDK4/6 inhibitor; therapeutically sensitive to the CDK4/6 inhibitor; or sensitive to the CDK4/6 inhibitor but without having therapeutic benefit over classical treatment by surgery and hormone therapy, such as anti-estrogen therapy.

In certain embodiments, the step of providing three reference profiles of the level of expression of the 11 genes or fragments thereof may comprise the steps of: providing a plurality of reference samples from a plurality of reference subjects having CDK4 modification profile 1, CDK4 modification profile 2, or CDK4 modification profile 3; establishing a gene expression profile of the 11 genes or fragments thereof, for each of said reference samples individually; clustering said individual reference profiles according to a statistical procedure (such as for example centroid-based clustering); and assigning CDK4 modification profile 1, CDK4 modification profile 2, or CDK4 modification profile 3 to a cluster.

The term "clustering" or "cluster analysis" as used herein refers to the task or activity of grouping (assembling) a set of objects (such as levels of gene expression) in such a way that objects in the same group (cluster) are more similar (in some sense or another) to each other than to those in other groups (clusters).

The term "cluster" refers to a group or number of the same or similar items, gathered or occurring closely together based on similarity of characteristics.

The process of clustering used in a method of the present invention may be any mathematical process known to compare items for similarity in characteristics, attributes, properties, qualities, effects, parameters, etc.. Statistical analysis, such as for instance multivariance analysis, or other methods of analysis may be used. Preferably methods of analysis such as self-organizing maps, hierarchical clustering, multidimensional scaling, principle component analysis, supervised learning, k-nearest neighbors, and support vector machines.

In an embodiment, the step of clustering may be performed according to centroid-based clustering or *k*-means clustering.

The terms "*k*-means clustering" or "centroid-based clustering" generally refers to a method of vector quantization. In centroid-based clustering, clusters are represented by a central vector, which may not necessarily be a member of the data set. When the number of clusters is fixed to *k, k-*means clustering gives a formal definition as an optimization problem: find the *k* cluster centers and assign the objects to the nearest cluster center, such that the squared distances from the cluster are minimized.

In certain embodiments, each reference profile may comprise a reference centroid for each gene of the gene expression signature. Hence, in certain embodiments, each reference profile may comprise 11 reference centroids.

In certain embodiments, the method comprises correlating the level of expression of the 11 genes or fragments thereof in the sample with the level of expression of the genes in the three reference profiles.

The terms "correlating" or "determining the correlation" can be used interchangeably herein and refers to the task or activity of comparing two variables or two sets of data.

The term "correlation" generally refers to the extent to which two variables or two sets of data have a relationship (such as a linear relationship) with each other.

In certain embodiments, the method comprises correlating the gene expression profile of the 11 genes or fragments thereof in the sample with the gene expression profiles of the genes in the three reference profiles.

The process of correlating used in a method of the present invention may be any mathematical process known to compare two variables or two sets of data. Statistical analysis, such as for instance multivariance analysis, or other methods of analysis may be used.

In certain embodiments of the methods or uses as taught herein, the step of correlating the level of expression of the 11 genes or fragments thereof in the sample with the level of expression of the genes in the three reference profiles may be performed using Spearman rank correlation, Euclidean distance; Manhattan distance; Average dot product; Pearson correlation; Pearson uncentered; Pearson squared; Cosine correlation; Covariance value; Kedall's Tau; or Mutual information.

In certain embodiments, the method may comprise correlating the level of expression of each of the 11 genes or fragments thereof in the sample with the level of expression of the corresponding reference centroids in the reference profile.

In certain embodiments, the method comprises identifying the reference profile which provides the highest correlation with the levels of expression of the 11 genes or fragments thereof in the sample. In certain embodiments, the method further comprises inferring from the identification of the reference profile which provides the highest correlation with the level of expression of the 11 genes or fragments thereof in the sample that the subject has the CDK4 modification profile corresponding to the CDK4 modification profile of the identified reference profile.

In certain embodiments, the method may comprise inferring from the finding that:
(i) reference profile 1 provides the highest correlation with the level of expression of the 11 genes or fragments thereof in the sample that the subject has CDK4 modification profile 1;
(ii) reference profile 2 provides the highest correlation with the level of expression of the 11 genes or fragments thereof in the sample that the subject has CDK4 modification profile 2; or
(iii) reference profile 3 provides the highest correlation with the level of expression of the 11 genes or fragments thereof in the sample that the subject has CDK4 modification profile 3.

As corroborated by the experimental section, which illustrates certain representative embodiments of the invention, the inventors found that the CDK4 modification profile in a subject corresponds to a particular sensitivity of the subject to a CDK4/6 inhibitor.

Accordingly; in certain embodiments of the methods or uses as taught herein, the CDK4 modification profile corresponds to a particular sensitivity of the subject to a CDK4/6 inhibitor, wherein:
- the CDK4 modification profile 1 corresponds to insensitivity of the subject to the CDK4/6 inhibitor;
- the CDK4 modification profile 2 corresponds to therapeutic sensitivity of the subject to the CDK4/6 inhibitor; and
- the CDK4 modification profile 3 corresponds to sensitivity of the subject to the CDK4/6 inhibitor but without clinical benefit over classical treatment by surgery and hormone (e.g., anti-estrogen) therapy.

As mentioned above, the CDK4 modification profile may be suitably determined by measuring (e.g., by mass spectrometry or by 2D gel electrophoresis in combination with Western blotting) the abundance of the first modified form of CDK4 and the second modified form of CDK4, calculating the ratio of the abundance of the first modified form to the abundance of the second modified form, and attributing to the subject CDK4 modification profile 1 when the ratio is below 0.1; attributing to the subject CDK4 modification profile 2 when the ratio is above 0.9; or attributing to the subject CDK4 modification profile 3 when the ratio is between 0.1 and 0.9.

Hence, also provided herein is a method for determining sensitivity to a CDK4/6 inhibitor in a human subject having breast cancer, the method comprising the steps of:
- determining in a sample from the subject the CDK4 modification profile, wherein the profile is:
   (i) CDK4 modification profile 1 comprising no phosphorylation on threonine at amino acid position 172 of CDK4;
   (ii) CDK4 modification profile 2 comprising a first modified form of CDK4 which is phosphorylated on threonine at amino acid position 172 and a second modified form of CDK4, wherein the abundance of the first modified form of CDK4 is equal to or more than 90% of the abundance of the second modified form of CDK4; or
   (iii) CDK4 modification profile 3 comprising a first modified form of CDK4 which is phosphorylated on threonine at amino acid position 172 and a second modified form of CDK4, wherein the abundance of the first modified form of CDK4 is less than 90% of the abundance of the second modified form of CDK4; and
- inferring from the finding that the CDK4 modification profile in the sample is:
   (i) CDK4 modification profile 1, that the subject is insensitive to the CDK4/6 inhibitor,
   (ii) CDK4 modification profile 2, that the subject is therapeutically sensitive to the CDK4/6 inhibitor, or
   (iii) CDK4 modification profile 3, that the subject is sensitive to the CDK4/6 inhibitor but has no clinical benefit from treatment with the CDK4/6 inhibitor.

A further aspect of the invention provides a method for determining sensitivity to a CDK4/6 inhibitor in a human subject having breast cancer, the method comprising the steps of:
- determining in a sample from the subject the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C;
- providing three reference profiles of the level of expression of the 11 genes or fragments thereof: a first reference profile representing insensitivity to the CDK4/6 inhibitor; a second reference profile representing therapeutic sensitivity to the CDK4/6 inhibitor; and a third reference profile representing sensitivity to the CDK4/6 inhibitor but without therapeutic benefit over classical treatment by surgery and hormone therapy;
- correlating the level of expression of the 11 genes or fragments thereof in the sample with the level of expression of the 11 genes or fragments thereof in the three reference profiles;
- identifying the reference profile which provides the highest correlation with the level of expression of the 11 genes or fragments thereof in the sample; and
- inferring from the finding that:
   (i) the first reference profile provides the highest correlation with the level of expression of the 11 genes or fragments thereof in the sample, that the subject is insensitive to the CDK4/6 inhibitor;
   (ii) the second reference profile provides the highest correlation with the level of expression of the 11 genes or fragments thereof in the sample, that the subject is therapeutically sensitive to the CDK4/6 inhibitor; or
   (iii) the third reference profile provides the highest correlation with the level of expression of the 11 genes or fragments thereof in the sample, that the subject is sensitive to the CDK4/6 inhibitor but has no clinical benefit from treatment with the CDK4/6 inhibitor.

In certain embodiments, the method comprises providing three reference profiles of the level of expression of the 11 genes or fragments thereof: a first reference profile representing insensitivity to the CDK4/6 inhibitor; a second reference profile representing therapeutic sensitivity to the CDK4/6 inhibitor; and a third reference profile representing sensitivity to the CDK4/6 inhibitor but without therapeutic benefit over treatment by surgery and hormone therapy, such as anti-estrogen therapy.

In certain embodiments, the step of providing three reference profiles of the level of expression of the 11 genes or fragments thereofmay comprise the steps of: providing a plurality of reference samples from a plurality of reference subjects being insensitive to the CDK4/6 inhibitor; therapeutically sensitive to the CDK4/6 inhibitor; or sensitive to the CDK4/6 inhibitor but without having therapeutic benefit over classical treatment by surgery and hormone therapy; establishing a gene expression profile of the 11 genes or fragments thereof, for each of said reference samples individually; clustering said individual reference profiles according to a statistical procedure (such as for example centroid-based clustering); and assigning insensitivity to the CDK4/6 inhibitor; therapeutically sensitivity to the CDK4/6 inhibitor; or sensitivity to the CDK4/6 inhibitor but without therapeutic benefit over classical treatment by surgery and hormone therapy, to a cluster. In certain embodiments, when the second reference profile provides the highest correlation with the level of expression of the 11 genes or fragments thereof in the sample, the subject is therapeutically sensitive to the CDK4/6 inhibitor and the subject may require immediate treatment with the CDK4/6 inhibitor.

In certain embodiments, when the third reference profile provides the highest correlation with the level of expression of the 11 genes or fragments thereof in the sample, the subject is sensitive to the CDK4/6 inhibitor but has no additional clinical benefit from treatment with the CDK4/6 inhibitor compared to standard care, such as surgery and hormone (e.g., anti-estrogen) therapy.

In the disclosure herein, the method may comprise determining the level of expression of olfactomedin 4 (OLFM4) or a fragment thereof in a sample from the subject instead of or in addition to determining the level of expression of TP53TG1 or a fragment thereof in a sample from the subject.

In the disclosure herein, the method may comprise determining the level of expression of a gene or a fragment thereof selected from the group consisting of olfactomedin 4 (OLFM4), midline 1 (MIDI), cyclin-dependent kinase inhibitor 2C (CDKN2C), tripartite motif containing 24 (TRIM24), interleukin 6 (IL6), lamin B2 (LMNB2), cytidine monophosphate N-acetylneuraminic acid synthetase (CMAS), serine/threonine protein kinase 26 (MST4), complement C1r subcomponent like (C1RL), and non-SMC condensin II complex subunit G2 (NCAPG2), in a sample from the subject instead of or in addition to determining the level of expression of PPP1R3C or a fragment thereof in a sample from the subject.

In the disclosure herein, the method may comprise determining the level of expression of a gene or a fragment thereof selected from the group consisting of cadherin 1(CDH1), interleukin 6 signal transducer (IL6ST), G protein subunit gamma 4 (GNG4), plexin C1 (PLXNC1), MAGE family member A1 (MAGEA1), neuropeptide Y receptor Y5 (NPY5R), progesterone receptor (PGR), tissue factor pathway inhibitor (TFPI), thymopoietin (TMPO), fucosyltransferase 9 (FUT9), estrogen receptor 1 (ESR1), tenascin C (TNC), Homo sapiens clone 24457 mRNA sequence (216007_at), collagen type II alpha 1 (COL2A1), SCY1 like pseudokinase 2 (SCYL2), and TATA-box binding protein associated factor 9b (TAF9B), in a sample from the subject instead of or in addition to determining the level of expression of FBXL5 or a fragment thereof in a sample from the subject.

In the disclosure herein, the method may comprise determining the level of expression of a gene or a fragment thereof selected from the group consisting of cadherin 1 (CDH1), interleukin 6 signal transducer (IL6ST), G protein subunit gamma 4 (GNG4), plexin C1 (PLXNC1), MAGE family member A1 (MAGEA1), neuropeptide Y receptor Y5 (NPY5R), progesterone receptor (PGR), tissue factor pathway inhibitor (TFPI), thymopoietin (TMPO), fucosyltransferase 9 (FUT9), estrogen receptor 1 (ESR1), tenascin C (TNC), Homo sapiens clone 24457 mRNA sequence (216007_at), collagen type II alpha 1 (COL2A1), SCY1 like pseudokinase 2 (SCYL2), and TATA-box binding protein associated factor 9b (TAF9B), muscleblind like splicing regulator 2 (MBNL2), cytidine monophosphate N-acetylneuraminic acid synthetase (CNTNAP2), exocyst complex component 5 (EXOC5), dual specificity phosphatase 1 (DUSP1), lectin, mannose binding 1 (LMAN1), neuronal cell adhesion molecule (NRCAM), endothelin receptor type A (EDNRA), endothelin 2 (EDN2), nuclear transport factor 2 like export factor 2 (NXT2), transforming growth factor beta 2 (TGFB2), prostaglandin E receptor 3 (PTGER3), golgin A8 family member B (GOLGA8B)/ golgin A8 family member A (GOLGA8A), ELOVL fatty acid elongase 5 (ELOVL5), periostin (POSTN), striatin 3 (STRN3), PDS5 cohesin associated factor B (PDS5B), RRN3 RNA polymerase I transcription factor homolog pseudogene 1 (RRN3P1)/ RRN3 RNA polymerase I transcription factor homolog pseudogene 2 (RRN3P2)/ RRN3 RNA polymerase I transcription factor homolog (RRN3), protein tyrosine phosphatase, non-receptor type 12 (PTPN12), MyoD family inhibitor domain containing (MDFIC), Wolf-Hirschhom syndrome candidate 1-like 1 (WHSC1L1), transmembrane protein 70 (TMEM70), mitogen-activated protein kinase kinase kinase 2 (MAP3K2), synovial sarcoma, X breakpoint 2 interacting protein (SSX2IP), checkpoint kinase 1 (CHEK1), NIMA related kinase 2 (NEK2), CCAAT/enhancer binding protein delta (CEBPD), zinc finger MYND-type containing 11 (ZMYND11), fibrillin 2 (FBN2), cadherin 2 (CDH2), ELOVL fatty acid elongase 6 (ELOVL6), kinesin family member 23 (KIF23), aldehyde oxidase 1 (AOX1), potassium voltage-gated channel subfamily J member 8 (KCNJ8), diaphanous related formin 2 (DIAPH2), MIS 18 binding protein 1 (C14orf106), zinc finger protein 43 (ZNF43), anaphase promoting complex subunit 5 (ANAPC5), four and a half LIM domains 1 (FHL1), alpha thalassemia/mental retardation syndrome X-linked (ATRX), cyclin-dependent kinase 2 (CDK2), scaffolding protein involved in DNA repair (KIAA0146), heterogeneous nuclear ribonucleoprotein H1 (H) (HNRNPH1), par-6 family cell polarity regulator beta (PARD6B), regulator of calcineurin 1 (RCAN1), neurotrophic receptor tyrosine kinase 3 (NTRK3), primase (DNA) subunit 2 (PRIM2), cDNA DKFZp564P016 (215768_at), pleckstrin homology like domain family A member 1 (PHLDA1), vasohibin 2 (VASH2), synaptopodin 2 like (SYNPO2L), family with sequence similarity 198 member B (FAM198B), zinc finger matrin-type 4 (ZMAT4), ganglioside induced differentiation associated protein 1 (GDAP1), fibroblast growth factor receptor 1 (FGFR1), ETS proto-oncogene 2 (ETS2), and SR-related CTD associated factor 4 (SFRS15), in a sample from the subject instead of or in addition to determining the level of expression of NUP155 or a fragment thereof in a sample from the subject.

In the disclosure herein, the method may comprise determining the level of expression of a gene or a fragment thereof selected from the group consisting of muscleblind like splicing regulator 2 (MBNL2), cytidine monophosphate N-acetylneuraminic acid synthetase (CNTNAP2), exocyst complex component 5 (EXOC5), dual specificity phosphatase 1 (DUSP1), lectin, mannose binding 1 (LMAN1), neuronal cell adhesion molecule (NRCAM), endothelin receptor type A (EDNRA), endothelin 2 (EDN2), nuclear transport factor 2 like export factor 2 (NXT2), transforming growth factor beta 2 (TGFB2), prostaglandin E receptor 3 (PTGER3), golgin A8 family member B (GOLGA8B)/ golgin A8 family member A (GOLGA8A), ELOVL fatty acid elongase 5 (ELOVL5), periostin (POSTN), striatin 3 (STRN3), PDS5 cohesin associated factor B (PDS5B), RRN3 RNA polymerase I transcription factor homolog pseudogene 1 (RRN3P1)/ RRN3 RNA polymerase I transcription factor homolog pseudogene 2 (RRN3P2)/ RRN3 RNA polymerase I transcription factor homolog (RRN3), protein tyrosine phosphatase, non-receptor type 12 (PTPN12), MyoD family inhibitor domain containing (MDFIC), Wolf-Hirschhom syndrome candidate 1-like 1 (WHSC1L1), transmembrane protein 70 (TMEM70), and mitogen-activated protein kinase kinase kinase 2 (MAP3K2), in a sample from the subject instead of or in addition to determining the level of expression of GSN or a fragment thereof in a sample from the subject.

The method of the disclosure may comprise:
- determining the level of expression of OLFM4 or a fragment thereof in a sample from the subject instead of or in addition to determining the level of expression of TP53TG1 or a fragment thereof in a sample from the subject; and/or
- determining the level of expression of a gene or a fragment thereof selected from the group consisting of OLFM4, MIDI, CDKN2C, TRIM24, IL6, LMNB2, CMAS, MST4, C1RL, and NCAPG2, in a sample from the subject instead of or in addition to determining the level of expression of PPP1R3C or a fragment thereof in a sample from the subject; and/or
- determining the level of expression of a gene or a fragment thereof selected from the group consisting of CDH1, IL6ST, GNG4, PLXNC1, MAGEA1, NPY5R, PGR, TFPI, TMPO, FUT9, ESR1, TNC, 216007_at, COL2A1, SCYL2, and TAF9B, in a sample from the subject instead of or in addition to determining the level of expression of FBXL5 or a fragment thereof in a sample from the subject; and/or
- determining the level of expression of a gene or a fragment thereof selected from the group consisting of CDH1, IL6ST, GNG4, PLXNC1, MAGEA1, NPY5R, PGR, TFPI, TMPO, FUT9, ESR1, TNC, 216007at, COL2A1, SCYL2, TAF9B, MBNL2, CNTNAP2, EXOC5, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, MAP3K2, SSX2IP, CHEK1, NEK2, CEBPD, ZMYND11, FBN2, CDH2, ELOVL6, KIF23, AOX1, KCNJ8, DIAPH2, C14orf106, ZNF43, ANAPC5, FHL1, ATRX, CDK2, KIAA0146, HNRNPH1, PARD6B, RCAN1, NTRK3, PRIM2, 215768at, PHLDA1, VASH2, SYNPO2L, FAM198B, ZMAT4, GDAP1, FGFR1, ETS2, and SFRS15, in a sample from the subject instead of or in addition to determining the level of expression of NUP155 or a fragment thereof in a sample from the subject; and/or
- determining the level of expression of a gene or a fragment thereof selected from the group consisting of MBNL2, CNTNAP2, EXOC5, CNTNAP2, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, and MAP3K2, in a sample from the subject instead of or in addition to determining the level of expression of GSN or a fragment thereof in a sample from the subject.

Table 2 provides exemplary human genes to be used instead of or in addition to TP53TG1 or a fragment thereof, PPP1R3C or a fragment thereof, FBXL5 or a fragment thereof, NUP155 or a fragment thereof, and/or GSN or a fragment thereof.

The method of the disclosure may comprise determining the level of expression of a gene or a fragment thereof in a sample from the subject instead of or in addition to determining the level of expression of another gene or a fragment thereof, in a sample from the subject, as provided in Table 2.

A reference profile of the disclosure may comprise the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, wherein the reference profile may comprise:
- the level of expression of OLFM4 or a fragment thereof instead of or in addition to the level of expression of TP53TG1 or a fragment thereof; and/or
- the level of expression of a gene or a fragment thereof selected from the group consisting of OLFM4, MIDI, CDKN2C, TRIM24, IL6, LMNB2, CMAS, MST4, C1RL, and NCAPG2, instead of or in addition to the level of expression of PPP1R3C or a fragment thereof; and/or
- the level of expression of a gene or a fragment thereof selected from the group consisting of CDH1, IL6ST, GNG4, PLXNC1, MAGEA1, NPY5R, PGR, TFPI, TMPO, FUT9, ESR1, TNC, 216007_at, COL2A1, SCYL2, and TAF9B, instead of or in addition to the level of expression of FBXL5 or a fragment thereof; and/or
- the level of expression of a gene or a fragment thereof selected from the group consisting of CDH1, IL6ST, GNG4, PLXNC1, MAGEA1, NPY5R, PGR, TFPI, TMPO, FUT9, ESR1, TNC, 216007_at, COL2A1, SCYL2, TAF9B, MBNL2, CNTNAP2, EXOC5, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, MAP3K2, SSX2IP, CHEK1, NEK2, CEBPD, ZMYND11, FBN2, CDH2, ELOVL6, KIF23, AOX1, KCNJ8, DIAPH2, C14orf106, ZNF43, ANAPC5, FHL1, ATRX, CDK2, KIAA0146, HNRNPH1, PARD6B, RCAN1, NTRK3, PRIM2, 215768at, PHLDA1, VASH2, SYNPO2L, FAM198B, ZMAT4, GDAP1, FGFR1, ETS2, and SFRS15, instead of or in addition to the level of expression of NUP155 or a fragment thereof; and/or
- the level of expression of a gene or a fragment thereof selected from the group consisting of MBNL2, CNTNAP2, EXOC5, CNTNAP2, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, and MAP3K2, instead of or in addition to the level of expression of GSN or a fragment thereof.

In certain embodiments of the methods, uses, or products as taught herein, the subject may be treated by surgery, optionally by a chemotherapeutic agent and/or by radiotherapy, if the subject is insensitive to the CDK4/6 inhibitor.

The surgery may be performed as known in the art, e.g. as known to a surgeon.

The term "chemotherapeutic agent" refers to any pharmacologic agent that is known to be of use in the treatment of proliferative diseases.

In certain embodiments, the chemotherapeutic agent may be a chemotherapeutic agent useful in the treatment of breast cancer.

In certain embodiments, the chemotherapeutic agent may be an alkylating agent, a cytotoxic compound, an anti-metabolite, a plant alkaloid, a terpenoid, or a topoisomerase inhibitor.

The term "alkylating agent" generally refers to an agent capable to alkylate nucleophilic functional groups under physiological conditions.

Exemplary alkylating agents include but are not limited to cyclophosphamide, carmustine, cisplatin, carboplatin, oxaliplatin, mechlorethamine, melphalan (hydrochloride), chlorambucil, ifosfamide, and busulfan.

The term "cytotoxic compound" generally refers to an agent toxic to a cell.

Exemplary cytotoxic compound include but are not limited to actinomycin (also known as dactinomycin); anthracyclines such as doxorubicin, daunorubicin, valrubicin, idarubicin, and epirubicin; bleomycin; plicamycin; mitoxantrone; and mitomycin.

The term "anti-metabolite" generally refers to an agent capable to inhibit the use of a metabolite such as purines or pyrimidines. Anti-metabolites prevent purines and pyrimidines from becoming incorporated into DNA during the S phase of the cell cycle and thereby stop normal development and division.

Exemplary anti-metabolites include but are not limited to azathioprine, fluorouracil, mercaptopurine, methotrexate, nelarabine, and pemetrexed.

Plant alkaloids and terpenoids are derived from plants and block cell division by preventing microtubule function. Non-limiting examples include vinca alkaloids and taxanes.

Exemplary vinca alkaloids include but are not limited to vincristine, vinblastine, vinorelbine, and vindesine.

Exemplary taxanes include but are not limited to paclitaxel, and docetaxel.

The term "topoisomerase inhibitor" generally refers to enzymes that maintain the topology of DNA. Non-limiting examples include type I and type II topoisomerase inhibitors.

Exemplary type I topoisomerase inhibitors include camptothecins such as irinotecan and topotecan. Exemplary type II topoisomerase inhibitors include amsacrine, etoposide, etoposide phosphate, and teniposide.

In certain embodiments, the chemotherapeutic agent, may be selected from the group consisting of cyclophosphamide, adriamycin, doxorubicin, idarubicin, mitoxantrone, oxaliplatin, bortezomib, digoxin, digitoxin, hypericin, shikonin, wogonin, sorafenib, everolimus, imatinib, geldanamycin, panobinostat, carmustine, cisplatin, carboplatin, mechlorethamine, melphalan (hydrochloride), chlorambucil, ifosfamide, busulfan, actinomycin, daunorubicin, valrubicin, epirubicin, bleomycin, plicamycin, mitoxantrone, mitomycin, azathioprine, mercaptopurine, fluorouracil, methotrexate, nelarabine, pemetrexed, vincristine, vinblastine, vinorelbine, vindesine, paclitaxel, docetaxel, irinotecan, topotecan, amsacrine, etoposide, etoposide phosphate, teniposide, anastrozole, exemestane, bosutinib, irinotecan, vandetanib, bicalutamide, lomustine, clofarabine, cabozantinib, cytarabine, cytoxan, decitabine, dexamethasone, hydroxyurea, decarbazine, leuprolide, epirubicin, asparaginase, estramustine, vismodegib, amifostine, flutamide, toremifene, fulvestrant, letrozole, degarelix, fludarabine, pralatrexate, floxuridine, gemcitabine, carmustine wafer, eribulin, altretamine, topotecan, axitinib, gefitinib, romidepsin, ixabepilone, ruxolitinib, cabazitaxel, carfilzomib, chlorambucil, sargramostim, cladribine, leuprolide, mitotane, procarbazine, megestrol, mesna, strontium-89 chloride, mitomycin, filgrastim, pegfilgrastim, sorafenib, nilutamide, pentostatin, tamoxifen, pegaspargase, denileukin diftitox, alitretinoin, carboplatin, prednisone, aldesleukin, mercaptopurine, zoledronic acid, lenalidomide, interferon alfa-2a, octreotide, dasatinib, regorafenib, histrelin, sunitinib, peginterferon alfa-2b, omacetaxine, thioguanine, erlotinib, bexarotene, decarbazine, temozolomide, thiotepa, thalidomide, BCG, temsirolimus, bendamustine hydrochloride, triptorelin, arsenic trioxide, lapatinib, valrubicin intravesical, tretinoin, azacitidine, pazopanib, teniposide, leucovorin, crizotinib, capecitabine, enzalutamide, ziv-aflibercept, streptozocin, vemurafenib, goserelin, vorinostat, zoledronic acid, and abiraterone.

In certain embodiments of the methods or uses, as taught herein, the chemotherapeutic agent, may be selected from cyclophosphamide, adriamycin, epirubicin, fluorouracil, paclitaxel, docetaxel, capecitabine, cisplatin, carboplatin, and methotrexate, or a combination thereof, such as a combination of one or more thereof, such as a combination of two, three, four, five, or more thereof.

In certain embodiments, the chemotherapeutic agent may be a biomolecule, such as preferably a protein, (poly)peptide, peptide, nucleic acid, or small molecule (such as primary metabolite, secondary metabolite, or natural product), or a combination of any two, any three, or any four thereof. Non-limiting examples of suitable biomolecules are humanized antibodies.

In certain embodiments, the chemotherapeutic agent may be a therapeutic proteins, peptides, nucleic acids, or (poly)peptides, in particular interleukins, cytokines, anti-cytokines and cytokines receptors, vaccines, interferons, tumour necrosis factors (TNFs), enzymes, or antibodies.

In certain embodiments, the chemotherapeutic agent may be one or more anti-cancer or anti-tumour therapeutic antibodies. In certain embodiments, the anti-cancer or anti-tumour therapeutic antibody may be modified for delivery of a toxin, radioisotope, cytokine or other active conjugate. In certain embodiments, the anti-cancer or anti-tumour therapeutic antibody may be a bispecific antibody that can bind with its Fab region both to target antigen and to a conjugate or effector cell. In certain embodiments, the antineoplastic agent may be a monoclonal anti-cancer therapeutic antibody.

In certain embodiments, the chemotherapeutic agent, in particular the anti-cancer therapeutic antibody, may be selected from the group consisting of trastuzumab (Herceptin), Trastuzumabemtansine, Ertumaxomab, Glembatumumab vedotin, Adecatumumab, Pertuzumab, Cetuximab, panitumumab.

In certain embodiments of the methods, uses, or products as taught herein, the subject may be treated by administration of the CDK4/6 inhibitor, if the subject is therapeutically sensitive to the CDK4/6 inhibitor. In certain embodiments of the methods, uses, or products as taught herein, the subject is to be treated by administration of the CDK4/6 inhibitor, optionally in combination with hormone therapy as taught herein, if the subject is therapeutically sensitive to the CDK4/6 inhibitor. In certain embodiments of the methods, uses, or products as taught herein, the subject is to be treated by administration of the CDK4/6 inhibitor, optionally in combination with letrozole, if the subject is therapeutically sensitive to the CDK4/6 inhibitor. In certain embodiments of the methods, uses, or products as taught herein, the subject is to be treated by administration of the CDK4/6 inhibitor, optionally in combination with any other suitable therapy, such as in combination with cell signaling inhibitors including inhibitors of MEK, PI3K, mTOR, or RAF, if the subject is therapeutically sensitive to the CDK4/6 inhibitor.

In certain embodiments of the methods, uses, or products as taught herein, the subject may be treated by surgery and hormone therapy, if the subject is sensitive to the CDK4/6 inhibitor but has no clinical benefit from the treatment with the CDK4/6 inhibitor. In certain embodiments, when there is a relapse of the subject after treatment by surgery and hormone therapy, the methods as taught herein may be repeated to determine sensitivity to a CDK4/6 inhibitor.

In certain embodiments, the hormone therapy may be selected from the group consisting of estrogen receptor antagonists (such as Tamoxifen, toremifen (Foresta), raloxifene, ormeloxifene, clomifen, lasofoxifene, or ospemifene); estrogen receptor degrader (such as fulvestrant (Faslodex)); aromatase inhibitors (such as letrozole (Femara), Anastrozole (Arimidex), exemestane (Aromasin)); and luteinising hormone blockers (such as goserelin (Zoladex)). Preferably, the hormone therapy may be anti-estrogen therapy.

The term "CDK4/6 inhibitor" as used herein refers to any agent that inhibits the kinase activity of CDK4 and/or CDK6, preferably of CDK4.

In certain embodiments, the CDK4/6 inhibitor may be derived from pyridopyrimidine, pyrrolopyrimidine or indolocarbazole compounds.

In certain embodiments of the methods, uses, or products as taught herein, the CDK4/6 inhibitor may be palbociclib (PD0332991), LEE-011 (ribociclib), LY2835219 (abemaciclib), G1T28-1, SHR6390, or P276-00, or a derivative of any one of palbociclib, LEE-011, LY2835219, G1T28-1, SHR6390, or P276-00. In certain preferred embodiments of the methods, uses, or products as taught herein, , the CDK4/6 inhibitor is palbociclib.

The terms "palbociclib" or "PD-0332991" (commercial or trade name Ibrance) generally refer to a selective CDK4 and CDK6 inhibitor having the Formula I.

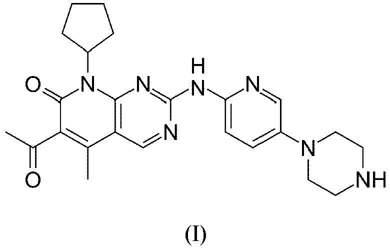

The terms "LEE-011" or "ribociclib" generally refer to a selective CDK4 and CDK6 inhibitor having CAS Number 1211441-98-3.

The terms "LY2835219" or "Abemaciclib" generally refer to an oral selective CDK4 and CDK6 inhibitor having Cas Number 1231929-97-7.

The term "G1T28-1" refers to a selective CDK4 and CDK6 inhibitor developed and tested by G1 Terapeutics Inc.

The term "SHR6390" refers to a selective CDK4 and CDK6 inhibitor developed and tested by Jiangsu HengRui Medicine Co.

The term "P276-00" refers to a selective CDK4 and CDK6 inhibitor having CAS Number 920113-03-7.

Suitable non-limiting examples of derivatives and salts include Palbociclib Isethionate (CAS Number 827022-33-3); Palbociclib hydrochloride (CAS Number 827022-32-2); Ribociclib succinate (CAS Number 1374639-75-4); Abemaciclib mesylate (CAS Number 1231930-82-7).

In certain embodiments, the CDK4/6 inhibitor may comprise or consist of antisense oligonucleotides, shRNA molecules and siRNA molecules that specifically inhibit the expression or activity of CDK4 and/or CDK6, preferably of CDK4. One non-limiting example of a CDK4 inhibitor comprises an antisense, shRNA, or siRNA nucleic acid sequence which is substantially identical (i.e., largely but not wholly identical) to the sequence of at least a portion of a CDK4 nucleic acid sequence, wherein the identity of the portion re lative to the CDK4 sequence is at least about 75%, or at least about 80%, or at least about 85%, or at least about 90%, or at least about 95%, or at least about 98%, where percent identity can be determined as known in the art, for example, BLAST or FASTA software. Sequence identity may be determined using suitable algorithms for performing sequence alignments and determination of sequence identity as *know per* se. Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x dropoff = 50, expectation value = 10.0, word size = 28; or for the BLASTP algorithm: matrix = Blosum62 (Henikoff et al., 1992, Proc. Natl. Acad. Sci., 89:10915-10919), cost to open a gap = 11, cost to extend a gap = 1, expectation value = 10.0, word size = 3).

In certain embodiments, the complementary portion may constitute at least 10 nucleotides, or at least 15 nucleotides or at least 20 nucleotides, or at least 25 nucleotides, or at least 30 nucleotides, and the antisense nucleic acid, shRNA or siRNA molecules may be up to 15 nucleotides, or up to 20 nucleotides, or up to 25 nucleotides, or up to 30 nucleotides, or up to 35 nucleotides, or up to 40 nucleotides, or up to 45 nucleotides, or up to 50 nucleotides, or up to 75 nucleotides, or up to 100 nucleotides in length.

As mentioned above, in certain embodiments, the subject is to be treated by administration of a CDK4/6 inhibitor, such as palbociclib, optionally in combination with letrozole.

The term "letrozole" (commercial name Femara) generally refers an oral non-steroidal aromatase inhibitor having CAS Number 112809-51-5.

In certain embodiments of the methods, uses, or products as taught herein, the level of expression of the 11 genes or fragments thereof may be determined by quantitative real-time PCR (qPCR), reverse transcription-qPCR (RT-qPCR), a nucleic acid microarray, digital molecular barcoding technology (nCounter Nanostring), branched DNA (bDNA) signal amplification technology (Quantigene), mass spectrometry (Sequenom), or a combination of said methods.

A further aspect provides the use of a kit of parts for determining sensitivity to a CDK4/6 inhibitor in a human subject having breast cancer, the kit comprising means for determining the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, in a sample from a human subject.

The term "kit" as used herein refers to any combination of reagents or apparatus that can be used to perform the methods as taught herein.

In the disclosure herein, a kit of parts may comprise means for determining the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, wherein the kit of parts may comprise:
- means for determining the level of expression of OLFM4 or a fragment thereof instead of or in addition to means for determining the level of expression of TP53TG1 or a fragment thereof; and/or
- means for determining the level of expression of a gene or a fragment thereof selected from the group consisting of OLFM4, MIDI, CDKN2C, TRIM24, IL6, LMNB2, CMAS, MST4, C1RL, and NCAPG2, instead of or in addition to means for determining the level of expression of PPP1R3C or a fragment thereof; and/or
- means for determining the level of expression of a gene or a fragment thereof selected from the group consisting of CDH1, IL6ST, GNG4, PLXNC1, MAGEA1, NPY5R, PGR, TFPI, TMPO, FUT9, ESR1, TNC, 216007_at, COL2A1, SCYL2, and TAF9B, instead of or in addition to means for determining the level of expression of FBXL5 or a fragment thereof; and/or
- means for determining the level of expression of a gene or a fragment thereof selected from the group consisting of CDH1, IL6ST, GNG4, PLXNC1, MAGEA1, NPY5R, PGR, TFPI, TMPO, FUT9, ESR1, TNC, 216007_at, COL2A1, SCYL2, TAF9B, MBNL2, CNTNAP2, EXOC5, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, MAP3K2, SSX2IP, CHEK1, NEK2, CEBPD, ZMYND11, FBN2, CDH2, ELOVL6, KIF23, AOX1, KCNJ8, DIAPH2, C14orf106, ZNF43, ANAPC5, FHL1, ATRX, CDK2, KIAA0146, HNRNPH1, PARD6B, RCAN1, NTRK3, PRIM2, 215768at, PHLDA1, VASH2, SYNPO2L, FAM198B, ZMAT4, GDAP1, FGFR1, ETS2, and SFRS15, instead of or in addition to means for determining the level of expression of NUP155 or a fragment thereof; and/or
- means for determining the level of expression of a gene or a fragment thereof selected from the group consisting of MBNL2, CNTNAP2, EXOC5, CNTNAP2, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, and MAP3K2, instead of or in addition to means for determining the level of expression of GSN or a fragment thereof.

In certain embodiments of the kits of parts as taught herein, the means for determining of the level of expression of the 11 genes or fragments thereof may be means for performing quantitative real-time PCR (qPCR), reverse transcription-qPCR (RT-qPCR), a nucleic acid microarray, digital molecular barcoding technology (nCounter Nanostring), branched DNA (bDNA) signal amplification technology (Quantigene), mass spectrometry (Sequenom), or a combination of said methods.

In certain embodiments, the kits as taught herein may further comprise a plurality of reagents, each of which is capable of specifically binding with a nucleic acid or polypeptide corresponding to a gene or a fragment thereof as taught herein. Suitable probes for binding with a nucleic acid (e.g. a genomic DNA, an mRNA, a spliced mRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of polymerase chain reaction (PCR) primers, molecular beacon probes, and the like.

In certain embodiments, the kits as taught herein may comprise a nucleic acid probe that binds specifically with a nucleic acid of a gene or fragment thereof as taught herein.

In certain embodiments, the kits may comprise primers capable of specifically binding the 11 genes or fragments thereof as defined herein. Such kits advantageously allow specifically amplifying the 11 genes or fragments thereof as defined herein, for instance by PCR.

The term "specifically binding" as used herein refers to the ability of a probe or primer to preferentially bind to a particular nucleic acid that is present alongside different nucleic acids. In certain embodiments, a specific binding interaction will discriminate between desirable (target) and undesirable (non-target) nucleic acids (nucleic acid sequences), in some embodiments more than about 10-fold, more than about 100-fold (e.g., more than about 1000- or 10,000-fold).

In certain embodiments, the kits as taught herein may further comprise means for performing polymerase chain reaction (PCR). The kit may further comprise media and solution suitable for taking a sample and/or for extracting RNA from a sample as taught herein. In certain embodiments, the kits as taught herein may further comprise a buffer suitable for use in the kit.

In certain embodiments, the kits as taught herein may further comprise additional components for carrying out the method of the invention, such as RNA extraction solutions, purification column and buffers and the like. In certain embodiments, the kits as taught herein may further include any additional reagents, reporter molecules, buffers, excipients, containers and/or devices as required described herein or known in the art, to practice the methods as taught herein.

The various components of the kits may be present in separate containers or certain compatible components may be pre-combined into a single container, as desired. In addition to the above components, the kits may further include instructions for practicing the methods as taught herein. These instructions may be present in the kits in a variety of forms, one or more of which may be present in the kits as taught herein. In certain embodiments, the instructions may be present as printed information on a suitable medium or substrate, e.g., a piece or pieces of paper on which the information is printed, in the packaging of the kit, in a package insert, etc. In certain embodiments, the instructions may be present on a computer readable medium, e.g., diskette, CD, etc., on which the information has been recorded.

In certain embodiments, the kit of parts may further comprise a computer-readable medium comprising one or more digitally encoded gene expression profiles, where each gene expression profile has one or more values representing the level of expression of the 11 genes or fragments thereof as defined herein.

In certain embodiments, said digitally encoded expression profiles are three reference profiles of the level of expression of the 11 genes or fragments thereof as defined herein: a first reference profile representing insensitivity to the CDK4/6 inhibitor; a second reference profile representing therapeutic sensitivity to the CDK4/6 inhibitor; and a third reference profile representing sensitivity to the CDK4/6 inhibitor but without therapeutic benefit over classical treatment by surgery and hormone therapy.

A further aspect relates to the use of the kit as defined herein for determining sensitivity to a CDK4/6 inhibitor in a human subject having breast cancer. In certain embodiments, the sensitivity to the CDK4/6 inhibitor in the human subject having breast cancer may be insensitivity of the subject to the CDK4/6 inhibitor; therapeutic sensitivity of the subject to the CDK4/6 inhibitor; or sensitivity of the subject to the CDK4/6 inhibitor but without clinical benefit over classical treatment by surgery and hormone therapy.

Provided herein, in certain embodiments, are arrays comprising probes for detection of polynucleotides (transcriptional state) or for detection of proteins (translational state) in order to detect differentially-expressed genes of the invention. The term "array" refers to a solid support or substrate with peptide or nucleic acid probes attached to said support or substrate. Arrays typically comprise a plurality of different nucleic acid or peptide capture probes that are coupled to a surface of a substrate in different, known locations. These arrays, also described as "microarrays" or "chips" have been generally described in the art. These arrays may generally be produced using mechanical synthesis methods or light directed synthesis methods which incorporate a combination of photolithographic methods and solid phase synthesis methods.

In certain embodiments, microarrays are provided and used to measure the values to be included in the expression profiles. Microarrays are particularly well suited for this purpose because of the reproducibility between different experiments.

The skilled person is capable of designing probes, such as nucleic acid probes, that can be used in the products or methods as taught herein. Preferably, such probes are immobilized on a solid surface as to form a nucleic acid microarray as taught herein. The nucleic acid probes useful in products or methods as taught herein are capable of hybridizing under stringent conditions (such as such as high hybridization temperature and low salt in hybridization buffers, as known in the art) to the at least 11 genes or fragments thereof as described herein.

In some embodiments, each probe in the array detects a nucleic acid molecule selected from the at least 11 genes or fragments thereof as described herein.

Although a planar array surface is preferred, the array may be fabricated on a surface of virtually any shape or even a multiplicity of surfaces. Arrays may be peptides or nucleic acids on beads, gels, polymeric surfaces, and fibers such as fiber optics, glass or any other appropriate substrate. Arrays may be packaged in such a manner as to allow for diagnostics or other manipulation of an all-inclusive device.

Expression of a gene or fragment thereof as taught herein may be assessed by any of a wide variety of well-known methods for detecting expression of a protein or transcribed molecule. Non-limiting examples suitable determination steps include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods. Such methods may also include physical methods such as liquid and gas chromatography, mass spectroscopy, nuclear magnetic resonance and other imaging technologies.

In a preferred embodiment, the step of determination of the level of expression is performed using microarray, preferably nucleic acid microarray. Suitable probes for said microarray are identified hereunder.

In particular, a mixture of transcribed polynucleotides obtained from the sample is contacted with a substrate having fixed thereto a polynucleotide complementary to or homologous with at least a portion (e. g. at least 7, 10, 15, 20, 25, 30, 40, 50, 100, 250, 296, or more nucleotide residue) of a RNA transcript encoded by a gene or fragment thereof as taught herein. If polynucleotides complementary to or homologous with a RNA transcript encoded by the gene or fragment thereof as taught herein are differentially detectable on the substrate (e.g., detectable using radioactivity, different chromophores or fluorophores), are fixed to different selected positions, then the levels of expression of a plurality of genes can be assessed simultaneously using a single substrate.

When the assay has an internal control, which can be, for example, a known quantity of a nucleic acid derived from a gene for which the expression level is either known or can be accurately determined, unknown expression levels of other genes can be compared to the known internal control. More specifically, when the assay involves hybridizing labeled total RNA to a solid support comprising a known amount of nucleic acid derived from reference genes, an appropriate internal control could be a housekeeping gene (e.g., glucose-6-phosphate dehydrogenase or elongation factor-1). The term "housekeeping gene" refers to a gene for which the expression level in all cell types and under all conditions is substantially the same. Use of such an internal control allows a discrete expression level for a gene to be determined (e.g., relative to the expression of the housekeeping gene) both for the nucleic acids present on the solid support and also between different experiments using the same solid support. This discrete expression level can then be normalized to a value relative to the expression level of the control gene (for example, a housekeeping gene). As used herein, the term "normalized" refers to a manipulation of discrete expression level data wherein the expression level of a reference gene is expressed relative to the expression level of a control gene. For example, the expression level of the control gene can be set at 1, and the expression levels of all reference genes can be expressed in units relative to the expression of the control gene.

In one embodiment, nucleic acids isolated from a biological sample are hybridized to a microarray, wherein the microarray comprises nucleic acids corresponding to those genes to be tested as well as internal control genes. The genes are immobilized on a solid support, such that each position on the support identifies a particular gene. Solid supports include, but are not limited to nitrocellulose and nylon membranes. Solid supports can also be glass or silicon-based (i.e. gene "chips"). Any solid support can be used in the products and methods as taught herein, so long as the support provides a substrate for the localization of a known amount of a nucleic acid in a specific position that can be identified subsequent to the hybridization and detection steps.

A microarray can be assembled using any suitable method known to one of skill in the art, and any one microarray configuration or method of construction is not considered to be a limitation of the disclosure.

A further aspect relates to the use of a nucleic acid array or nucleic acid microarray for determining sensitivity to a CDK4/6 inhibitor in a human subject having breast cancer, the nucleic acid array or nucleic acid microarray comprising probes capable of specifically binding to (hybridizing with) 11 genes or fragments thereof selected from the group consisting of CDKN2A, CCNE1, TAGLN2, TIMM17A, RAB31, SPDL1, NUP155, FBXL5, GSN, TP53TG1, and PPP1R3C.

The kits of parts, nucleic acid arrays, or nucleic acid microarrays as taught herein may be conveniently denoted as "products".

In the disclosure herein, a nucleic acid array or microarray may comprise probes capable of specifically binding to 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, wherein the nucleic acid array or microarray may comprise:
- probes capable of specifically binding to OLFM4 or a fragment thereof instead of or in addition to probes capable of specifically binding to TP53TG1 or a fragment thereof; and/or
- probes capable of specifically binding to a gene or a fragment thereof selected from the group consisting of OLFM4, MIDI, CDKN2C, TRIM24, IL6, LMNB2, CMAS, MST4, C1RL, and NCAPG2, instead of or in addition to probes capable of specifically binding to PPP1R3C or a fragment thereof; and/or
- probes capable of specifically binding to a gene or a fragment thereof selected from the group consisting of CDH1, IL6ST, GNG4, PLXNC1, MAGEA1, NPY5R, PGR, TFPI, TMPO, FUT9, ESR1, TNC, 216007_at, COL2A1, SCYL2, and TAF9B, instead of or in addition to probes capable of specifically binding to FBXL5 or a fragment thereof; and/or
- probes capable of specifically binding to a gene or a fragment thereof selected from the group consisting of CDH1, IL6ST, GNG4, PLXNC1, MAGEA1, NPY5R, PGR, TFPI, TMPO, FUT9, ESR1, TNC, 216007at, COL2A1, SCYL2, TAF9B, MBNL2, CNTNAP2, EXOC5, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, MAP3K2, SSX2IP, CHEK1, NEK2, CEBPD, ZMYND11, FBN2, CDH2, ELOVL6, KIF23, AOX1, KCNJ8, DIAPH2, C14orf106, ZNF43, ANAPC5, FHL1, ATRX, CDK2, KIAA0146, HNRNPH1, PARD6B, RCAN1, NTRK3, PRIM2, 215768at, PHLDA1, VASH2, SYNPO2L, FAM198B, ZMAT4, GDAP1, FGFR1, ETS2, and SFRS15, instead of or in addition to probes capable of specifically binding to NUP155 or a fragment thereof; and/or
- probes capable of specifically binding to a gene or a fragment thereof selected from the group consisting of MBNL2, CNTNAP2, EXOC5, CNTNAP2, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, and MAP3K2, instead of or in addition to probes capable of specifically binding to GSN or a fragment thereof.

In certain embodiments, the nucleic acid arrays or nucleic acid microarrays may further comprise a computer-readable medium comprising one or more digitally encoded gene expression profiles, where each gene expression profile has one or more values representing the level of expression of the 11 genes or fragments thereof as defined herein. In certain embodiments, said digitally encoded expression profiles are three reference profiles of the level of expression of the 11 genes or fragments thereof as defined herein: a first reference profile representing insensitivity to the CDK4/6 inhibitor; a second reference profile representing therapeutic sensitivity to the CDK4/6 inhibitor; and a third reference profile representing sensitivity to the CDK4/6 inhibitor but without therapeutic benefit over classical treatment by surgery and hormone therapy.

A further aspect relates to the use of the nucleic acid array or microarray as defined herein, for determining sensitivity to a CDK4/6 inhibitor in a human subject having breast cancer. In certain embodiments, the sensitivity to the CDK4/6 inhibitor in the human subject having breast cancer may be insensitivity of the subject to the CDK4/6 inhibitor; therapeutic sensitivity of the subject to the CDK4/6 inhibitor; or sensitivity of the subject to the CDK4/6 inhibitor but without clinical benefit over classical treatment by surgery and hormone therapy.

A further aspect provides a CDK4/6 inhibitor for use in treating breast cancer in a human subject, wherein the subject has been selected to have a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, whereby the gene expression profile of the subject provides the highest correlation with a reference profile representing therapeutic sensitivity to the CDK4/6 inhibitor (e.g., with a reference profile of the level of expression of the 11 genes or fragments thereof obtained from reference samples of reference subjects having therapeutic sensitivity to the CDK4/6 inhibitor).

The expression "the gene expression profile of the subject provides the highest correlation with a reference profile representing therapeutic sensitivity to the CDK4/6 inhibitor" as used herein means that the gene expression profile of the subject provides the highest correlation with a reference profile representing therapeutic sensitivity to the CDK4/6 inhibitor, when the gene expression profile of the subject is correlated with a reference profile representing therapeutic sensitivity to the CDK4/6 inhibitor; a reference profile representing insensitivity to the CDK4/6 inhibitor; and a reference profile representing sensitivity of the subject to the CDK4/6 inhibitor but without clinical benefit over classical treatment by surgery and hormone therapy.

A related aspect provides a CDK4/6 inhibitor for use in treating breast cancer in a human subject, wherein the subject has a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, that provides the highest correlation with a reference profile representing therapeutic sensitivity to the CDK4/6 inhibitor (e.g., with a reference profile of the level of expression of the 11 genes or fragments thereof obtained from reference samples of reference subjects having therapeutic sensitivity to the CDK4/6 inhibitor).

In the disclosure herein, the subject may have been selected to have or may have a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, wherein the gene expression profile may comprise:
- the level of expression of OLFM4 or a fragment thereof instead of or in addition to the level of expression of TP53TG1 or a fragment thereof; and/or
- the level of expression of a gene or a fragment thereof selected from the group consisting of OLFM4, MIDI, CDKN2C, TRIM24, IL6, LMNB2, CMAS, MST4, C1RL, and NCAPG2, instead of or in addition to the level of expression of PPP1R3C or a fragment thereof; and/or
- the level of expression of a gene or a fragment thereof selected from the group consisting of CDH1, IL6ST, GNG4, PLXNC1, MAGEA1, NPY5R, PGR, TFPI, TMPO, FUT9, ESR1, TNC, 216007_at, COL2A1, SCYL2, and TAF9B, instead of or in addition to the level of expression of FBXL5 or a fragment thereof; and/or
- the level of expression of a gene or a fragment thereof selected from the group consisting of CDH1, IL6ST, GNG4, PLXNC1, MAGEA1, NPY5R, PGR, TFPI, TMPO, FUT9, ESR1, TNC, 216007_at, COL2A1, SCYL2, TAF9B, MBNL2, CNTNAP2, EXOC5, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, MAP3K2, SSX2IP, CHEK1, NEK2, CEBPD, ZMYND11, FBN2, CDH2, ELOVL6, KIF23, AOX1, KCNJ8, DIAPH2, C14orf106, ZNF43, ANAPC5, FHL1, ATRX, CDK2, KIAA0146, HNRNPH1, PARD6B, RCAN1, NTRK3, PRIM2, 215768at, PHLDA1, VASH2, SYNPO2L, FAM198B, ZMAT4, GDAP1, FGFR1, ETS2, and SFRS15, instead of or in addition to the level of expression of NUP155 or a fragment thereof; and/or
- the level of expression of a gene or a fragment thereof selected from the group consisting of MBNL2, CNTNAP2, EXOC5, CNTNAP2, DUSP1, LMAN1, NRCAM, EDNRA, EDN2, NXT2, TGFB2, PTGER3, GOLGA8B/GOLGA8A, ELOVL5, POSTN, STRN3, PDS5B, RRN3P1/RRN3P2/RRN3, PTPN12, MDFIC, WHSC1L1, TMEM70, and MAP3K2, instead of or in addition to the level of expression of GSN or a fragment thereof.

A further aspect provides a CDK4/6 inhibitor for use in a method of treating breast cancer in a human subject, wherein the method comprises the steps of:
- determining the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, in a sample from the subject, thereby obtaining a gene expression profile of the subject; and
- identifying that the gene expression profile of the subject provides the highest correlation with a reference profile representing therapeutic sensitivity to a CDK4/6 inhibitor (e.g., with a reference profile of the level of expression of the 11 genes or fragments thereof obtained from reference samples of reference subjects having therapeutic sensitivity to the CDK4/6 inhibitor).

The specification provides a method of treating breast cancer in a human subject in need of such a treatment, comprising administering a therapeutically effective amount of the CDK4/6 inhibitor to the subject, wherein the subject has been selected to have a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, whereby the gene expression profile of the subject provides the highest correlation with a reference profile representing therapeutic sensitivity to the CDK4/6 inhibitor (e.g., with a reference profile of the level of expression of the 11 genes or fragments thereof obtained from reference samples of reference subjects having therapeutic sensitivity to the CDK4/6 inhibitor).

The specification further provides a method of treating breast cancer in a human subject in need of such a treatment, comprising administering a therapeutically effective amount of the CDK4/6 inhibitor to the subject, wherein the subject has a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, that provides the highest correlation with a reference profile representing therapeutic sensitivity to the CDK4/6 inhibitor (e.g., with a reference profile of the level of expression of the 11 genes or fragments thereof obtained from reference samples of reference subjects having therapeutic sensitivity to the CDK4/6 inhibitor). The specification further provides a method of treating breast cancer in a human subject in need of such a treatment, the method comprising:
- determining in a sample from the subject the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, thereby obtaining a gene expression profile of the subject;
- identifying that the gene expression profile of the subject provides the highest correlation with a reference profile representing therapeutic sensitivity to a CDK4/6 inhibitor (e.g., with a reference profile of the level of expression of the 11 genes or fragments thereof obtained from reference samples of reference subjects having therapeutic sensitivity to the CDK4/6 inhibitor); and
- if the gene expression profile of the subject provides the highest correlation with the reference profile, administering a therapeutically effective amount of the CDK4/6 inhibitor to the subject, thereby treating the subject.

The specification provides the use of a CDK4/6 inhibitor for the manufacture of a medicament for the treatment of breast cancer in a human subject, characterized in that the subject has been selected to have a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, whereby the gene expression profile of the subject provides the highest correlation with a reference profile representing therapeutic sensitivity to the CDK4/6 inhibitor (e.g., with a reference profile of the level of expression of the 11 genes or fragments thereof obtained from reference samples of reference subjects having therapeutic sensitivity to the CDK4/6 inhibitor).

The specification further provides the use of a CDK4/6 inhibitor for the manufacture of a medicament for the treatment of breast cancer in a human subject, characterized in that the subject has a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, whereby the gene expression profile of the subject provides the highest correlation with a reference profile representing therapeutic sensitivity to the CDK4/6 inhibitor (e.g., with a reference profile of the level of expression of the 11 genes or fragments thereof obtained from reference samples of reference subjects having therapeutic sensitivity to the CDK4/6 inhibitor).

The specification also provides the use of a CDK4/6 inhibitor for the manufacture of a medicament for the treatment of breast cancer in a human subject, characterized in that the subject has been selected for treatment by a method comprising:
- determining in a sample from the subject the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, thereby obtaining a gene expression profile of the subject;
- identifying that the gene expression profile of the subject provides the highest correlation with a reference profile representing therapeutic sensitivity to a CDK4/6 inhibitor; and
- if the gene expression profile of the subject provides the highest correlation with the reference profile, selecting the subject for the treatment (e.g., with a reference profile of the level of expression of the 11 genes or fragments thereof obtained from reference samples of reference subjects having therapeutic sensitivity to the CDK4/6 inhibitor).

The term "diseased subject" as used herein refers to a subject diagnosed with or having breast cancer.

As used herein, a phrase such as "a subject in need of treatment" includes subjects that would benefit from treatment of a given condition, particularly breast cancer. Such subjects may include, without limitation, those that have been diagnosed with said condition, those prone to develop said condition and/or those in who said condition is to be prevented.

The terms "treat" or "treatment" encompass both the therapeutic treatment of an already developed disease or condition, such as the therapy of an already developed breast cancer, as well as prophylactic or preventive measures, wherein the aim is to prevent or lessen the chances of incidence of an undesired affliction, such as to prevent occurrence, development and progression of breast cancer. Beneficial or desired clinical results may include, without limitation, alleviation of one or more symptoms or one or more biological markers, diminishment of extent of disease, stabilised (i.e., not worsening) state of disease, delay or slowing of disease progression, amelioration or palliation of the disease state, and the like. "Treatment" can also mean prolonging survival as compared to expected survival if not receiving treatment.

The term "prophylactically effective amount" refers to an amount of an active compound or pharmaceutical agent that inhibits or delays in a subject the onset of a disorder as being sought by a researcher, veterinarian, medical doctor or other clinician. The term "therapeutically effective amount" as used herein, refers to an amount of active compound or pharmaceutical agent that elicits the biological or medicinal response in a subject that is being sought by a researcher, veterinarian, medical doctor or other clinician, which may include inter alia alleviation of the symptoms of the disease or condition being treated. Methods are known in the art for determining therapeutically and prophylactically effective doses for the pharmaceutical formulation as taught herein.

The products and methods as taught herein allow to administer a therapeutically effective amount of the CDK4/6 inhibitor as taught herein in subjects having breast cancer which will benefit from such treatment. The term "therapeutically effective amount" as used herein, refers to an amount of active compound or pharmaceutical agent that elicits the biological or medicinal response in a subject that is being sought by a surgeon, researcher, veterinarian, medical doctor or other clinician, which may include inter alia alleviation of the symptoms of the disease or condition being treated. Methods are known in the art for determining therapeutically effective doses of the CDK4/6 inhibitor as taught herein.

In the context of the present invention a "therapeutically effective dose" means an amount of CDK4/6 inhibitor as taught herein, such as palbociclib, that when administered brings about a positive therapeutic response with respect to treatment of a patient having breast cancer.

Appropriate therapeutically effective doses of the CDK4/6 inhibitor as taught herein may be determined by a qualified physician with due regard to the nature of the CDK4/6 inhibitor, the disease condition and severity, and the age, size and condition of the patient.

Without limitation, a typical dose of the CDK4/6 inhibitor, for instance palbociclib, to be administered may range from about 10 mg to about 1000 mg per administration. For example, the dose of the CDK4/6 inhibitor, for instance palbociclib, to be administered may range from about 100 mg to about 500 mg or from about 75 mg to about 200 mg, per administration. Preferably, the dose of the CDK4/6 inhibitor, for instance palbociclib, to be administered may be about 125 mg per administration.

For instance, a CDK4/6 inhibitor such as PD-0332991 may be orally administered once a day (75 mg to 200 mg; typically 125 mg) for 2 or 3 weeks followed by 1 week off treatment, then continuing treatment with the same schedule. Alternatively, a CDK4/6 inhibitor such as PD-0332991 may be administered by intravenous administration (typically 50 mg intravenous dose) as an infusion (e.g., 4-hours infusion). Further for instance, a CDK4/6 inhibitor such as LEE011 (50-600 mg, typically 600 mg) may be orally administered daily 3 weeks on/1 week off in 28 day cycles. For instance, a CDK4/6 inhibitor such as LY2835219 (typically 150-200 mg) may be administered orally once every 12 hours in 21 or 28 day cycles.

While the invention has been described in conjunction with specific embodiments thereof, it is evident that many alternatives, modifications, and variations will be apparent to those skilled in the art in light of the foregoing description.

The herein disclosed aspects and embodiments of the invention are further supported by the following non-limiting examples.

### EXAMPLES

### Materials and methods

### Cell culture

Human breast carcinoma cell lines were purchased at the ATCC Cell Biology Collection and maintained in a humidified atmosphere (5% CO₂) at 37°C.

### DNA synthesis and cell growth assays

Cells were seeded in triplicates, incubated for at least 16 hours to attach to the 96-wells plates and challenged with the indicated serial dilutions of PD0332991 for 24 hours. One hour before fixation with methanol, 100 µM 5-Bromo-2'-deoxyuridine (BrdU, Sigma-Aldrich) and 4 µM 5-Fluoro-2'-deoxyuridine (FldU, Sigma-Aldrich) were added to the cells. Immuno-detection of DNA incorporated BrdU was performed. 4',6-Diamidino-2-phenylindole, dilactate (DAPI, Thermo Fisher Scientific) solution (1 µg/ml) was used as nuclear counterstain and round coverslips were mounted in each well of the 96 well plate with ProLong Gold Antifade Mountant (Thermo Fisher Scientific). The impact of PD0332991on cellular growth was also evaluated using the sulforhodamine B (SRB) assay. In brief, cells seeded in 96-wells plates were allowed to attach for at least 16 hours before being fixed (time 0 point) or incubated with serial concentrations of PD0332991 for 72 hours and 144 hours. Protein-bound SRB solubilized with Tris base solution (10mM, pH 10.5) was quantified fluorometrically in a Tecan GENios microplate reader at excitation and emission wavelengths of 485 nm and 600 nm, respectively.

Alternatively, cells seeded in triplicates in 96-wells plates were allowed to attach for at least 16 hours before being incubated with serial dilutions of PD0332991 for 48 hours. Thiazolyl Blue Tetrazolium Bromide at a final concentration of 0.6 mg/ml (MTT, Sigma-Aldrich) was added to the cell cultures and incubated for 2 hours. After removal of the medium, accumulated formazan derivatives were solubilized with DMSO for 30 minutes with shaking before absorbance was measured in a iMark microplate reader (Bio-Rad) at 540 nm.

### Image acquisition and analysis

Culture plates were imaged with a Zeiss Axio Observer.Z 1 wide-field microscope equipped with a 10/0.3x EC Plan Neofluar objective and an AxioCam HSm camera. For each well, a grid of 3x3 fields (each of them 660 µm x 492 µm) was automatically defined using the Mark&Find option of the AxioVision software. DAPI signal was recorded using a 365 nm LED excitation and the Zeiss filterset 49. Alexa Fluor 488 signal was recorded using a 470 nm LED excitation and the Zeiss filterset 52. Exposure times were chosen so as to maximize the signal while avoiding saturation. They were kept constant through all images within one experiment. A software-based autofocus was performed on the DAPI channel, using a general offset of 8-10 µm. A 10 µm offset was added for the A488 channel. Images were analyzed semi-automatically with a custom-made ImageJ macro. For each field of view, two masks were created, representing the areas positive for the DAPI or for both the DAPI and the BrdU stainings, respectively. Nuclei were then segmented within these masks using the "Analyze particles" function. The total number of nuclei and the area that they represent were recorded to compute the BrdU-positive to total nuclei ratios. The segmentation of the nuclei was verified visually and only values extracted from correctly segmented images were selected for further analysis. Good correlation between the automatic counts obtained using this macro and manual counts using the Cell Counter Image J pluggin was achieved except when only a few cells are stained. Each processed image was therefore systematically inspected to correct low counts and remove image with staining artefacts from the analysis. Images with less than 10 labeled nuclei were counted manually with the Image J cell counter plugin.

### Proteomic analysis

Equal amounts of whole cell extract proteins were separated according to molecular mass and immunodetected. CDK4-containing complexes were co-immunoprecipited as described (Paternot et al., 2003, J Biol Chem, 278, 26533-40; Bockstaele et al., 2006, Mol Cell Biol, 26, 5070-85). Used antibodies are listed in Table 3. For 2D gel electrophoresis, cells were lysed in a buffer containing 7 M urea and 2 M thiourea. Frozen tumor slides were solubilised in 30 mM Tris cold buffer pH 8.5 containing 7 M urea, 2 M thiourea and 4% CHAPS with continuous vortexing until defreezing and kept under agitation during 20 min. After centrifugation at 14000 rpm for 10 min at 4°C, proteins were quantified. An equal volume of 2-D-sample buffer (7 M urea, 2 M thiourea, 2%CHAPS, 0.4% 3-10 Pharmalytes and 0.4% DTT) was added to samples normalized at 150 µg proteins. Proteins were separated by isoelectric focusing on immobilized linear pH gradient strips (pH 5 to 8, Biorad) before being separated by SDS-PAGE and immunoblotted as described (Bockstaele et al., 2006, Mol Cell Biol, 26, 5070-85).

**Table 3: List of used antibodies**

| **Antibodies (antigen listed) and reagents** | **Provider** | **Reference** | **use** |
|---|---|---|---|
| cyclin D 1 | Novus | EPR2241 | IP |
| cyclin D3 | Neomarkers | DCS-28 | IP |
| p16 | Santa Cruz | DCS-50 | IP |
| CDK4 | Santa Cruz | H-22 | IP |
| BrdU | BD Bioscience | #347580 | IHC |
| biotinylated anti-mouse | GE Healthcare | RPN1001V | IHC |
| Alexa Fluor 488-conjugated Streptavidin | Jackson ImmunoResearch | 3016-480-084 | IHC |
| cyclin D1 (monoclonal) | Neomarkers | DCS-6 | WB |
| cyclin D3 | Neomarkers | DCS-22 | WB |
| pRb | BD Pharmingen | #554136 | WB |
| phospho-pRb | CST | S807/811 | WB |
| cyclin E | Pierce | HE12 | WB |
| CDK4 (monoclonal) | Santa Cruz | DCS-31 | WB |
| CDK4 (polyclonal) | Santa Cruz | H-22 | WB |
| P-CDK4 (polyclonal) | CST | * | WB |
| p16 | Santa Cruz | DCS-50 | WB |
| CDK7 | Santa Cruz | C4 | WB |
| CDK2 | Santa Cruz | M2 | WB |
| CDK6 | Santa Cruz | C-21 | WB |
| HRP-coupled anti-mouse | Santa Cruz | sc-2005 | WB |
| HRP-coupled anti-rabbit | Santa Cruz | sc-2370 | WB |
| DyLight 680-couplet anti-mouse | Pierce Biotechnology | #35518 | WB |
| DyLight 800-couplet anti-rabbit | Pierce Biotechnology | #SA5-35571 | WB |

| | | | |
|---|---|---|---|
| * Non-commercialized phospho-specific-CDK4 (Thr172) antibody produced by immunizing rabbits with a keyhole limpet hemocyanin-coupled peptide fragment of human CDK4 phosphorylated on T172 and purified by protein A- and immunogen-based affinity column separation IP: immunoprecipitation; IHC: immunohistochemistry; WB: Western blot | | | |

### Genomic analysis

RNA was extracted with Trizol from 50-80% confluent cells seeded in 10 cm Petri dishes or from tumor samples. The microarray experiment, including probe labeling, hybridization on Affymetrix GeneChip (Human Genome U133 Plus 2·0 (HG133plus2)) was performed at the Bordet Institute or at the University of Antwerp. In addition, the cell files of breast cell lines and tumors performed on HG133A or HG133plus2 Affymetrix array platforms published in GEO or Array Express were downloaded and processed. Expression data were extracted from the cell files, background-subtracted, normalized, summarized (median polish option) in R using frozen RMA (McCall et al., 2010, Biostatistics, 11, 242-53) and converted to signal intensity values. Probe sets with differential expression according to the CDK4 modification profiles of the corresponding tumors were selected in R using either the SAMr and pROC packages. Reference centroids were generated by computing, for each selected probe set, the average of the expression values observed in the selected tumors displaying the same CDK4 modification profile. The expression values recorded for a given patient of each selected probe sets are compared to the three centroids corresponding to the three CDK4 modification profiles by Spearman correlation. The predicted profile is the one corresponding to the centroid which is best correlated with the considered patient expression profile.

### Example 1: Correlation between the sensitivity of breast cancer cell lines to CDK4/6 inhibitor palbociclib and the detection of phosphorylated CDK4 and key cell cycle markers

The relation between T172 CDK4 phosphorylation and sensitivity to palbociclib (PD0332991) was analyzed. IC50 values for PD0332991 in breast cancer cell lines have previously been reported. A set of 20 breast cancer cell lines was selected to obtain a balanced panel representative of diverse PD0332991 sensitivities, molecular subtypes, pRb, p16 and HER2 status (Table 4).

**Table 4: Characteristics of selected breast cancer cell lines**

| **Cell line** | **Culture medium *** | **Source** | **Split ratio** | **BrdU density** | **SRB** density** | **MTT density** | **Type** | **Observed profile** | **IC50** |
|---|---|---|---|---|---|---|---|---|---|
| BT20 | DMEM | ATCC | 1/3 | 20000 | 5000 | 5000 | Ba | 2 | 3 |
| BT474 | RPMI | ATCC | 1/3 | 20000 | 5000 | 20000 | HER | 2 | 15 |
| BT549 | RPMI | ATCC | 1/3 | 20000 | 20000 | 5000 | Ba | 1 | >1000 |
| HCC1500 | RPMI | ATCC | 1/3 | 20000 | 20000 | 20000 | Ba | 2 | 10 |
| HCC1569 | RPMI | ATCC | 1/3 | 20000 | 5000 | 10000 | HER | 1 | >1000 |
| HCC1806 | RPMI | ATCC | 1/5 | 20000 | 20000 | 5000 | Ba | 2 | 10 |
| HCC1937 | RPMI | ATCC | 1/3 | 20000 | 20000 | 5000 | Ba | 1 | >1000 |
| HCC1954 | RPMI | ATCC | 1/5 | 20000 | 20000 | 5000 | Her | 2 | 25 |
| HCC202 | RPMI | ATCC | 1/2 | 40000 | 40000 | 20000 | Her | 2 | 25 |
| HCC38 | RPMI | ATCC | 1/3 | 20000 | 20000 | 5000 | Ba | 2 | 15 |
| HCC70 | RPMI | ATCC | 1/4 | 20000 | 20000 | 5000 | Ba | 1 | >1000 |
| MCF7 | RPMI | ATCC | 1/3 | 20000 | 5000 | 20000 | Lum | 2 | 15 |
| MDAMB134VI | RPMI | ATCC | 1/3 | 20000 | 20000 | 20000 | Lum | 2 | 3 |
| MDAMB231 | DMEM | ATCC | 1/5 | 20000 | 5000 | 20000 | Ba | 2 | 15 |
| MDAMB361 | DMEM | ATCC | 1/3 | 30000 | 20000 | 20000 | HER | 2 | 10 |
| MDAMB436 | DMEM | ATCC | 1/5 | 20000 | 20000 | 20000 | Ba | 1 | >1000 |
| MDAMB468 | DMEM | ATCC | 1/5 | 20000 | 20000 | 10000 | Ba | 1 | >1000 |
| SKBR3 | DMEM | ATCC | 1/3 | 20000 | 20000 | 20000 | HER | 2 | 15 |
| T47D | RPMI | ATCC | 1/3 | 20000 | 20000 | 10000 | Lum | 2 | 15 |
| ZR751 | RPMI | ATCC | 1/3 | 20000 | 5000 | 10000 | HER | 2 | 15 |

| | | | | | | | | | |
|---|---|---|---|---|---|---|---|---|---|
| *supplemented with 10% serum ** Sulforhodamine B | | | | | | | | | |

As the IC50s observed in previous studies diverged, the cell cycle impact of palbociclib was investigated by transposing the BrdU DNA incorporation assay into a 96 well format. Effects of palbociclib were also determined with sulforhodamine or MTT assays to compare our results with the previous ones. The basal proportion of DNA replicating cells determined after one hour of BrdU incubation varied between 50% in fast growing cells to 20 % in slowly growing cells (FIG. 1A). This proportion was reduced by PD0332991 in a concentration-dependent way in 13 out of these 20 cell lines (FIG. 1B for two representative sensitive and two representative insensitive cell lines). With the exception of the HER2+ HCC1954 cell line (sensitive), the responses of the cell lines to PD0332991were consistent with previous studies.

IC50s were obtained ranging from 5 to 50 nM with the BrdU incorporation assay (Table 4). These IC50 values were close to the PD0332991 IC50 on CDK4 activity *in vitro* (11 nM) (Toogood et al., 2005, J Med Chem, 48, 2388-406) and below its serum levels (> 50ng/ml, 112nM) achieved after oral daily intake of 125 mg of palbociclib (Finn et al., 2015, Lancet Oncol, 16, 25-35). The impact of PD0332991 on cell growth was generally less observed when measured either by the sulforhodamine or MTT assays (FIG. 1C). Either complete or partial reduction was observed (as in MCF7, HCC1954 or BT474 cells) of the proportion of cells in S phase measured with the BrDU assay (FIG. 1A, 1B, 1C). In the case of HCC1806 cells, this proportion slightly decreased in average to 80 % of the control value (FIG. 1C)) in 5 independent experiments. Hence, this cell line seemed to conserve a residual sensitivity to the CDL4/6 inhibitor, which was confirmed by a marked inhibition of pRb phosphorylations in response to PD0332991 (not shown). All luminal (MCF7, T47D, MDAMB134IV) and most HER2+ cell lines (HCC1954, SKBR3, ZR75-1, MDAMB361, BT474) were sensitive to PD0332991 while basal-like cells were either sensitive (HCC38, HCC1500, BT20, MDAMB231) or insensitive (MDAMB436, MDAMB468, HCC1937, BT459, HCC70) (FIG. 1A, FIG. 1B).

Next, whole protein extracts of the 20 analyzed cell lines were resolved on 2D gel electrophoresis and revealed by a CDK4 antibody. CDK4 could be resolved by its charge in three main forms (FIG. 1D). The most basic form (form 1) observed in all samples was identified as the native CDK4. The most acidic form (form 3) detected in the MCF7 and MDAMB231 samples for example corresponds to the form previously identified as the T172-phosphorylated CDK4 using several approaches including a T172-phosphospecific antibody (FIG. 2A and 2B). Another modified CDK4 form (form 2) observed in the normal breast reference sample and most cell lines is yet unidentified (FIG. 1D). The CDK4 modification profiles of each of the 20 cell lines were determined (FIG. 4). In some cell lines such as ZR75-1, MCF7, T47D and HCC1937, a minor more acidic form was also detected using antibodies directed to both CDK4 and its T172-phosphosphorylation. The presence of the phosphorylated CDK4 was enriched in CDK4 complexes immunoprecipitated using anti-cyclin D antibodies (FIG. 3). The unidentified CDK4 modification was observed both in CDK4 and in p16 immunoprecipitates and enriched in the latter compared to anti-cyclin D precipitates. Furthermore, the unidentified CDK4 modification was less present in anti-cyclin D3 precipitates compared to anti-cyclin D1 ones (FIG. 3). Importantly, the T172 phosphorylated CDK4 form was detected in all sensitive breast cancer cell lines including in the HCC1806 cell line with reduced sensitivity to PD0332991 (FIG. 4). Except in this cell line, resistance to PD0332991 was always associated with the complete absence of phosphorylated CDK4. The unidentified form 2 of CDK4 was detected in all insensitive cell lines but also in some sensitive ones (FIG. 4). The level of the unidentified form 2 of CDK4 was low in HCC1806, BT20, MCF7 cells while it was almost undetectable in MDAMB231, MDAMB134VI and BT474 cells (FIG. 4).

Further, a 1D western blot analysis was performed to determine the levels of key players in the regulation of the cell cycle. As shown in FIG. 4, pRb was detected not only in all the sensitive cell lines and HCC1806 cells, but also at a lower level in three of six insensitive cell lines (HCC1937, HCC70, and HCC1569). pRb was undetectable in the other three resistant cells (MDAMB346, MDAMB468 and BT459). Phosphorylation of pRb on T826 was high in all sensitive cell lines as well as in HCC1806 (FIG. 4). It was also detected in two resistant cell lines (HCC1569 and HCC70) (FIG. 4). The levels of CDK4 varied among the selected breast cancer cell lines and even tended to be higher in insensitive lines (FIG. 4). Cyclin D1 level was high in several sensitive cell lines but higher in insensitive HCC1937 cells than in sensitive BT20 and SKBR3 cells (FIG. 4). Cyclin D3 levels were also variable and unrelated to the PD0332991 sensitivity (FIG. 4). By contrast, the two cell lines that were resistant or partially resistant to PD0332991 despite pRb phosphorylation, i.e., HER2+ HCC1569 and basal-like HCC1806 cell lines, were observed to present very high levels of cyclin E1 (FIG. 4). In the other cell lines, levels of cyclin E1 were very variable and unrelated to pRb phosphorylation and PD0332991 sensitivity (FIG. 4). The lower molecular weight bands were also detectable in some of these lines but their levels were much lower than in HCC1569 or HCC1806 cells.

Finally, p16 was undetectable due to *CDKN2A* locus deletion (e.g. MCF7) or methylation (e.g. T47D and MDAMB134VI) in several cell lines which were perfectly sensitive to PD0332991 (FIG. 4). P16 was detectable but low in four of the five PD0332991-sensitive cell lines with amplified *HER2* locus (SKBR3, ZR75-1, MDAMB361 and BT474) (not shown). On the other hand, p16 was strongly elevated not only in the insensitive cell lines (mostly basal-like cells) with absent pRb expression and phosphorylation but also in the two insensitive cell lines with detectable pRb phosphorylation (HCC70 and HCC1569). In the partially resistant basal-like HCC1806 with high cyclin E1 expression, p16 was absent due to *CDKN2A* locus deletion. The probable increased CDK2 activity in these cells could by-pass the need for CDK4 activity to initiate DNA synthesis in this cell line. To test this hypothesis, HCC1806 cell lines were treated with increasing concentrations of PD0332991 in the presence of low concentrations of Roscovitine known to block CDK2 in these conditions. As shown in FIG. 5, Roscovitine indeed dose-dependently increased the sensitivity of HCC1806 cells to PD0332991.

### Example 2: CDK4 modification profiles vary in breast tumors and are to some extent associated with the molecular subtype of the breast tumor

The 2D gel electrophoresis assay was adapted to tumor samples to show that modified and native CDK4 can be extracted from 5 to 7 tumor samples cryosections (7 µm thickness) in urea buffer. The same three main forms of CDK4 as those resolved in samples of breast cancer cell lines were also detected in tumor or normal breast samples (FIG. 6). The most acidic CDK4 form detected in breast tumor samples was identified as phosphorylated CDK4 using a T172-phosphospecific CDK4 antibody.

The relative abundances of the different CDK4 forms were compared in samples extracted from normal breast obtained from mammary reduction and from an exploratory cohort of 19 breast tumors for which the clinical records and microarray data were accessible (Dedeurwaerder et al., 2011, EMBO Mol Med, 3, 726-41). Three types of profiles were consistently observed in this cohort (FIG. 6). In normal breast as in most PD0332991-insensitive breast cancer cell lines, only the basic unmodified form 1 and the unidentified CDK4 form 2 were detected (FIG. 6, upper panel). Surprisingly, in 4 tumors, the same profile (referred to herein as CDK4 modification profile 1) was observed despite a high proliferation index. In another subset of 9 tumors, also characterized with a high proliferation rate, a CDK4 modification profile (referred to herein as CDK4 modification profile 2) was observed similar to the sensitive breast cancer cells lines. In this second profile, both the phosphorylated form 3 and unidentified CDK4 form 2 were detected but the phosphorylated form was fairly abundant, and reached at least 90% of the intensity of the intermediate form. Finally, in a third group of 6 tumors, the phosphorylated form was also detected but in contrast to breast cancer cell lines, its abundance was much lower than the one of the unidentified CDK4 form 2 (referred to herein as CDK4 modification profile 3).

To confirm these observations, 31 new tumors were analyzed from the Jules Bordet Institute grouped in a confirmatory set (7 HER2+, 5 Luminal A, 9 Luminal B and 10 triple negative tumors). The three types of profiles were also observed in this set (7 profiles 1, 12 profiles 2 and 12 profiles 3). The molecular subtype of each tumor of the two cohorts was defined based both on their immunohistochemical and published gene expression profiles. Chi-square test run on the merged cohorts excluded that the CDK4 modification profile could be unrelated to molecular subtype (p value = 0.0002). The samples with profile 1 devoid of phosphorylated CDK4 despite high proliferation index were more frequent among basal-like but also present in present in some Luminal B tumors (FIG. 7). The second profile (profile 2) with preponderant phosphorylated CDK4 was the most frequent profile among HER2+ tumors but was also present among basal-like and Luminal B tumors (FIG. 7). The third profile (profile 3) was most frequent in Luminal A tumors but also present in a few Luminal B tumors (FIG. 7). CDK4 modification profiles were also analyzed in 6 non-inflammatory breast cancer samples obtained from an independent cohort of breast tumors analyzed at the University of Antwerp (E-MTAB1006). These samples did not include any basal-like or triple negative tumors and, consistently, no profile 1 was observed. Two of the samples (one Luminal A and one Luminal B as defined by the GENEFU package) displayed a type 2 profile while the remaining displayed a type 3 profile (FIG. 7).

Determination of CDK4 modification profile 1, 2 or 3 was performed by quantifications of the spot3/spot2 ratio. In brief, Image J was used to quantify the volumes of spot 2 and spot 3 corresponding to the two main modified forms of CDK4 from 16 bit scans of the 2D gel electrophoresis Western blot autoradiography. A circle selection matching the largest spot was created to measure the volume of the two spots and to quantify the background from an area without detectable signal. The background-subtracted volume ratio (spot3/spot2) was considered to define the type of CDK4 modification profile. A profile 1 was attributed to the tumor when this ratio was below 0.1. A profile 3 was attributed to the tumor if this ratio lied between 0.1 and 0.9 while a profile 2 was given in the other cases.

### Example 3: Method according to an embodiment of the invention for determining breast tumor sensitivity to CDK4/6 inhibitors

Protein phosphorylation events are poorly preserved during formalin fixation of clinical samples. In view thereof, the CDK4 phosphorylation profiles could not be determined on formalin fixed paraffin embedded (FFPE) material routinely used in the clinic. The CDK4 phosphorylation profiles were impractical and insufficient as a marker for the sensitivity to CDK4/6 inhibitors in the clinic.

The present inventors found a method for determining the sensitivity to a CDK4/6 inhibitor in a human subject having breast cancer using the expression values of a set of 11 genes: CDKN2A, CCNE1, TAGLN2, TIMM17A, RAB31, SPDL1, NUP155, FBXL5, GSN, TP53TG1, and PPP1R3C, and 3 corresponding reference profiles.

The set of 11 genes was realized based on the correlation of the breast tumor CDK4 modification profiles to their gene expression profiles. In particular, the set of 11 genes was realized based on its performances in predicting the CDK4 modification profile of the tumors analyzed at the Bordet Institute or at the University of Antwerp, of breast cancer cell lines and of breast tumors described by the TCGA (http://cancergenome.nih.gov/) with either amplified CCNE1 locus or deleted RB1 locus (supposed to display profile 1) or deleted CDKN2A locus (supposed not to display profile 1) according to their published DNA copy number alteration profile. The predicted CDK4 modification profile of a given patient was the one corresponding to the reference profile for which the highest Spearman correlation coefficient was observed.

**Table 5: Probe set identification and reference centroids of the gene expression signature used in a method according to an embodiment of the present invention**

| **Gene** | **Probe set id** | **Ref profile1** | **Ref profile 2** | **Ref profile 3** |
|---|---|---|---|---|
| CDKN2A | 207039_at | 547.639055973636 | 56.1972154855 | 92.6030048221053 |
| CCNE1 | 213523_at | 447.625523672727 | 181.5592452515 | 111.379608112632 |
| TAGLN2 | 200916_at | 3650.70826481818 | 2586.3722851 | 1599.42003368421 |
| TIMM17A | 215171_s_at | 2481.66956654545 | 2119.86122625 | 1808.45400994737 |
| RAB31 | 217764_s_at | 1333.9815656 | 1267.272403005 | 2351.42372714737 |
| CCDC99 | 221685_s_at | 324.743109172727 | 302.440368295 | 198.530239357895 |
| NUP155 | 206550_s_at | 439.865625245455 | 373.354214715 | 263.219477157895 |
| FBXL5 | 209004_s_at | 1143.65402231818 | 1586.95538408 | 1956.58646357895 |
| GSN | 200696_s_at | 2237.07633790909 | 2053.4028651 | 2724.92328768421 |
| TP53TG1 | 209917_s_at | 113.644862124545 | 223.2499731745 | 292.019702584211 |
| PPP1R3C | 204284_at | 133.391118643636 | 249.590933195 | 706.02643132 |

A method according to an embodiment of the invention using reference centroids as provided in Table 5 correctly predicted the CDK4 modification profile of 89% of the initial 19 tumors analyzed at the Bordet Institute and 87 % of the 31 samples of the confirmation cohort. The concordance rate was 84 % when all 56 samples from the Bordet Institute and the University of Antwerp are considered (FIG. 8). This performance fell outside the distribution of the concordance rates obtained with 1000 random classifiers built on 11 randomly selected probe sets (FIG. 9A) or with 1000 random permutations of the patient labels of the merged cohort of 50 patients studied at the Bordet Institute (FIG. 9B). The molecular subtype of the tumors analyzed at the Bordet Institute (FIG. 8) was defined with the R GENEFU package (Gendoo et al., Genefu: an R/Bioconductor package for computation of gene expression-based signatures in breast cancer, 2015, Bioinformatics, 60).

A concordance rate of 100% was achieved when we applied the method according to an embodiment of the invention on the gene expression profiles of the 20 selected breast cancer cell lines (FIG. 10). Finally, the 11 genes of the gene expression signature also separated in three main clusters 4034 breast tumor gene expression profiles published in GEO or ArrayExpress (not shown).

### Example 4: Clinical relevance and utility of a method according to an embodiment of the present invention for determining the sensitivity to a CDK 4/6 inhibitor

The distribution of the CDK4 modification profiles observed with the tumors analyzed at the Bordet Institute varied according to their molecular subtypes as defined with the PAM50 references of the R GENEFU package (Gendoo et al., Genefu: an R/Bioconductor package for computation of gene expression-based signatures in breast cancer, 2015, Bioinformatics, 60) (FIG. 10 and 11A). A similar variation was noted when the CDK4 modification profile was predicted using a method according to an embodiment of the present invention (11 genes and corresponding reference centroids as provided in Example 3) in the 56 tumors analyzed at the Bordet Institute (FIG. 11B) or in the 4034 published breast tumor gene expression profiles (FIG. 11C).

Between 50% to 70% of the basal-like tumors were predicted to have the first profile (CDK4 profile 1) wherein phosphorylated CDK4 is undetectable in the tumors analyzed in this study or in the 4034 tumors with published gene expression profiles, respectively (FIG. 11B and FIG. 11C). The proportion of the tumors predicted to display the first profile, linked to unresponsiveness to CDK4/6 inhibitors such as palbociclib, reached about 20% in the HER2+ tumors of both cohorts while it ranged around 5% in Luminal B tumors from both cohorts (FIG. 11B and FIG. 11C).

The second profile (CDK4 modification profile 2) in which CDK4 phosphorylation is preponderant was predicted in 40% or 25% of the basal-like tumors of both cohorts, respectively (FIG. 11B and FIG. 11C). This second profile, linked to sensitivity to CDK4/6 inhibitor such as palbociclib, was also the most frequent CDK4 modification profile in Luminal B tumors (60% and 40%, respectively), and in HER2+ tumors (60% and 50%, respectively) (FIG. 11B and FIG. 11C).

Finally, the proportion of tumors predicted to have the third CDK4 modification profile, wherein phosphorylated CDK4 is detectable but minor, reached about 90% in Luminal A of both cohorts and 70% in normal-like tumors, 30% and 55% in Luminal B tumors of both cohorts, respectively and 20 to 30 % in HER2+ tumors of both cohorts, respectively (FIG. 11B and FIG. 11C). Only around 5% of the basal-like tumors were predicted to present this profile 3.

These results show that although the CDK4 modification profiles are to some extent associated with the molecular subtype of the breast tumor, the molecular subtype is insufficient to determine whether a patient will benefit from a treatment with a CDK4/6 inhibitor such as palbociclib.

Most tumors predicted to have the first and second CDK4 modification profiles were enriched in the Oncotype DX highest risk category, high GGI risk (defined using the GENEFU package) and grade 3 tumors (FIG. 12A, FIG. 12B, FIG. 12C). The opposite was observed for those tumors predicted to have the third CDK4 modification profile enriched mainly in tumors of grade 1 and 2 (FIG. 12C). Between 40 and 50 % of ER positive tumors with high or intermediate Oncotype DX risk or high GGI risk were predicted to have a profile 2 and will therefore benefit from a Palbociclib treatment (FIG. 12D, FIG. 12E). Yet, about 10 % of them were predicted to have a profile 1 and to be unresponsive to the treatment while the others were predicted to have a profile 3. Finally, the gene expression-based CDK4 modification profile prediction was also associated with pathological complete response (pCR) to anthracyclin-taxane treatment (highest in profile 1 tumors and lowest in profile 3 ones (FIG. 12F)).

### Example 5: Redundancy within the gene expression profile of 11 genes or fragments thereof

Generally, the expression of different genes is coordinated to drive a specific phenotype. This coordination often leads to gene co-expression and redundancy with specific genes able to replace others when necessary. To evaluate the impact of this redundancy on the performance of the gene expression profile of the 11 genes or a fragment thereof, we systematically replaced each of the 11 genes by each of the probe set present on the microarray platform used. The identity of the probe sets able to replace any of the 11 genes of the gene expression profile of the 11 genes or a fragment thereof is listed in Table 2.

Columns of Table 2 correspond to the five genes (in particular probes sets) of the original 11 genes that can be replaced by another one. Rows correspond to the genes (probe sets) that can replace the gene (probe set) indicated at the top of each column. The numbers correspond to the concordance rate, i.e., the proportion of correctly classified tumors.

## Claims

1. A method for determining sensitivity to a cyclin-dependent kinase 4/6 (CDK4/6) inhibitor in a human subject having breast cancer, the method comprising the steps of:
- determining in a sample from the subject the level of expression of 11 genes or fragments thereof consisting of cyclin-dependent kinase inhibitor 2A (CDKN2A); cyclin E1 (CCNE1); transgelin 2 (TAGLN2); translocase of inner mitochondrial membrane 17 homolog A (yeast) (TIMM17A); RAB31, member RAS oncogene family (RAB31); spindle apparatus coiled-coil protein 1 (SPDL1); nucleoporin 155kDa (NUP155); F-box and leucine-rich repeat protein 5 (FBXL5); gelsolin (GSN); TP53 target 1 (non-protein coding) (TP53TG1); and protein phosphatase 1 regulatory subunit 3C (PPP1R3C);
- providing three reference profiles of the level of expression of the 11 genes or fragments thereof: a first reference profile representing insensitivity to the CDK4/6 inhibitor; a second reference profile representing therapeutic sensitivity to the CDK4/6 inhibitor; and a third reference profile representing sensitivity to the CDK4/6 inhibitor but without therapeutic benefit over classical treatment by surgery and hormone therapy;
- correlating the level of expression of the 11 genes or fragments thereof in the sample with the level of expression of the 11 genes or fragments thereof in the three reference profiles;
- identifying the reference profile which provides the highest correlation with the level of expression of the 11 genes or fragments thereof in the sample; and
- inferring from the finding that:
(i) the first reference profile provides the highest correlation with the level of expression of the 11 genes or fragments thereof in the sample, that the subject is insensitive to the CDK4/6 inhibitor;
(ii) the second reference profile provides the highest correlation with the level of expression of the 11 genes or fragments thereof in the sample, that the subject is therapeutically sensitive to the CDK4/6 inhibitor; or
(iii) the third reference profile provides the highest correlation with the level of expression of the 11 genes or fragments thereof in the sample, that the subject is sensitive to the CDK4/6 inhibitor but has no clinical benefit from treatment with the CDK4/6 inhibitor.

2. The method according to claim 1, wherein:
- if the subject is insensitive to the CDK4/6 inhibitor, the subject is suitable to be treated by surgery, optionally by a chemotherapeutic agent and/or by radiotherapy; or
- if the subject is therapeutically sensitive to the CDK4/6 inhibitor, the subject is suitable to be treated by administration of the CDK4/6 inhibitor; or
- if the subject is sensitive to the CDK4/6 inhibitor but has no clinical benefit from the treatment with the CDK4/6 inhibitor, the subject is suitable to be treated by surgery and hormone therapy.

3. The method according to claim 1 or 2, wherein the step of correlating the level of expression of the 11 genes or fragments thereof in the sample with the level of expression of the genes in the three reference profiles is performed according to Spearman rank correlation, Euclidean distance; Manhattan distance; Average dot product; Pearson correlation; Pearson uncentered; Pearson squared; Cosine correlation; Covariance value; Kedall's Tau; or Mutual information.

4. The method according to any one of claims 1 to 3, wherein the sample is a formalin-fixed paraffin-embedded (FFPE) sample or fresh frozen sample, preferably a FFPE sample.

5. The method according to any one of claims 1 to 4, wherein the CDK4/6 inhibitor is palbociclib (PD0332991), LEE-011 (ribociclib), LY2835219 (Abemaciclib), G1T28-1, SHR6390, or P276-00, or a derivative of any one of palbociclib, LEE-011, LY2835219, G1T28-1, SHR6390, or P276-00, preferably wherein the CDK4/6 inhibitor is palbociclib.

6. The method according to any one of claims 1 to 5, wherein the level of expression of the 11 genes or fragments thereof is determined by quantitative real-time PCR (qPCR), reverse transcription-qPCR (RT-qPCR), a nucleic acid microarray, digital molecular barcoding technology (nCounter Nanostring), branched DNA (bDNA) signal amplification technology (Quantigene), mass spectrometry (Sequenom), or a combination of said methods.

7. A method for determining a cyclin-dependent kinase 4 (CDK4) modification profile in a human subject having breast cancer, the method comprising the steps of:
- determining in a sample from the subject the level of expression of 11 genes or fragments thereof selected from the group consisting of cyclin-dependent kinase inhibitor 2A (CDKN2A), cyclin E1 (CCNE1), transgelin 2 (TAGLN2), translocase of inner mitochondrial membrane 17 homolog A (yeast) (TIMM17A), RAB31, member RAS oncogene family (RAB31), spindle apparatus coiled-coil protein 1 (SPDL1), nucleoporin 155kDa (NUP155), F-box and leucine-rich repeat protein 5 (FBXL5), gelsolin (GSN), TP53 target 1 (non-protein coding) (TP53TG1), and protein phosphatase 1 regulatory subunit 3C (PPP1R3C);
- providing three reference profiles of the level of expression of the 11 genes or fragments thereof:
(i) a first reference profile representing CDK4 modification profile 1 comprising no phosphorylation on threonine at amino acid position 172 of CDK4;
(ii) a second reference profile representing CDK4 modification profile 2 comprising a first modified form of CDK4 which is phosphorylated on threonine at amino acid position 172 and a second modified form of CDK4, wherein the abundance of the first modified form of CDK4 is equal to or more than 90% of the abundance of the second modified form of CDK4; and
(iii) a third reference profile representing CDK4 modification profile 3 comprising a first modified form of CDK4 which is phosphorylated on threonine at amino acid position 172 and a second modified form of CDK4, wherein the abundance of the first modified form of CDK4 is less than 90% of the abundance of the second modified form of CDK4;
- correlating the level of expression of the 11 genes or fragments thereof in the sample with the level of expression of the 11 genes or fragments thereof in the three reference profiles;
- identifying the reference profile which provides the highest correlation with the level of expression of the 11 genes or fragments thereof in the sample; and
- inferring from the identification of the reference profile which provides the highest correlation with the level of expression of the 11 genes or fragments thereof in the sample that the subject has the CDK4 modification profile corresponding to the CDK4 modification profile of the identified reference profile.

8. The method according to claim 7, wherein the CDK4 modification profile corresponds to a particular sensitivity of the subject to a CDK4/6 inhibitor, wherein:
- the CDK4 modification profile 1 corresponds to insensitivity of the subject to the CDK4/6 inhibitor;
- the CDK4 modification profile 2 corresponds to therapeutic sensitivity of the subject to the CDK4/6 inhibitor; and
- the CDK4 modification profile 3 corresponds to sensitivity of the subject to the CDK4/6 inhibitor but without clinical benefit over classical treatment by surgery and hormone therapy.

9. Use of a kit of parts for determining sensitivity to a CDK4/6 inhibitor in a human subject having breast cancer, the kit comprising means for determining the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, in a sample from a human subject.

10. The use of the kit according to claim 9, wherein the kit comprises primers capable of specifically binding the 11 genes or fragments thereof.

11. Use of a nucleic acid array or nucleic acid microarray for determining sensitivity to a CDK4/6 inhibitor in a human subject having breast cancer, the nucleic acid array or nucleic acid microarray comprising probes capable of binding to 11 genes or fragments thereof selected from the group consisting of CDKN2A, CCNE1, TAGLN2, TIMM17A, RAB31, SPDL1, NUP155, FBXL5, GSN, TP53TG1, and PPP1R3C.

12. A CDK4/6 inhibitor for use in a method of treating breast cancer in a human subject, wherein the subject has been selected to have or has a gene expression profile of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, whereby the gene expression profile of the subject correlates with a reference profile representing therapeutic sensitivity to the CDK4/6 inhibitor.

13. A CDK4/6 inhibitor for use in a method of treating breast cancer in a human subject, wherein the method comprises the steps of:
- determining the level of expression of 11 genes or fragments thereof selected from the group consisting of CDKN2A, CCNE1, TAGLN2, TIMM17A, RAB31, SPDL1, NUP155, FBXL5, GSN, TP53TG1, and PPP1R3C, in a sample from the subject, thereby obtaining a gene expression profile of the subject; and
- identifying whether the gene expression profile of the subject correlates with a reference profile representing therapeutic sensitivity to a CDK4/6 inhibitor.

14. The CDK4/6 inhibitor for use according to claim 12 or 13, wherein the CDK4/6 inhibitor is palbociclib (PD0332991), LEE-011 (ribociclib), LY2835219 (Abemaciclib), G1T28-1, SHR6390, or P276-00, or a derivative of any one of palbociclib, LEE-011, LY2835219, G1T28-1, SHR6390, or P276-00, preferably wherein the CDK4/6 inhibitor is palbociclib.

## Patentansprüche

1. Verfahren zum Bestimmen der Empfindlichkeit gegenüber einem Cyclinabhängige-Kinase-4/6(CDK4/6)-Inhibitor bei einem menschlichen Subjekt mit Brustkrebs, wobei das Verfahren die folgenden Schritte umfasst:
- Bestimmen, in einer Probe von dem Subjekt, des Expressionspegels von 11 Genen oder Fragmenten davon, bestehend aus Cyclin-abhängige-Kinase-2A(CDKN2A)-Inhibitor; Cyclin E1 (CCNE1); Transgelin 2 (TAGLN2); Translokase des Homologen A der inneren Mitochondrienmembran 17 (Hefe) (TIMM17A); RAB31, Element der RAS-Onkogenfamilie (RAB31); Spindelapparat-Coiled-Coil-Protein 1 (SPDL1); Nucleoporin 155 kDa (NUP155); F-Box und Leucin-reiches Wiederholungsprotein 5 (FBXL5); Gelsolin (GSN); TP53-Ziel 1 (nicht für Protein kodierend) (TP53TG1); und regulatorische Untereinheit 3C der Proteinphosphatase 1 (PPP1R3C);
- Bereitstellen von drei Referenzprofilen des Expressionspegels der 11 Gene oder Fragmente davon: einem ersten Referenzprofil, das die Unempfindlichkeit gegenüber dem CDK4/6-Inhibitor darstellt; einem zweiten Referenzprofil, das die therapeutische Empfindlichkeit gegenüber dem CDK4/6-Inhibitor darstellt; und einem dritten Referenzprofil, das die Empfindlichkeit gegenüber dem CDK4/6-Inhibitor darstellt, jedoch ohne therapeutischen Nutzen gegenüber der klassischen Behandlung durch Operation und Hormontherapie;
- Korrelieren des Expressionspegels der 11 Gene oder Fragmente davon in der Probe mit dem Expressionspegel der 11 Gene oder Fragmente davon in den drei Referenzprofilen;
- Identifizieren des Referenzprofils, das die höchste Korrelation mit dem Expressionspegel der 11 Gene oder Fragmente davon in der Probe liefert; und
- Ableiten, aus dem Ergebnis:
(i) in dem das erste Referenzprofil die höchste Korrelation mit dem Expressionspegel der 11 Gene oder Fragmente davon in der Probe liefert, dass das Subjekt gegenüber den CDK4/6-Inhibitor unempfindlich ist;
(ii) in dem das zweite Referenzprofil die höchste Korrelation mit dem Expressionspegel der 11 Gene oder Fragmente davon in der Probe liefert, dass das Subjekt gegenüber den CDK4/6-Inhibitor therapeutisch empfindlich ist; oder
(iii) in dem das dritte Referenzprofil die höchste Korrelation mit dem Expressionspegel der 11 Gene oder Fragmente davon in der Probe liefert, dass das Subjekt gegenüber den CDK4/6-Inhibitor empfindlich ist, aber keinen klinischen Nutzen aus der Behandlung mit dem CDK4/6-Inhibitor hat.

2. Verfahren nach Anspruch 1, wobei:
- wenn das Subjekt unempfindlich gegenüber dem CDK4/6-Inhibitor ist, das Subjekt operativ, optional mit einem Chemotherapeutikum und/oder einer Strahlentherapie behandelt werden kann; oder
- wenn das Subjekt therapeutisch empfindlich gegenüber dem CDK4/6-Inhibitor ist, das Subjekt zur Behandlung durch Verabreichung des CDK4/6-Inhibitors geeignet ist; oder
- wenn das Subjekt gegenüber dem CDK4/6-Inhibitor empfindlich ist, aber keinen klinischen Nutzen aus der Behandlung mit dem CDK4/6-Inhibitor hat, das Subjekt zur Behandlung durch Operation und Hormontherapie geeignet ist.

3. Verfahren nach Anspruch 1 oder 2, wobei der Schritt des Korrelierens des Expressionspegels der 11 Gene oder Fragmente davon in der Probe mit dem Expressionspegel der Gene in den drei Referenzprofilen durchgeführt wird gemäß Spearman-Rangkorrelation, euklidischem Abstand; Manhattan-Abstand; durchschnittlichem Skalarprodukt; Pearson-Korrelation; Pearson unzentriert; Pearson quadriert; Kosinuskorrelation; Kovarianzwert; Kedalls Tau; oder gegenseitiger Information.

4. Verfahren nach einem der Ansprüche 1 bis 3, wobei die Probe eine formalinfixierte in Paraffin eingebettete (FFPE) Probe oder eine frische gefrorene Probe ist, vorzugsweise eine FFPE-Probe.

5. Verfahren nach einem der Ansprüche 1 bis 4, wobei der CDK4/6-Inhibitor Palbociclib (PD0332991), LEE-011 (Ribociclib), LY2835219 (Abemaciclib), G1T28-1, SHR6390 oder P276-00, oder ein Derivat von einem beliebigen aus Palbociclib, LEE-011, LY2835219, G1T28-1, SHR6390 oder P276-00 ist, vorzugsweise wobei der CDK4/6-Inhibitor Palbociclib ist.

6. Verfahren nach einem der Ansprüche 1 bis 5, wobei der Expressionspegel der 11 Gene oder Fragmente davon durch quantitative Echtzeit-PCR (qPCR), reverse Transkriptions-qPCR (RT-qPCR), ein Nukleinsäuremikroarray, digitale molekulare Barcodierungstechnologie (nCounter Nanostring), Signalamplifikationstechnologie für verzweigte DNA (bDNA) (Quantigene), Massenspektrometrie (Sequenom) oder eine Kombination dieser Verfahren bestimmt wird.

7. Verfahren zum Bestimmen eines Cyclin-abhängige-Kinase-4(CDK4)-Modifikationsprofils bei einem menschlichen Subjekt mit Brustkrebs, wobei das Verfahren die folgenden Schritte umfasst:
- Bestimmen, in einer Probe von dem Subjekt, des Expressionspegels von 11 Genen oder Fragmenten davon, ausgewählt aus der Gruppe bestehend aus Cyclin-abhängige-Kinase-2A(CDKN2A)-Inhibitor, Cyclin E1 (CCNE1), Transgelin 2 (TAGLN2), Translokase des Homologen A der inneren Mitochondrienmembran 17 (Hefe) (TIMM17A), RAB31, Element der RAS-Onkogenfamilie (RAB31), Spindelapparat-Coiled-Coil-Protein 1 (SPDL1), Nucleoporin 155 kDa (NUP155), F-Box und Leucin-reiches Wiederholungsprotein 5 (FBXL5), Gelsolin (GSN), TP53-Ziel 1 (nicht für Protein kodierend) (TP53TG1), und regulatorische Untereinheit 3C der Proteinphosphatase 1 (PPP1R3C);
- Bereitstellen von drei Referenzprofilen des Expressionspegels der 11 Gene oder Fragmente davon:
(i) einem ersten Referenzprofil, das das CDK4-Modifikationsprofil 1 darstellt, welches keine Phosphorylierung an Threonin an der Aminosäureposition 172 von CDK4 umfasst;
(ii) einem zweiten Referenzprofil, das das CDK4-Modifikationsprofil 2 darstellt, welches eine erste modifizierte Form von CDK4, die an Threonin an Aminosäureposition 172 phosphoryliert ist, und eine zweite modifizierte Form von CDK4 umfasst, wobei die Häufigkeit der ersten modifizierten Form von CDK4 mindestens 90 % der Häufigkeit der zweiten modifizierten Form von CDK4 entspricht; und
(iii) einem dritten Referenzprofil, das das CDK4-Modifikationsprofil 3 darstellt, welches eine erste modifizierte Form von CDK4, die an Threonin an Aminosäureposition 172 phosphoryliert ist, und eine zweite modifizierte Form von CDK4 umfasst, wobei die Häufigkeit der ersten modifizierten Form von CDK4 weniger als 90 % der Häufigkeit der zweiten modifizierten Form von CDK4 entspricht;
- Korrelieren des Expressionspegels der 11 Gene oder Fragmente davon in der Probe mit dem Expressionspegel der 11 Gene oder Fragmente davon in den drei Referenzprofilen;
- Identifizieren des Referenzprofils, das die höchste Korrelation mit dem Expressionspegel der 11 Gene oder Fragmente davon in der Probe liefert; und
- Ableiten, aus der Identifizierung des Referenzprofils, das die höchste Korrelation mit dem Expressionspegel der 11 Gene oder Fragmente davon in der Probe liefert, dass das Subjekt das CDK4-Modifikationsprofil aufweist, welches dem CDK4-Modifikationsprofil des identifizierten Referenzprofils entspricht.

8. Verfahren nach Anspruch 7, wobei das CDK4-Modifikationsprofil einer bestimmten Empfindlichkeit des Subjekts auf einen CDK4/6-Inhibitor entspricht, wobei:
- das CDK4-Modifikationsprofil 1 einer Unempfindlichkeit des Subjekts auf den CDK4/6-Inhibitor entspricht;
- das CDK4-Modifikationsprofil 2 einer therapeutischen Empfindlichkeit des Subjekts auf den CDK4/6-Inhibitor entspricht; und
- das CDK4-Modifikationsprofil 3 einer Empfindlichkeit des Subjekts auf den CDK4/6-Inhibitor entspricht, jedoch ohne klinischen Nutzen gegenüber der klassischen chirurgischen Behandlung und Hormontherapie.

9. Verwendung eines Kits von Teilen zum Bestimmen der Empfindlichkeit gegenüber einem CDK4/6-Inhibitor bei einem menschlichen Subjekt mit Brustkrebs, wobei das Kit Mittel zum Bestimmen des Expressionspegels von 11 Genen oder Fragmenten davon, ausgewählt aus der Gruppe bestehend aus CDKN2A, CCNE1, TAGLN2, TIMM17A, RAB31, SPDL1, NUP155, FBXL5, GSN, TP53TG1 und PPP1R3C in einer Probe von einem menschlichen Subjekt umfasst.

10. Verwendung des Kits nach Anspruch 9, wobei das Kit Primer umfasst, die in der Lage sind, die 11 Gene oder Fragmente davon spezifisch zu binden.

11. Verwendung eines Nukleinsäurearrays oder eines Nukleinsäuremikroarrays zum Bestimmen der Empfindlichkeit gegenüber einem CDK4/6-Inhibitor bei einem menschlichen Subjekt mit Brustkrebs, wobei das Nukleinsäurearray oder -mikroarray Sonden umfasst, die in der Lage sind an 11 Gene oder Fragmente davon zu binden, ausgewählt aus der Gruppe bestehend aus CDKN2A, CCNE1, TAGLN2, TIMM17A, RAB31, SPDL1, NUP155, FBXL5, GSN, TP53TG1 und PPP1R3C.

12. CDK4/6-Inhibitor zur Verwendung in einem Verfahren zum Behandeln von Brustkrebs bei einem menschlichen Subjekt, wobei das Subjekt ausgewählt worden ist, ein Genexpressionsprofil von 11 Genen oder Fragmenten davon, ausgewählt aus der Gruppe bestehend aus CDKN2A, CCNE1, TAGLN2, TIMM17A, RAB31, SPDL1, NUP155, FBXL5, GSN, TP53TG1 und PPP1R3C, zu haben, oder dies hat, wobei das Genexpressionsprofil des Subjekts mit einem Referenzprofil korreliert, welches die therapeutische Empfindlichkeit gegenüber dem CDK4/6-Inhibitor darstellt.

13. CDK4/6-Inhibitor zur Verwendung in einem Verfahren zum Behandeln von Brustkrebs bei einem menschlichen Subjekt, wobei das Verfahren die folgenden Schritte aufweist:
- Bestimmen des Expressionspegels von 11 Genen oder Fragmenten davon, ausgewählt aus der Gruppe bestehend aus CDKN2A, CCNE1, TAGLN2, TIMM17A, RAB31, SPDL1, NUP155, FBXL5, GSN, TP53TG1 und PPP1R3C in einer Probe von dem Subjekt, wodurch ein Genexpressionsprofil des Subjekts erhalten wird; und
- Identifizieren, ob das Genexpressionsprofil des Subjekts mit einem Referenzprofil korreliert, das die therapeutische Empfindlichkeit gegenüber einem CDK4/6-Inhibitor darstellt.

14. CDK4/6-Inhibitor zur Verwendung in einem Verfahren nach einem der Ansprüche 12 und 13, wobei der CDK4/6-Inhibitor Palbociclib (PD0332991), LEE-011 (Ribociclib), LY2835219 (Abemaciclib), G1T28-1, SHR6390 oder P276-00, oder ein Derivat von einem beliebigen aus Palbociclib, LEE-011, LY2835219, G1T28-1, SHR6390 oder P276-00 ist, vorzugsweise wobei der CDK4/6-Inhibitor Palbociclib ist.

## Revendications

1. Procédé de détermination de la sensibilité à un inhibiteur de kinase dépendante des cyclines 4/6 (CDK4/6) chez un sujet humain ayant un cancer du sein, le procédé comprenant les étapes de :
- détermination, dans un échantillon provenant du sujet, du taux d'expression de 11 gènes ou fragments de ceux-ci constitués de l'inhibiteur de kinase dépendante des cyclines 2A (CDKN2A) ; cycline E1 (CCNE1) ; la transgéline 2 (TAGLN2) ; l'homologue A de translocase de membrane mitochondriale interne 17 (levure) (TIMM17A) ; RAB31, membre de la famille des oncogènes RAS (RAB31) ; spindle apparatus coiled-coil protein 1 (SPDL1) ; la nucléoporine de 155 kDa (NUP155) ; F-box and leucine-rich repeat protein 5 (FBXL5) ; la gelsoline (GSN) ; TP53 target 1 (ne codant pas pour une protéine) (TP53TG1) ; et la sous-unité régulatrice 3C de protéine phosphatase 1 (PPP1R3C) ;
- fourniture de trois profils de référence du taux d'expression des 11 gènes ou fragments de ceux-ci : un premier profil de référence représentant l'insensibilité à l'inhibiteur de CDK4/6 ; un deuxième profil de référence représentant la sensibilité thérapeutique à l'inhibiteur de CDK4/6 ; et un troisième profil de référence représentant la sensibilité à l'inhibiteur de CDK4/6 mais sans bénéfice thérapeutique par rapport à un traitement classique par chirurgie et hormonothérapie ;
- corrélation du taux d'expression des 11 gènes ou fragments de ceux-ci dans l'échantillon avec le taux d'expression des 11 gènes ou fragments de ceux-ci dans les trois profils de référence ;
- identification du profil de référence qui fournit la plus haute corrélation avec le taux d'expression des 11 gènes ou fragments de ceux-ci dans l'échantillon ; et
- déduction, à partir de l'observation du fait que :
(i) le premier profil de référence fournit la plus haute corrélation avec le taux d'expression des 11 gènes ou fragments de ceux-ci dans l'échantillon, que le sujet est insensible à l'inhibiteur de CDK4/6 ;
(ii) le deuxième profil de référence fournit la plus haute corrélation avec le taux d'expression des 11 gènes ou fragments de ceux-ci dans l'échantillon, que le sujet est thérapeutiquement sensible à l'inhibiteur de CDK4/6 ; ou
(iii) le troisième profil de référence fournit la plus haute corrélation avec le taux d'expression des 11 gènes ou fragments de ceux-ci dans l'échantillon, que le sujet est sensible à l'inhibiteur de CDK4/6 mais n'a aucun bénéfice clinique du traitement avec l'inhibiteur de CDK4/6.

2. Procédé selon la revendication 1, dans lequel :
- si le sujet est insensible à l'inhibiteur de CDK4/6, le sujet convient à un traitement par chirurgie, facultativement par un agent chimiothérapeutique et/ou par radiothérapie ; ou
- si le sujet est thérapeutiquement sensible à l'inhibiteur de CDK4/6, le sujet convient à un traitement par administration de l'inhibiteur de CDK4/6 ; ou
- si le sujet est sensible à l'inhibiteur de CDK4/6 mais n'a aucun bénéfice clinique du traitement avec l'inhibiteur de CDK4/6, le sujet convient à un traitement par chirurgie et hormonothérapie.

3. Procédé selon la revendication 1 ou 2, dans lequel l'étape de corrélation du taux d'expression des 11 gènes ou fragments de ceux-ci dans l'échantillon avec le taux d'expression des gènes dans les trois profils de référence est effectuée en fonction de la corrélation de rang de Spearman, de la distance euclidienne ; de la distance de Manhattan ; du produit scalaire moyen ; de la corrélation de Pearson ; du Pearson non centré ; du carré de Pearson ; de la corrélation cosinusoïdale ; de la valeur de covariance ; du tau de Kedall ; ou d'informations mutuelles.

4. Procédé selon l'une quelconque des revendications 1 à 3, dans lequel l'échantillon est un échantillon fixé au formol et inclus dans la paraffine (FFPE) ou un échantillon frais congelé, de préférence un échantillon FFPE.

5. Procédé selon l'une quelconque des revendications 1 à 4, dans lequel l'inhibiteur de CDK4/6 est le palbociclib (PD0332991), LEE-011 (ribociclib), LY2835219 (abémaciclib), G1T28-1, SHR6390 ou P276-00, ou un dérivé de l'un quelconque parmi le palbociclib, LEE-011, LY2835219, G1T28-1, SHR6390 ou P276-00, de préférence dans lequel l'inhibiteur de CDK4/6 est le palbociclib.

6. Procédé selon l'une quelconque des revendications 1 à 5, dans lequel le taux d'expression des 11 gènes ou fragments de ceux-ci est déterminé par PCR quantitative en temps réel (qPCR), qPCR par transcription inverse (RT-qPCR), un microréseau d'acide nucléique, une technologie de codage à barres moléculaire numérique (nCounter Nanostring), une technologie d'amplification de signal d'ADN ramifié (bDNA) (Quantigene), spectrométrie de masse (Sequenom), ou une combinaison desdits procédés.

7. Procédé de détermination d'un profil de modification de kinase dépendante des cyclines 4 (CDK4) chez un sujet humain ayant un cancer du sein, le procédé comprenant les étapes de :
- détermination, dans un échantillon provenant du sujet, du taux d'expression de 11 gènes ou fragments de ceux-ci choisis dans le groupe constitué de l'inhibiteur de kinase dépendante des cyclines 2A (CDKN2A), cycline E1 (CCNE1), la transgéline 2 (TAGLN2), l'homologue A de translocase de membrane mitochondriale interne 17 (levure) (TIMM17A), RAB31, membre de la famille des oncogènes RAS (RAB31), spindle apparatus coiled-coil protein 1 (SPDL1), la nucléoporine de 155 kDa (NUP155), F-box and leucine-rich repeat protein 5 (FBXL5), la gelsoline (GSN), TP53 target 1 (ne codant pas pour une protéine) (TP53TG1), et la sous-unité régulatrice 3C de protéine phosphatase 1 (PPP1R3C) ;
- fourniture de trois profils de référence du taux d'expression des 11 gènes ou fragments de ceux-ci :
(i) un premier profil de référence représentant un profil de modification de CDK4 1 ne comprenant aucune phosphorylation sur la thréonine à la position d'acide aminé 172 de CDK4 ;
(ii) un deuxième profil de référence représentant un profil de modification de CDK4 2 comprenant une première forme modifiée de CDK4 qui est phosphorylée sur la thréonine à la position d'acide aminé 172 et une deuxième forme modifiée de CDK4, où l'abondance de la première forme modifiée de CDK4 est supérieure ou égale à 90 % de l'abondance de la deuxième forme modifiée de CDK4 ; et
(iii) un troisième profil de référence représentant un profil de modification de CDK4 3 comprenant une première forme modifiée de CDK4 qui est phosphorylée sur la thréonine à la position d'acide aminé 172 et une deuxième forme modifiée de CDK4, où l'abondance de la première forme modifiée de CDK4 est inférieure à 90 % de l'abondance de la deuxième forme modifiée de CDK4 ;
- corrélation du taux d'expression des 11 gènes ou fragments de ceux-ci dans l'échantillon avec le taux d'expression des 11 gènes ou fragments de ceux-ci dans les trois profils de référence ;
- identification du profil de référence qui fournit la plus haute corrélation avec le taux d'expression des 11 gènes ou fragments de ceux-ci dans l'échantillon ; et
- déduction à partir de l'identification du profil de référence qui fournit la plus haute corrélation avec le taux d'expression des 11 gènes ou fragments de ceux-ci dans l'échantillon que le sujet a le profil de modification de CDK4 correspondant au profil de modification de CDK4 du profil de référence identifié.

8. Procédé selon la revendication 7, dans lequel le profil de modification de CDK4 correspond à une sensibilité particulière du sujet à un inhibiteur de CDK4/6, dans lequel :
- le profil de modification de CDK4 1 correspond à une insensibilité du sujet à l'inhibiteur de CDK4/6 ;
- le profil de modification de CDK4 2 correspond à une sensibilité thérapeutique du sujet à l'inhibiteur de CDK4/6 ; et
- le profil de modification de CDK4 3 correspond à une sensibilité du sujet à l'inhibiteur de CDK4/6 mais sans bénéfice clinique par rapport à un traitement classique par chirurgie et hormonothérapie.

9. Utilisation d'un kit de composants pour déterminer la sensibilité à un inhibiteur de CDK4/6 chez un sujet humain ayant un cancer du sein, le kit comprenant un moyen pour déterminer le taux d'expression de 11 gènes ou fragments de ceux-ci choisis dans le groupe constitué de CDKN2A, CCNE1, TAGLN2, TIMM17A, RAB31, SPDL1, NUP155, FBXL5, GSN, TP53TG1 et PPP1R3C, dans un échantillon provenant d'un sujet humain.

10. Utilisation du kit selon la revendication 9, dans laquelle le kit comprend des amorces capables de se lier spécifiquement aux 11 gènes ou fragments de ceux-ci.

11. Utilisation d'un réseau d'acide nucléique ou microréseau d'acide nucléique pour déterminer la sensibilité à un inhibiteur de CDK4/6 chez un sujet humain ayant un cancer du sein, le réseau d'acide nucléique ou microréseau d'acide nucléique comprenant des sondes capables de se lier à 11 gènes ou fragments de ceux-ci choisis dans le groupe constitué de CDKN2A, CCNE1, TAGLN2, TIMM17A, RAB31, SPDL1, NUP155, FBXL5, GSN, TP53TG1 et PPP1R3C.

12. Inhibiteur de CDK4/6 pour utilisation dans un procédé de traitement du cancer du sein chez un sujet humain, où le sujet a été sélectionné comme ayant ou a un profil d'expression génique de 11 gènes ou fragments de ceux-ci choisis dans le groupe constitué de CDKN2A, CCNE1, TAGLN2, TIMM17A, RAB31, SPDL1, NUP155, FBXL5, GSN, TP53TG1 et PPP1R3C, où le profil d'expression génique du sujet est corrélé à un profil de référence représentant la sensibilité thérapeutique à l'inhibiteur de CDK4/6.

13. Inhibiteur de CDK4/6 pour utilisation dans un procédé de traitement du cancer du sein chez un sujet humain, où le procédé comprend les étapes de :
- détermination du taux d'expression de 11 gènes ou fragments de ceux-ci choisis dans le groupe constitué de CDKN2A, CCNE1, TAGLN2, TIMM17A, RAB31, SPDL1, NUP155, FBXL5, GSN, TP53TG1 et PPP1R3C, dans un échantillon provenant du sujet, de façon à obtenir un profil d'expression génique du sujet ; et
- identification du fait que le profil d'expression génique du sujet est corrélé à un profil de référence représentant la sensibilité thérapeutique à un inhibiteur de CDK4/6.

14. Inhibiteur de CDK4/6 pour utilisation selon la revendication 12 ou 13, où l'inhibiteur de CDK4/6 est le palbociclib (PD0332991), LEE-011 (ribociclib), LY2835219 (abémaciclib), G1T28-1, SHR6390, ou P276-00, ou un dérivé de l'un quelconque parmi le palbociclib, LEE-011, LY2835219, G1T28-1, SHR6390 ou P276-00, de préférence où l'inhibiteur de CDK4/6 est le palbociclib.
